# EUROPEAN PATENT APPLICATION

(11) **EP 2 796 563 A1**
(43) Date of publication of application: **29.10.2014**
(21) Application number: 14169713.6
(22) Date of filing: 04.05.2011
(51) Int. Cl.: C12Q 1/00, G01N 33/48, G01N 33/53, G01N 33/68, C12Q 1/68, A61K 39/00, A61K 31/56, A61K 31/7088, A61K 35/34, A61K 39/395, A61K 45/00

(54) **Optimized degenerative muscle disease diagnostics and treatments**

(30) Priority: 04.05.2010 US 331171 P; 04.05.2010 US 331166 P
(62) Divisional of application: 11778240.9
(71) Applicant: MedImmune, LLC, Cambridge Cambridgeshire CB21 6GH (GB)
(72) Inventor: Yao, Yihong, Boyds, MD Maryland 20841 (US); Georgantas, Robert, William, Gaithersburg, MD Maryland 20878 (US); Zhu, Wei, Boyds, MD Maryland 20841 (US); Streicher, Katie, Bethesda, MD Maryland 20814 (US); Renade, Koustubh, Potomac, MD Maryland 20854 (US)
(74) Representative: Winter, Christopher Spencer

(57) **Abstract**

A method which comprises identifying the presence, absence or amount of a biomarker in a subject having an inflammatory myopathy to whom an immunosuppressive drug has been administered, wherein the biomarker is a microRNA-133 selected from one or more of the group consisting of microRNA-133a-1, microRNA-133a-2 and mircroRNA-133b; and maintaining a subsequent dosage of the drug or adjusting a subsequent dosage of the drug administered to the subject based on the presence, absence or amount of the biomarker identified in the subject.

SEQUENCE LISTING

<110> MEDIMMUNE, LLC

<120> OPTIMIZED DEGENERATIVE MUSCLE DISEASE DIAGNOSTICS AND TREATMENTS

<130> MED0567.PCT

<140> PCT/US2011/035125
<141> 2011-05-04

<150> 61/331,171
<151> 2010-05-04

<150> 61/331,166
<151> 2010-05-04

<160> 13

<170> PatentIn version 3.5

<210> 1
<211> 10
<212> DNA
<213> Unknown

<220>
<223> Description of Unknown: NF-kappa-B binding site
      oligonucleotide

<400> 1
gggactttcc                                                              10


<210> 2
<211> 2763
<212> DNA
<213> Homo sapiens

<400> 2
gctccctcag caaggacagc agaggaccag ctaagaggga gagaagcaac tacagacccc      60

ccctgaaaac aaccctcaga cgccacatcc cctgacaagc tgccaggcag gttctcttcc     120

tctcacatac tgacccacgg ctccaccctc tctcccctgg aaaggacacc atgagcactg     180

aaagcatgat ccgggacgtg gagctggccg aggaggcgct ccccaagaag acaggggggc     240

cccagggctc caggcggtgc ttgttcctca gcctcttctc cttcctgatc gtggcaggcg     300

ccaccacgct cttctgcctg ctgcactttg gagtgatcgg cccccagagg gaagaggtga     360

gtgcctggcc agccttcatc cactctccca cccaagggga aatggagacg caagagaggg     420

agagagatgg gatgggtgaa agatgtgcgc tgatagggag ggatggagag aaaaaaacgt     480

ggagaaagac ggggatgcag aaagagatgt ggcaagagat ggggaagaga gagagagaaa     540

gatggagaga caggatgtct ggcacatgga aggtgctcac taagtgtgta tggagtgaat     600

gaatgaatga atgaatgaac aagcagatat ataaataaga tatggagaca gatgtggggt     660

gtgagaagag agatggggga agaaacaagt gatatgaata aagatggtga gacagaaaga     720

gcgggaaata tgacagctaa ggagagagat gggggagata aggagagaag aagatagggt 780

gtctggcaca cagaagacac tcagggaaag agctgttgaa tgcctggaag gtgaatacac 840

agatgaatgg agagagaaaa ccagacacct cagggctaag agcgcaggcc agacaggcag 900

ccagctgttc ctcctttaag ggtgactccc tcgatgttaa ccattctcct tctccccaac 960

agttccccag ggacctctct ctaatcagcc ctctggccca ggcagtcagt aagtgtctcc 1020

aaacctcttt cctaattctg ggtttgggtt tgggggtagg gttagtaccg gtatggaagc 1080

agtgggggaa atttaaagtt ttggtcttgg gggaggatgg atggaggtga aagtaggggg 1140

gtattttcta ggaagtttaa gggtctcagc tttttctttt ctctctcctc ttcaggatca 1200

tcttctcgaa ccccgagtga caagcctgta gcccatgttg taggtaagag ctctgaggat 1260

gtgtcttgga acttggaggg ctaggatttg gggattgaag cccggctgat ggtaggcaga 1320

acttggagac aatgtgagaa ggactcgctg agctcaaggg aagggtggag gaacagcaca 1380

ggccttagtg ggatactcag aacgtcatgg ccaggtggga tgtgggatga cagacagaga 1440

ggacaggaac cggatgtggg gtgggcagag ctcgagggcc aggatgtgga gagtgaaccg 1500

acatggccac actgactctc ctctccctct ctccctccct ccagcaaacc ctcaagctga 1560

ggggcagctc cagtggctga accgccgggc caatgccctc ctggccaatg gcgtggagct 1620

gagagataac cagctggtgg tgccatcaga gggcctgtac ctcatctact cccaggtcct 1680

cttcaagggc caaggctgcc cctccaccca tgtgctcctc acccacacca tcagccgcat 1740

cgccgtctcc taccagacca aggtcaacct cctctctgcc atcaagagcc cctgccagag 1800

ggagacccca gagggggctg aggccaagcc ctggtatgag cccatctatc tgggaggggt 1860

cttccagctg gagaagggtg accgactcag cgctgagatc aatcggcccg actatctcga 1920

ctttgccgag tctgggcagg tctactttgg gatcattgcc ctgtgaggag gacgaacatc 1980

caaccttccc aaacgcctcc cctgccccaa tccctttatt accccctcct tcagacaccc 2040

tcaacctctt ctggctcaaa aagagaattg ggggcttagg gtcggaaccc aagcttagaa 2100

ctttaagcaa caagaccacc acttcgaaac ctgggattca ggaatgtgtg gcctgcacag 2160

tgaagtgctg gcaaccacta agaattcaaa ctggggcctc cagaactcac tggggcctac 2220

agctttgatc cctgacatct ggaatctgga gaccagggag cctttggttc tggccagaat 2280

gctgcaggac ttgagaagac ctcacctaga aattgacaca agtggacctt aggccttcct 2340

ctctccagat gtttccagac ttccttgaga cacggagccc agccctcccc atggagccag 2400

ctccctctat ttatgtttgc acttgtgatt atttattatt tatttattat ttatttattt 2460

acagatgaat gtatttattt gggagaccgg ggtatcctgg gggacccaat gtaggagctg 2520

ccttggctca gacatgtttt ccgtgaaaac ggagctgaac aataggctgt tcccatgtag 2580

ccccctggcc tctgtgcctt cttttgatta tgttttttaa aatatttatc tgattaagtt 2640

gtctaaacaa tgctgatttg gtgaccaact gtcactcatt gctgagcctc tgctccccag 2700

gggagttgtg tctgtaatcg ccctactatt cagtggcgag aaataaagtt tgcttagaaa 2760

aga 2763

<210> 3
<211> 1669
<212> DNA
<213> Homo sapiens

<400> 3
ctccctcagc aaggacagca gaggaccagc taagagggag agaagcaact acagaccccc 60

cctgaaaaca accctcagac gccacatccc ctgacaagct gccaggcagg ttctcttcct 120

ctcacatact gacccacggc tccaccctct ctcccctgga aaggacacca tgagcactga 180

aagcatgatc cgggacgtgg agctggccga ggaggcgctc cccaagaaga caggggggcc 240

ccagggctcc aggcggtgct tgttcctcag cctcttctcc ttcctgatcg tggcaggcgc 300

caccacgctc ttctgcctgc tgcactttgg agtgatcggc ccccagaggg aagagttccc 360

cagggacctc tctctaatca gccctctggc ccaggcagtc agatcatctt ctcgaacccc 420

gagtgacaag cctgtagccc atgttgtagc aaaccctcaa gctgaggggc agctccagtg 480

gctgaaccgc cgggccaatg ccctcctggc caatggcgtg gagctgagag ataaccagct 540

ggtggtgcca tcagagggcc tgtacctcat ctactcccag gtcctcttca agggccaagg 600

ctgcccctcc acccatgtgc tcctcaccca caccatcagc cgcatcgccg tctcctacca 660

gaccaaggtc aacctcctct ctgccatcaa gagcccctgc cagagggaga ccccagaggg 720

ggctgaggcc aagccctggt atgagcccat ctatctggga ggggtcttcc agctggagaa 780

gggtgaccga ctcagcgctg agatcaatcg gcccgactat ctcgactttg ccgagtctgg 840

gcaggtctac tttgggatca ttgccctgtg aggaggacga acatccaacc ttcccaaacg 900

cctcccctgc cccaatccct ttattacccc ctccttcaga caccctcaac ctcttctggc 960

tcaaaaagag aattgggggc ttagggtcgg aacccaagct tagaacttta agcaacaaga 1020

ccaccacttc gaaacctggg attcaggaat gtgtggcctg cacagtgaag tgctggcaac 1080

cactaagaat tcaaactggg gcctccagaa ctcactgggg cctacagctt tgatccctga 1140

catctggaat ctggagacca gggagccttt ggttctggcc agaatgctgc aggacttgag 1200

aagacctcac ctagaaattg acacaagtgg accttaggcc ttcctctctc cagatgtttc 1260

cagacttcct tgagacacgg agcccagccc tccccatgga gccagctccc tctatttatg 1320

tttgcacttg tgattattta ttatttattt attatttatt tatttacaga tgaatgtatt 1380

tatttgggag accggggtat cctgggggac ccaatgtagg agctgccttg gctcagacat 1440

gttttccgtg aaaacggagc tgaacaatag gctgttccca tgtagccccc tggcctctgt     1500

gccttctttt gattatgttt tttaaaatat ttatctgatt aagttgtcta aacaatgctg     1560

atttggtgac caactgtcac tcattgctga gcctctgctc cccaggggag ttgtgtctgt     1620

aatcgcccta ctattcagtg gcgagaaata aagtttgctt agaaaagaa                 1669

<210> 4
<211> 233
<212> PRT
<213> Homo sapiens

<400> 4
Met Ser Thr Glu Ser Met Ile Arg Asp Val Glu Leu Ala Glu Glu Ala
1               5                   10                  15

Leu Pro Lys Lys Thr Gly Gly Pro Gln Gly Ser Arg Arg Cys Leu Phe
            20                  25                  30

Leu Ser Leu Phe Ser Phe Leu Ile Val Ala Gly Ala Thr Thr Leu Phe
        35                  40                  45

Cys Leu Leu His Phe Gly Val Ile Gly Pro Gln Arg Glu Glu Phe Pro
    50                  55                  60

Arg Asp Leu Ser Leu Ile Ser Pro Leu Ala Gln Ala Val Arg Ser Ser
65                  70                  75                  80

Ser Arg Thr Pro Ser Asp Lys Pro Val Ala His Val Val Ala Asn Pro
                85                  90                  95

Gln Ala Glu Gly Gln Leu Gln Trp Leu Asn Arg Arg Ala Asn Ala Leu
            100                 105                 110

Leu Ala Asn Gly Val Glu Leu Arg Asp Asn Gln Leu Val Val Pro Ser
        115                 120                 125

Glu Gly Leu Tyr Leu Ile Tyr Ser Gln Val Leu Phe Lys Gly Gln Gly
    130                 135                 140

Cys Pro Ser Thr His Val Leu Leu Thr His Thr Ile Ser Arg Ile Ala
145                 150                 155                 160

Val Ser Tyr Gln Thr Lys Val Asn Leu Leu Ser Ala Ile Lys Ser Pro
                165                 170                 175

Cys Gln Arg Glu Thr Pro Glu Gly Ala Glu Ala Lys Pro Trp Tyr Glu
            180                 185                 190

Pro Ile Tyr Leu Gly Gly Val Phe Gln Leu Glu Lys Gly Asp Arg Leu
        195                 200                 205

Ser Ala Glu Ile Asn Arg Pro Asp Tyr Leu Asp Phe Ala Glu Ser Gly
    210                 215                 220

Gln Val Tyr Phe Gly Ile Ile Ala Leu
225                 230

<210> 5
<211> 2375
<212> DNA
<213> Homo sapiens

<400> 5
tacacagaga gaaaggctaa agttctctgg aggatgtggc tgcagagcct gctgctcttg      60

ggcactgtgg cctgcagcat ctctgcaccc gcccgctcgc ccagccccag cacgcagccc     120

tgggagcatg tgaatgccat ccaggaggcc cggcgtctcc tgaacctgag tagagacact     180

gctgctgaga tggtaagtga gagaatgtgg gcctgtgcct aggccaccca gctggcccct     240

gactggccac gcctgtcagc ttgataacat gacattttcc ttttctacag aatgaaacag     300

tagaagtcat ctcagaaatg tttgacctcc aggtaagatg cttctctctg acatagcttt     360

ccagaagccc ctgccctggg gtggaggtgg ggactccatt ttagatggca ccacacaggg     420

ttgtccactt tctctccagt cagctggctg caggaggagg gggtagcaac tgggtgctca     480

agaggctgct ggccgtgccc ctatggcagt cacatgagct cctttatcag ctgagcggcc     540

atgggcagac ctagcattca atggccagga gtcaccaggg gacaggtggt aaagtggggg     600

tcacttcatg agacaggagc tgtgggtttg gggcgctcac tgtgccccga gaccaagtcc     660

tgttgagaca gtgctgacta cagagaggca cagaggggtt tcaggaacaa cccttgccca     720

cccagcaggt ccaggtgagg ccccaccccc ctctccctga atgatggggt gagagtcacc     780

tccttcccta aggctgggct cctctccagg tgccgctgag ggtggcctgg gcggggcagt     840

gagaagggca ggttcgtgcc tgccatggac agggcagggt ctatgactgg acccagcctg     900

tgcccctccc aagccctact cctgggggct gggggcagca gcaaaaagga gtggtggaga     960

gttcttgtac cactgtgggc acttggccac tgctcaccga cgaacgacat tttccacagg    1020

agccgacctg cctacagacc cgcctggagc tgtacaagca gggcctgcgg ggcagcctca    1080

ccaagctcaa gggccccttg accatgatgg ccagccacta caagcagcac tgccctccaa    1140

ccccggtgag tgcctacggc agggcctcca gcaggaatgt cttaatctag ggggtggggt    1200

cgacatgggg agagatctat ggctgtggct gttcaggacc ccagggggtt tctgtgccaa    1260

cagttatgta atgattagcc ctccagagag gaggcagaca gcccatttca tcccaaggag    1320

tcagagccac agagcgctga agcccacagt gctccccagc aggagctgct cctatcctgg    1380

tcattattgt cattatggtt aatgaggtca gaggtgaggg caaacccaag gaaacttggg 1440

gcctgcccaa ggcccagagg aagtgcccag gcccaagtgc caccttctgg caggactttc 1500

ctctggcccc acatggggtg cttgaattgc agaggatcaa ggaagggagg ctacttggaa 1560

tggacaagga cctcaggcac tccttcctgc gggaagggag caaagtttgt ggccttgact 1620

ccactccttc tgggtgccca gagacgacct cagcccagct gccctgctct gccctgggac 1680

caaaaaggca ggcgtttgac tgcccagaag gccaacctca ggctggcact taagtcaggc 1740

ccttgactct ggctgccact ggcagagcta tgcactcctt ggggaacacg tgggtggcag 1800

cagcgtcacc tgacccaggt cagtgggtgt gtcctggagt gggcctcctg gcctctgagt 1860

tctaagaggc agtagagaaa catgctggtg cttccttccc ccacgttacc cacttgcctg 1920

gactcaagtg ttttttattt ttcttttttt aaaggaaact tcctgtgcaa cccagattat 1980

cacctttgaa agtttcaaag agaacctgaa ggactttctg cttgtcatcc cctttgactg 2040

ctgggagcca gtccaggagt gagaccggcc agatgaggct ggccaagccg gggagctgct 2100

ctctcatgaa acaagagcta gaaactcagg atggtcatct tggagggacc aaggggtggg 2160

ccacagccat ggtgggagtg gcctggacct gccctgggcc acactgaccc tgatacaggc 2220

atggcagaag aatgggaata ttttatactg acagaaatca gtaatattta tatatttata 2280

tttttaaaat atttatttat ttatttattt aagttcatat tccatattta ttcaagatgt 2340

tttaccgtaa taattattat taaaaatatg cttct 2375

<210> 6
<211> 781
<212> DNA
<213> Homo sapiens

<400> 6
acacagagag aaaggctaaa gttctctgga ggatgtggct gcagagcctg ctgctcttgg 60

gcactgtggc ctgcagcatc tctgcacccg cccgctcgcc cagccccagc acgcagccct 120

gggagcatgt gaatgccatc caggaggccc ggcgtctcct gaacctgagt agagacactg 180

ctgctgagat gaatgaaaca gtagaagtca tctcagaaat gtttgacctc caggagccga 240

cctgcctaca gacccgcctg gagctgtaca agcagggcct gcggggcagc ctcaccaagc 300

tcaagggccc cttgaccatg atggccagcc actacaagca gcactgccct ccaaccccgg 360

aaacttcctg tgcaacccag attatcacct ttgaaagttt caaagagaac ctgaaggact 420

ttctgcttgt catccccttt gactgctggg agccagtcca ggagtgagac cggccagatg 480

aggctggcca agccggggag ctgctctctc atgaaacaag agctagaaac tcaggatggt 540

catcttggag ggaccaaggg gtgggccaca gccatggtgg gagtggcctg gacctgccct 600

gggccacact gaccctgata caggcatggc agaagaatgg gaatatttta tactgacaga 660

aatcagtaat atttatatat ttatattttt aaaatattta tttatttatt tatttaagtt 720

catattccat atttattcaa gatgttttac cgtaataatt attattaaaa atatgcttct 780

a 781

<210> 7
<211> 144
<212> PRT
<213> Homo sapiens

<400> 7
Met Trp Leu Gln Ser Leu Leu Leu Leu Gly Thr Val Ala Cys Ser Ile
1               5                   10                  15

Ser Ala Pro Ala Arg Ser Pro Ser Pro Ser Thr Gln Pro Trp Glu His
            20                  25                  30

Val Asn Ala Ile Gln Glu Ala Arg Arg Leu Leu Asn Leu Ser Arg Asp
        35                  40                  45

Thr Ala Ala Glu Met Asn Glu Thr Val Glu Val Ile Ser Glu Met Phe
    50                  55                  60

Asp Leu Gln Glu Pro Thr Cys Leu Gln Thr Arg Leu Glu Leu Tyr Lys
65                  70                  75                  80

Gln Gly Leu Arg Gly Ser Leu Thr Lys Leu Lys Gly Pro Leu Thr Met
                85                  90                  95

Met Ala Ser His Tyr Lys Gln His Cys Pro Pro Thr Pro Glu Thr Ser
            100                 105                 110

Cys Ala Thr Gln Ile Ile Thr Phe Glu Ser Phe Lys Glu Asn Leu Lys
        115                 120                 125

Asp Phe Leu Leu Val Ile Pro Phe Asp Cys Trp Glu Pro Val Gln Glu
    130                 135                 140

<210> 8
<211> 22
<212> RNA
<213> Homo sapiens

<400> 8
uggaauguaa agaaguaugu au 22

<210> 9
<211> 22
<212> RNA
<213> Homo sapiens

<400> 9
uggaauguaa agaaguaugu au                                                22

<210> 10
<211> 22
<212> RNA
<213> Homo sapiens

<400> 10
uggaauguaa ggaagugugu gg                                                22

<210> 11
<211> 22
<212> RNA
<213> Homo sapiens

<400> 11
uuuggucccc uucaaccagc ug                                                22

<210> 12
<211> 22
<212> RNA
<213> Homo sapiens

<400> 12
uuuggucccc uucaaccagc ug                                                22

<210> 13
<211> 22
<212> RNA
<213> Homo sapiens

<400> 13
uuuggucccc uucaaccagc ua                                                22

## Description

### Related Patent Application

This application claims benefit under 35 U.S.C. §119(e) of the following U.S. Provisional Application Nos. 61/331,166 filed May 4, 2010 and 61/331,171 filed May 4, 2010. Each of the above listed applications is incorporated by reference herein in its entirety for all purposes.

### Field

Technology described herein relates in part to optimized treatments of degenerative muscle diseases, such as, for example, an inflammatory myopathy or myositis (e.g., inclusion body myositis).

### Background

A therapeutic drug can be cleared at different rates and modified in different manners in different subjects. This variability can result in varying effects of a drug in different subjects when treating a muscle degenerative disease. A number of degenerative muscle diseases exist. Examples of muscle diseases include, without limitation, muscular dystrophies, inflammatory myopathies, and congenital myopathies.

### Summary

Featured herein are personalized treatments of degenerative muscle diseases that optimize the therapeutic effect of a drug and reduce the likelihood of delivering a toxic dose. Thus, provided herein are methods comprising: (a) administering an immunosuppressive drug to a subject having an inflammatory myopathy; (b) identifying or determining the presence, absence or amount of at least one biomarker in the subject, where the biomarker is selected from the group consisting of colony-stimulating factor 2 (CFS2) transcript or polypeptide, NF-kappa-B-modulating pro-inflammatory cytokine transcript or polypeptide, microRNA-1, microRNA-133, microRNA-206 or a portion of the foregoing; and (c) maintaining a subsequent dosage of the drug or adjusting a subsequent dosage of the drug administered to the subject based on the presence, absence or amount of the biomarker identified in the subject. CFS2 is also commonly known as granulocyte macrophage colony-stimulating factor, abbreviated GM-CSF. CFS2 and GM-CSF are used interchangeably to refer to the human gene, protein, and mRNA.

Also provided herein are methods comprising: (a) administering an immunosuppressive drug to a subject having an inflammatory myopathy; (b) identifying or determining the presence, absence or amount of at least one biomarker in the subject, where the biomarker is selected from the group consisting of granulocyte macrophage colony-stimulating factor (GM-CSF) transcript or polypeptide, NF-kappa-B-modulating pro-inflammatory cytokine transcript or polypeptide, microRNA-1, microRNA-133, microRNA-206 or a portion of the foregoing; and (c) determining whether the dosage of the drug subsequently administered to the subject is adjusted based on the presence, absence or amount of the biomarker identified in the subject.

Provided also herein are methods comprising: (a) identifying or determining the presence, absence or amount of at least one biomarker in a subject having an inflammatory myopathy to whom an immunosuppressive drug has been administered, where the biomarker is selected from the group consisting of granulocyte macrophage colony-stimulating factor (GM-CSF) transcript or polypeptide, NF-kappa-B-modulating pro-inflammatory cytokine transcript or polypeptide, microRNA-1, microRNA-133, microRNA-206 or a portion of the foregoing; and (b) maintaining a subsequent dosage of the drug or adjusting a subsequent dosage of the drug administered to the subject based on the presence, absence or amount of the biomarker identified in the subject.

Also provided herein are methods comprising: (a) identifying or determining the presence, absence or amount of at least one biomarker in a subject having an inflammatory myopathy to whom an immunosuppressive drug has been administered, where the biomarker is selected from the group consisting of granulocyte macrophage colony-stimulating factor (GM-CSF) transcript or polypeptide, NF-kappa-B-modulating pro-inflammatory cytokine transcript or polypeptide, microRNA-1, microRNA-133, microRNA-206 or a portion of the foregoing; and (b) determining whether the dosage of the drug subsequently administered to the subject is adjusted based on the presence, absence or amount of the biomarker identified in the subject.

Provided also herein are methods comprising: (a) receiving information comprising the presence, absence or amount of a biomarker in a subject having an inflammatory myopathy to whom an immunosuppressive drug has been administered, where the biomarker is selected from the group consisting of granulocyte macrophage colony-stimulating factor (GM-CSF) transcript or polypeptide, NF-kappa-B-modulating pro-inflammatory cytokine transcript or polypeptide, microRNA-1, microRNA-133, microRNA-206 or a portion of the foregoing; and (b) maintaining a subsequent dosage of the drug or adjusting a subsequent dosage of the drug administered to the subject based on the presence, absence or amount of the biomarker identified in the subject.

Also provided herein are methods comprising: (a) identifying or determining the presence, absence or amount of at least one biomarker in a subject having an inflammatory myopathy to whom an immunosuppressive drug has been administered, where the biomarker is selected from the group consisting of granulocyte macrophage colony-stimulating factor (GM-CSF) transcript or polypeptide, NF-kappa-B-modulating pro-inflammatory cytokine transcript or polypeptide, microRNA-1, microRNA-133, microRNA-206 or a portion of the foregoing; and (b) transmitting the presence, absence or amount of the biomarker to a decision maker who maintains a subsequent dosage of the drug or adjusts a subsequent dosage of the drug administered to the subject based on the presence, absence or amount of the biomarker identified in the subject.

Provided also herein are methods comprising: (a) identifying or determining the presence, absence or amount of at least one biomarker in a subject having an inflammatory myopathy to whom an immunosuppressive drug has been administered, where the biomarker is selected from the group consisting of granulocyte macrophage colony-stimulating factor (GM-CSF) transcript or polypeptide, NF-kappa-B-modulating pro-inflammatory cytokine transcript or polypeptide, microRNA-1, microRNA-133, microRNA-206 or a portion of the foregoing; and (b) transmitting an indication to maintain a subsequent dosage of the drug or adjust a subsequent dosage of the drug administered to the subject based on the presence, absence or amount of the biomarker identified in the subject.

Additionally presented herein are methods for optimizing therapeutic efficacy of a treatment of an inflammatory myopathy, comprising: administering an immunosuppressive drug to a subject having an inflammatory myopathy, and identifying or determining the presence, absence or amount of at least one biomarker in the subject, where the biomarker is selected from the group consisting of granulocyte macrophage colony-stimulating factor (GM-CSF) transcript or polypeptide, NF-kappa-B-modulating pro-inflammatory cytokine transcript or polypeptide, microRNA-1, microRNA-133, microRNA-206 or a portion of the foregoing, and maintaining a subsequent dosage of the drug or adjusting a subsequent dosage of the drug administered to the subject based on the presence, absence or amount of the biomarker identified in the subject.

In certain embodiments provided are methods that comprise: reducing toxicity of a treatment of an inflammatory myopathy by administering an immunosuppressive drug to a subject having an inflammatory myopathy and identifying or determining the presence, absence or amount of a biomarker in the subject, where the biomarker is selected from the group consisting of granulocyte macrophage colony-stimulating factor (GM-CSF) transcript or polypeptide, NF-kappa-B-modulating pro-inflammatory cytokine transcript or polypeptide, microRNA-1, microRNA-133, microRNA-206 or a portion of the foregoing, and maintaining a subsequent dosage of the drug or adjusting a subsequent dosage of the drug administered to the subject based on the presence, absence or amount of the biomarker identified in the subject. Also provided herein are methods that comprise: administering an immunosuppressive drug to a subject having an inflammatory myopathy, and identifying the amount of a granulocyte macrophage colony-stimulating factor (GM-CSF) transcript or polypeptide or portion thereof in the subject, and maintaining a subsequent dosage of the drug or adjusting a subsequent dosage of the drug administered to the subject based on the amount of the GM-CSF transcript or polypeptide or portion thereof identified in the subject.

Further presented herein are methods comprising: administering an immunosuppressive drug to a subject having an inflammatory myopathy and identifying the amount of a tumor necrosis factor-alpha (TNF-alpha) transcript or polypeptide or portion thereof in the subject, and maintaining a subsequent dosage of the drug or adjusting a subsequent dosage of the drug administered to the subject based on the amount of the TNF-alpha identified in the subject.

In some embodiments methods comprise administering an immunosuppressive drug to a subject having an inflammatory myopathy, and identifying the amount of a microRNA-133 or portion thereof in the subject and maintaining a subsequent dosage of the drug or adjusting a subsequent dosage of the drug administered to the subject based on the amount of the microRNA-133 or portion thereof identified in the subject. In certain embodiments the microRNA-133 is one or more of microRNA-133a-1, microRNA-133a-2 and mircroRNA-133b. Such methods may further comprise identifying the presence, absence or amount of one or more of a microRNA-1 and microRNA-206 or portion thereof and determining whether the dosage of the drug is adjusted for the subject based on the presence, absence or amount of the one or more of the microRNA-1 and microRNA-206 or portion thereof. In such embodiments the microRNA-1 is microRNA-1-1, microRNA-1-2 or microRNA-1-1 and microRNA-1-2.

In certain embodiments the inflammatory myopathy is a myositis. In some embodiments the myositis is one or more of inclusion body myositis (IBM), dermatomyositis (DM), polymyositis (PM), and juvenile myositis (JM). The subject is human in some embodiments.

In certain embodiments a subject having inflammatory myopathy has levels of GM-CSF transcript or polypeptide elevated relative to healthy subjects prior to administration of the immunosuppressive agent. In some embodiments a subject having inflammatory myopathy has levels of TNF-alpha transcript or polypeptide elevated relative to healthy subjects prior to administration of the immunosuppressive agent. In certain embodiments a subject having inflammatory myopathy has levels of microRNA-1, microRNA-133 or microRNA-206 reduced relative to healthy subjects prior to administration of the immunosuppressive agent. In certain embodiments the microRNA-133 is one or more of: microRNA-133a-1; microRNA-133a-2; and mircroRNA-133b. In some embodiments the microRNA-1 is microRNA-1-1, microRNA-1-2 or microRNA-1-1 and microRNA-1-2.

In some embodiments the presence, absence or amount of the biomarker is determined from a biological sample from the subject. In some embodiments, a sample contains one or more of blood or a blood fraction; a muscle biopsy product; and one or more biomarkers of portion thereof described herein.

In some embodiments, a biomarker is GM-CSF. As herein presented, the presence, absence or amount of the GM-CSF transcript or polypeptide or portion thereof may be determined by a method that comprises one or more of: contacting the GM-CSF polypeptide or portion thereof with an antibody that specifically binds to the GM-CSF polypeptide or portion thereof; analyzing the GM-CSF polypeptide or portion thereof by high performance liquid chromatography; and analyzing the GM-CSF polypeptide or portion thereof by mass spectrometry.

In some embodiments the biomarker is a NF-kappa-B-modulating pro-inflammatory cytokine polypeptide or portion thereof. In certain embodiments the presence, absence or amount of the NF-kappa-B-modulating pro-inflammatory cytokine polypeptide or portion thereof is determined by a method that comprises one or more of; contacting a sample of the subject with an antibody that specifically binds to the NF-kappa-B-modulating pro-inflammatory cytokine polypeptide or portion thereof; analyzing the NF-kappa-B-modulating pro-inflammatory cytokine polypeptide or portion thereof by high performance liquid chromatography; and analyzing the NF-kappa-B-modulating pro-inflammatory cytokine polypeptide or portion thereof by mass spectrometry.

In certain embodiments the biomarker is the NF-kappa-B-modulating pro-inflammatory cytokine transcript or portion thereof. In some embodiments the NF-kappa-B-modulating pro-inflammatory cytokine may be chosen from tumor necrosis factor-alpha (TNF-alpha), interferon alpha (IFN α), IL-1beta, IL-6, chemokines monocyte chemoattractant protein-1 (CCL-2), IL-8, and the AP-1-dependent basic fibroblast growth factor. In such embodiments the presence, absence or amount of the NF-kappa-B-modulating pro-inflammatory cytokine transcript or portion thereof is determined by a method that comprises one or more of: contacting a sample of the subject with a nucleic acid substantially complementary to the NF-kappa-B-modulating pro-inflammatory cytokine transcript or portion thereof; and when the nucleic acid substantially complementary to the TNF- alpha transcript or portion thereof is in a nucleic acid array.

In some embodiments the biomarker is one or more of the microRNA-1, microRNA-133, microRNA-206 or portion of the foregoing. In such embodiments the presence, absence or amount of the one or more of the microRNA-1, microRNA-133, microRNA-206 or portion thereof is determined by a method that comprises one or more of: contacting a sample of the subject with a nucleic acid substantially complementary to the microRNA-1, microRNA-133, microRNA-206 or portion thereof; and when the nucleic acid substantially complementary to the microRNA-1, microRNA-133, microRNA-206 or portion thereof is in a nucleic acid array configured for polymerase chain reaction (PCR) or real-time polymerase chain reaction (RT-PCR).

As used herein, "a portion" of the biomarker is any fragment or portion thereof that sufficient to allow identification or determination by a suitable method.

Featured herein are therapeutic treatments of degenerative muscle diseases that mediate the levels of triggering molecules, including inflammatory cytokines for example. In some embodiments, the treatments reduce or inhibit the levels of triggering molecules. Also presented herein are personalized medicine methods that optimize the therapeutic effect of a drug and reduce the likelihood of delivering a toxic dose. In some embodiments, the drug is an immunosuppressive drug.

Provided also herein are methods for treating an inflammatory myopathy. In certain embodiments, provided are methods comprising administering a pharmaceutical composition in an amount effective to reduce the amount of granulocyte macrophage colony-stimulating factor (GM-CSF) in the subject. In various embodiments one or more drugs may be administered to a subject, which drugs include, without limitation, an immunosuppressive drug, an antibody, a siNA (short inhibitory nucleic acid), an anti-inflammatory agent, an antibiotic agent, an anti-viral agent, a steroid agent, and a chemotherapy agent, provided that the one or more drugs reduce the amount of GM-CSF in the subject.

In certain embodiments the pharmaceutical composition comprises a nucleic acid composition. The pharmaceutical composition may sometimes comprise a microRNA or consist of a microRNA. In some embodiments the microRNA is one or more of microRNA-1, microRNA-133, microRNA-206, microRNA133a-1, microRNA133a-2, microRNA-1-1, microRNA-1-2 or any combination thereof. In various embodiments the nucleic acid composition comprises or consists of a siNA.

Also as presented herein the method may comprise identifying or determining the presence, absence or amount of at least one biomarker in the subject. The biomarker may be selected from the group consisting of granulocyte macrophage colony-stimulating factor (GM-CSF) transcript or polypeptide, tumor necrosis factor alpha (TNFα), NF-kappa-B-modulating pro-inflammatory cytokine transcript or polypeptide, microRNA-1, microRNA-133, microRNA-206 or a portion of the foregoing. In various embodiments the method comprises administering and/or maintaining a subsequent dosage of the drug or adjusting a subsequent dosage of the drug administered to the subject based on the presence, absence or amount of the biomarker identified in the subject.

In certain embodiments the subject having the inflammatory myopathy is identified as having levels of GM-CSF transcript or polypeptide elevated relative to healthy subjects prior to administration of the immunosuppressive agent to the subject. In some embodiments the subject having the inflammatory myopathy is identified as having levels of TNF-alpha transcript or polypeptide elevated relative to healthy subjects prior to administration of the immunosuppressive agent to the subject. The subject having the inflammatory myopathy may sometimes be identified as having levels of microRNA-1, microRNA-133, and/or microRNA-206 reduced relative to healthy subjects prior to administration of the nucleic acid composition to the subject. In various embodiments the inflammatory myopathy is a myositis including but not limited to inclusion body myositis (IBM), dermatomyositis (DM) or polymyositis (PM).

Also presented herein is a method for treating an inflammatory myopathy in a subject, comprising administering a pharmaceutical composition in an amount effective to reduce the amount of tumor necrosis factor-alpha (TNF-alpha) transcript or polypeptide in the subject. In various embodiments the method comprises administering an immunosuppressive drug, an antibody, an siNA, an anti-inflammatory agent, an antibiotic agent, an anti-viral agent, a steroid agent or a chemotherapy agent to the subject.

In various embodiments the pharmaceutical composition comprises a nucleic acid. The pharmaceutical composition may comprise or consist of at least one microRNA. In certain embodiments the microRNA may be one or more of microRNA-1, microRNA-133, microRNA-206, microRNA133a-1, microRNA133a-2, microRNA-1-1, microRNA-1-2 or any combination thereof. In some embodiments the pharmaceutical composition comprises or consists of a siNA.

The method may sometimes comprise: (a) identifying or determining the presence, absence or amount of at least one biomarker in the subject, wherein the biomarker is selected from the group consisting of granulocyte macrophage colony-stimulating factor (GM-CSF) transcript or polypeptide, tumor necrosis factor alpha (TNF-alpha), NF-kappa-B-modulating pro-inflammatory cytokine transcript or polypeptide, microRNA-1, microRNA-133, microRNA-206 or a portion of the foregoing; and (b) maintaining a subsequent dosage of the drug or adjusting a subsequent dosage of the drug administered to the subject based on the presence, absence or amount of the biomarker identified in the subject.

In certain embodiments the subject having the inflammatory myopathy is identified as having levels of GM-CSF transcript or polypeptide elevated relative to healthy subjects prior to administration of the immunosuppressive agent to the subject. In some embodiments the subject having the inflammatory myopathy is identified as having levels of TNF-alpha transcript or polypeptide elevated relative to healthy subjects prior to administration of the immunosuppressive agent to the subject. In various embodiments the subject having the inflammatory myopathy is identified as having levels of microRNA-1, microRNA-133 and/or microRNA-206 reduced relative to healthy subjects prior to administration of the nucleic acid composition to the subject. As presented herein the inflammatory myopathy may be a myositis including but not limited to inclusion body myositis (IBM), dermatomyositis (DM) and polymyositis (PM).

In various embodiments a method for treating an inflammatory myopathy in a subject comprises administering a pharmaceutical composition in an amount effective to modulate the amount of microRNA-1 in the subject. In some embodiments the method comprises administering a pharmaceutical composition in an amount effective to modulate the amount of microRNA-133 in the subject. The method may sometimes comprise administering a pharmaceutical composition in an amount effective to modulate the amount of microRNA-206 in the subject. As presented herein the pharmaceutical composition may comprise a microRNA including but not limited to microRNA-1, microRNA-133 and microRNA-206.

Provided in some embodiments is a method for differentiating myoblasts, comprising contacting myoblasts with a composition that reduces the amount of active TNF-alpha in the myoblasts, which composition is in an amount effective to differentiate the myoblasts to myocytes and/or myotubes. In some embodiments, the composition comprises an siRNA against a TNF-alpha transcript, and sometimes the composition comprises an antibody that neutralizes TNF-alpha. Provided also in some embodiments is a method for differentiating myoblasts, comprising contacting myoblasts with a composition that reduces the amount of active GM-CSF in the myoblasts, which composition is in an amount effective to differentiate the myoblasts to myocytes and/or myotubes. In some embodiments, the composition comprises an siRNA against a GM-CSF transcript, and sometimes the composition comprises an antibody that neutralizes GM-CSF. Provided also in certain embodiments is a method for differentiating myoblasts, comprising contacting myoblasts with a composition that increases the amount of miRNA-1, miRNA-133 and/or miRNA-206 in the myoblasts, which composition is in an amount effective to differentiate the myoblasts to myocytes and/or myotubes. In some embodiments, the composition comprises the miRNA-1, miRNA-133 and/or miRNA-206 and sometimes the composition comprises one or more nucleic acids that encode the miRNA-1, miRNA-133 and/or miRNA-206. In some embodiments, the myoblasts express increased levels of GM-CSF and/or TNF-alpha, which, without being limited by theory, blocks differentiation of the myoblasts to myocytes and/or myotubes. In certain embodiments, contacting the myoblasts with the composition restores differentiation of such myoblasts. In certain embodiments, the myoblasts are in vitro, and in some embodiments the myoblasts are in vitro and then inserted into a subject after the myoblasts are contacted with the composition. In certain embodiments the myoblasts are in a subject and the composition is administered to the subject. In the latter embodiments, a method sometimes includes administering an immunosuppressive drug to the subject, administering an antibody to the subject, administering an siNA to the subject, administering an anti-inflammatory agent to a subject, administering an antibiotic agent to a subject, administering an anti-viral agent to a subject, administering a steroid agent to a subject and/or administering a chemotherapy agent to a subject. In certain embodiments, the microRNA-133 is one or more of microRNA133a-1 and microRNA133a-2, and sometimes the microRNA-1 is one or more of microRNA-1-1 and microRNA-1-2. In some embodiments, a method comprises (a) identifying the presence, absence or amount of a biomarker in the myoblasts, myocytes and/or myotubes, wherein the biomarker is selected from the group consisting of granulocyte macrophage colony-stimulating factor (GM-CSF) polypeptide, tumor necrosis factor alpha (TNH-alpha), NF-kappa-B-modulating pro-inflammatory cytokine transcript or polypeptide, microRNA-1, microRNA-133, microRNA-206 or a portion of the foregoing; and (b) maintaining a subsequent dosage of the composition or adjusting a subsequent dosage of the composition contacting the myoblasts based on the presence, absence or amount of the biomarker identified in the subject. In certain embodiments, the myoblasts are from a subject diagnosed with an inflammatory myopathy, sometimes the inflammatory myopathy is a myositis, and in some embodiments, the myositis is inclusion body myositis (IBM), dermatomyositis (DM) or polymyositis (PM).

The presence, absence or amount of a biomarker may be determined from any suitable biological sample from the subject by any suitable method. A biological sample can include without limitation a blood fraction and a biopsy product. In various embodiments as presented herein the subject is human.

As presented herein the biomarker may be a GM-CSF polypeptide or portion thereof. In some embodiments the presence, absence or amount of the GM-CSF polypeptide or portion thereof may be determined by a method that comprises contacting the GM-CSF polypeptide or portion thereof with an antibody that specifically binds to the GM-CSF polypeptide or portion thereof. In certain embodiments the presence, absence or amount of the GM-CSF polypeptide or portion thereof is determined by a method that comprises analyzing the GM-CSF polypeptide or portion thereof by high performance liquid chromatography. The presence, absence or amount of the GM-CSF polypeptide or portion thereof may sometimes be determined by a method that comprises analyzing the GM-CSF polypeptide or portion thereof by mass spectrometry.

As presented herein the biomarker may be a GM-CSF transcript or portion thereof.
In some embodiments the presence, absence or amount of the GM-CSF transcript or portion thereof is determined by a method that comprises contacting a sample of the subject with a nucleic acid substantially complementary to the TNF-alpha transcript or portion thereof. The nucleic acid substantially complementary to the TNF-alpha transcript or portion thereof may sometimes be in a nucleic acid array.

As presented herein the biomarker may be a TNF-alpha polypeptide or a portion thereof.
The presence, absence or amount of the TNF-alpha polypeptide or portion thereof may sometimes be determined by a method that comprises contacting a sample of the subject with an antibody that specifically binds to the TNF-alpha polypeptide or portion thereof. In certain embodiments the presence, absence or amount of the TNF-alpha polypeptide or portion thereof is determined by a method that comprises analyzing the TNF-alpha polypeptide or portion thereof by high performance liquid chromatography.

In various embodiments the presence, absence or amount of the TNF-alpha polypeptide or portion thereof is determined by a method that comprises analyzing a nucleic acid that binds to the TNF-alpha polypeptide or portion thereof by PCR or real-time PCR. The presence, absence or amount of the TNF-alpha polypeptide or portion thereof may sometimes be determined by a method that comprises analyzing the TNF-alpha polypeptide or portion thereof by mass spectrometry.

As presented herein the biomarker may be a TNF-alpha transcript or portion thereof.
In some embodiments the presence, absence or amount of the TNF-alpha transcript or portion thereof is determined by a method that comprises contacting a sample of the subject with a nucleic acid substantially complementary to the TNF-alpha transcript or portion thereof. The nucleic acid substantially complementary to the TNF-alpha transcript or portion thereof may sometimes be in a nucleic acid array.

As presented herein the biomarker may be one or more of microRNA-1, microRNA-133, microRNA-206 or portion thereof. In certain embodiments the presence, absence or amount of the one or more of the microRNA-1, microRNA-133, microRNA-206 or portion thereof may be determined by a method that comprises contacting a sample of the subject with a nucleic acid substantially complementary to the microRNA-1, microRNA-133, microRNA-206 or portion thereof. The nucleic acid substantially complementary to microRNA-1, microRNA-133, microRNA-206 or portion thereof may sometimes be in a nucleic acid array.

Certain embodiments are described further in the following description, examples, claims and drawings.

### Brief Description of the Drawings

The drawings illustrate embodiments of the technology and are not limiting. For clarity and ease of illustration, the drawings are not made to scale and, in some instances, various aspects may be shown exaggerated or enlarged to facilitate an understanding of particular embodiments.

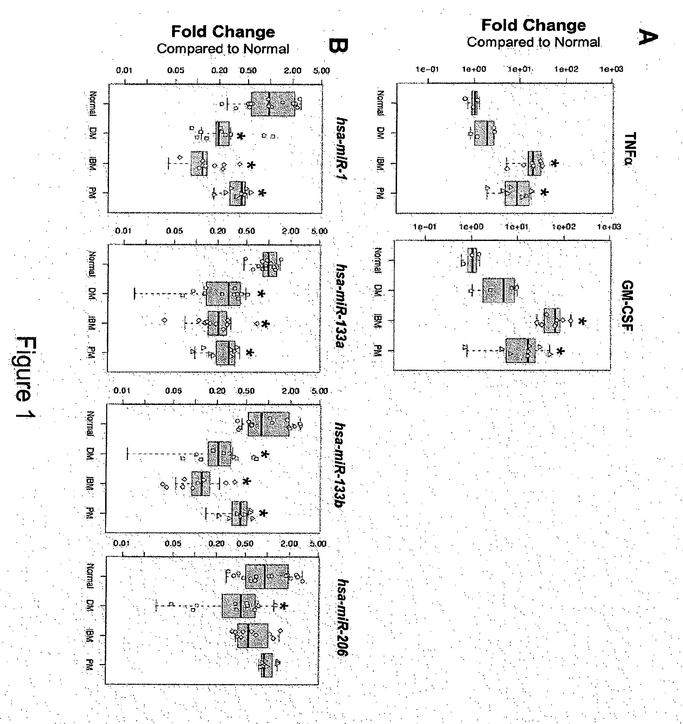

Figure 1. In myositis, and predominantly in IBM, patient muscle TNF-alpha and GM-CSF are overexpressed, where *miR-1, miR-133a, miR-133b,* and *miR-206* are under expressed. (A) TNF-alpha and GM-CSF were over-expressed and (B) microRNAs miR-1, -133a, -133b, and -206 were underexpressed in myositis muscle biopsies as compared to muscle biopsies of normal donors. TNF-alpha, GM-CSF, and reference mRNAs were measured by TaqMan qRT-PCR. MicroRNAs and reference small RNAs were measured by TLDA array as described in Methods. Results are shown as fold change as compared to median of the normal donor controls.

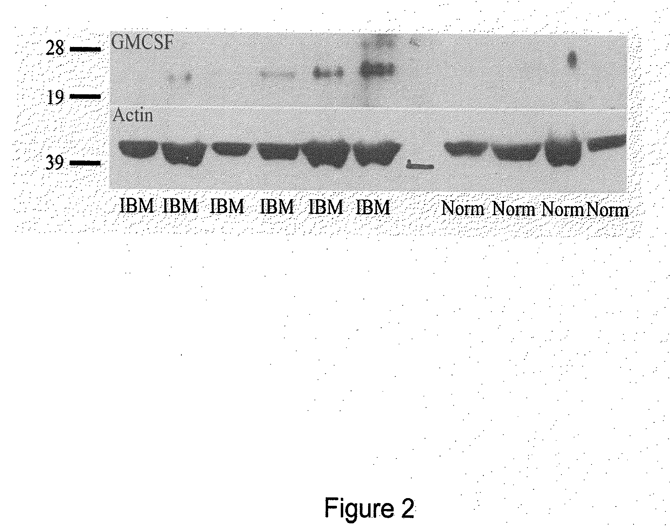

**Figure 2****. GM-CSF protein was over-expressed in IBM muscle biopsies compared to normal donor muscle biopsies**. Muscle from 6 IBM and 4 normal donors were prepared and probed as previously described (Ann Neurol (2009) 67:53)]. Briefly, 10 mg of muscle was Dounce homogenized in 100 microliters of lysis buffer (2% SDS, 20mM Tris buffer pH 7.6 and Roche Complete Protease Inhibitor) and centrifuged at 10,000 x g for 10 minutes at 4°C. The supernatant was removed and after heating for 10 min at 72°C in the presence of 10mM DTT, 30 micrograms was loaded in individual wells of 4-12% NuPAGE Novex Bis-Tris Gels. Proteins were separated using SDS-PAGE and transferred to a nitrocellulose membrane. Blots were incubated overnight at 4° C with goat anti-human GMCSF (1:200 dilution; cat# BAF215, R&D Systems), and after washing, were incubated for 2 hours at room temperature with bovine anti-goat IgG-HRP (1:1000 dilution; cat# SC2352, Santa Cruz Biotechnology). Blots were developed using the SuperSignal West Pico kit (cat# 34077, Pierce Biotechnology, 5 min, room temperature).

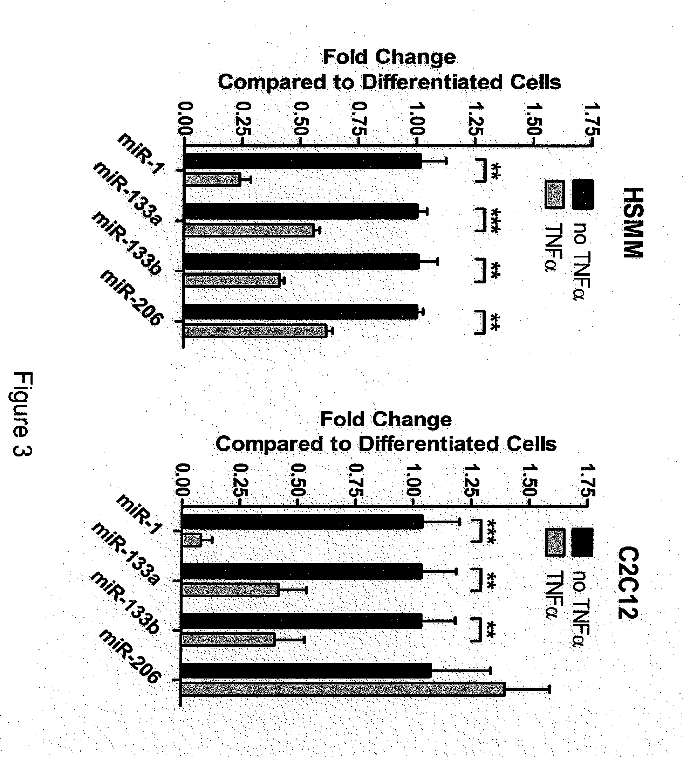

**Figure 3****. TNF-alpha blocked expression of microRNA-1, -133, and -206 in Human myoblasts and microRNA-1 and -133 mouse myoblasts**. HSMM and C2C12 cells were plated at 8x10⁴ cells per well in 6 well plates in normal media. The next day media was replaced with differentiation media +/- TNF-alpha (10 ng/ml). MicroRNA expression was measured by TaqMan qRT-PCR, and normalized to mean expression of sno135, sno202, and sno429 RNAs. T-tests were used to determine statistical significance. NS signifies "not significant", * a p-value < 0.05, ** a p-value <0.01, *** a p-value <0.001.

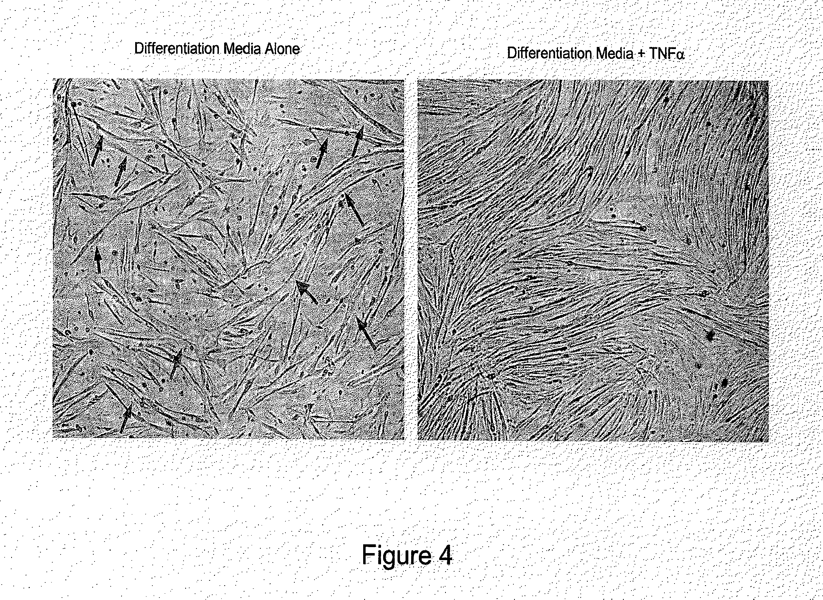

Figure 4. TNF-alpha blocked the differentiation of HSMM myoblasts to myocytes/myotubes. Shown are photos of HSMM at day 4 after exposure to differentiation media with or without TNF-alpha (10 ng/ml). Cells treated with differentiation media alone show lower cell density and have fused to long multi-nucleated cells indicative of myocyte/myotubes. Cells also treated with TNF-alpha display short single-cell morphology indicative of undifferentiated myoblasts.

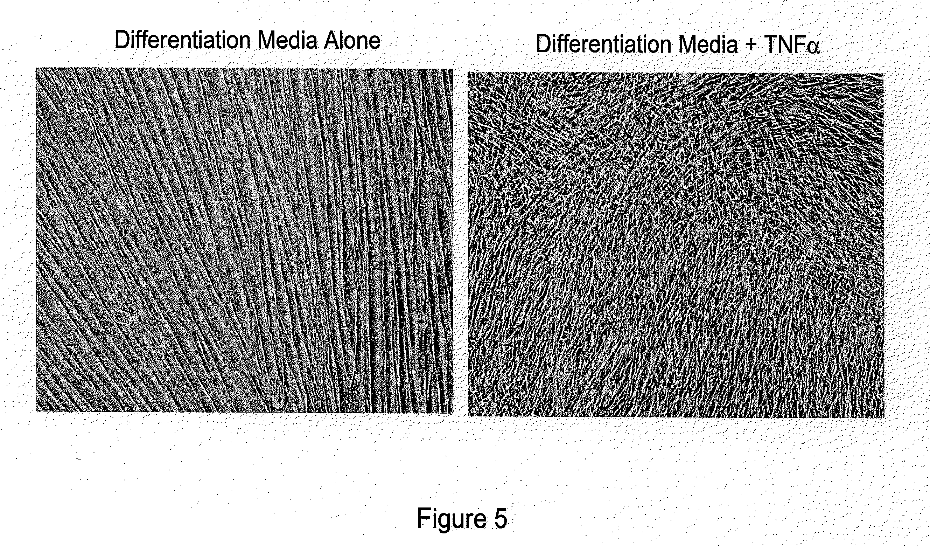

**Figure 5****. TNF-alpha blocked the differentiation of C2C12 myoblasts to myocytes/myotubes.** Shown are photos of C2C12 at day 7 after exposure to differentiation media with or without TNF-alpha (10 ng/ml). Cells treated with differentiation media alone show lower cell density and have fused to long multi-nucleated cells indicative of myocyte/myotubes. Cells also treated with TNF-alpha display short single-cell morphology indicative of undifferentiated myoblasts.

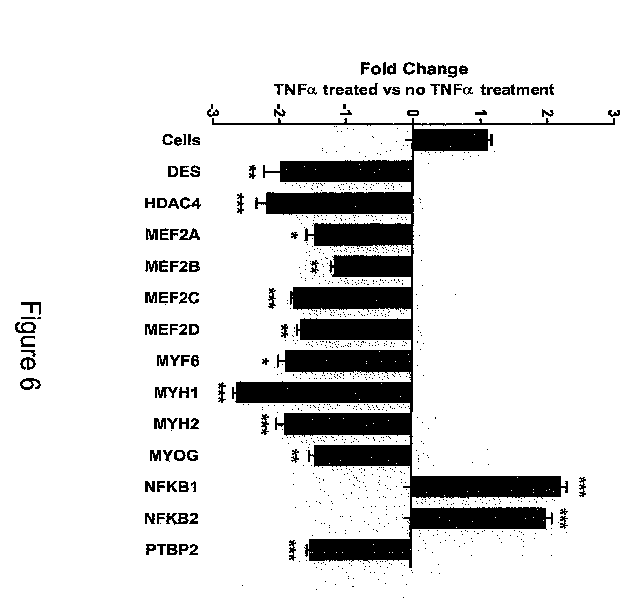

**Figure 6****. TNF-alpha decreased myocyte/myotube marker gene expression, and increased myoblast marker gene expression in HSMM cells.** Myocyte-associated gene expression of HSMM cells at day 4 post treatment with differentiation media +/- TNF-alpha (10 ng/ml) was examined. Shown is the fold change in gene expression of TNF-alpha treated HSMM cells compared to HSMM cells cultured in differentiation media alone. * a p-value < 0.05, ** a p-value <0.01, *** a p-value <0.001.

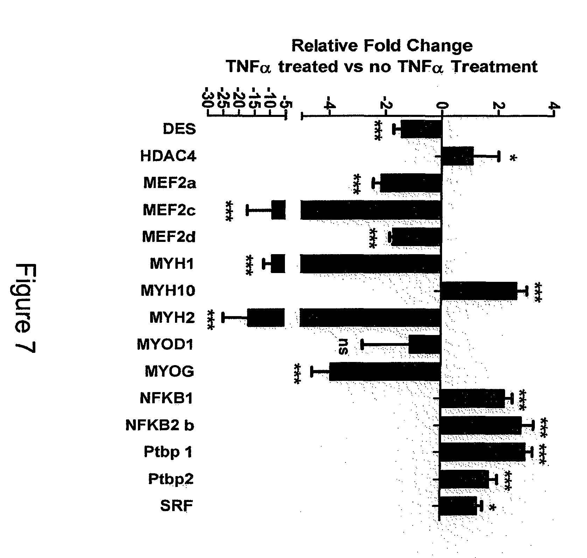

**Figure 7****. TNF-alpha decreased myocyte/myotube marker gene expression, and increased myoblast marker gene expression in C2C12 cells**. Myocyte-associated gene expression of C2C12 cells at day 4 post treatment with differentiation media +/- TNF-alpha (10 ng/ml) was examined. Shown is the fold change in gene expression of TNF-alpha treated C2C12 cells compared to C2C12 cells cultured in differentiation media alone. * a p-value < 0.05, ** a p-value <0.01, *** a p-value <0.001.

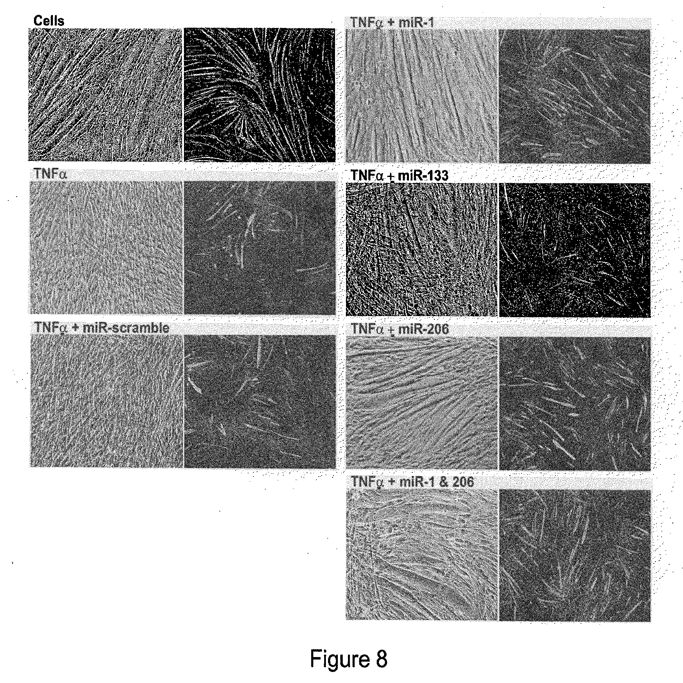

**Figure 8****. MiR-1 or miR-206 restored myoblast-to-myocyte differentiation blocked by TNF-alpha.** C2C12 cells in maintenance media were transfected with miR scrambled control, miR-1, miR-133, or miR-206 at 200 nM, or with miR-1 and miR-206 at 100 nM each. After transfection, cells were cultured in differentiation media alone (Cells group) or differentiation media with TNF-alpha at 20 ng/ml (all other groups). Media +/- TNF-alpha was changed every two days. At day 7 post transfection, cells were photographed (left brightfield panels), and then stained with DAPI and anti-MHY2-FITC (right immunofluorescence panels).

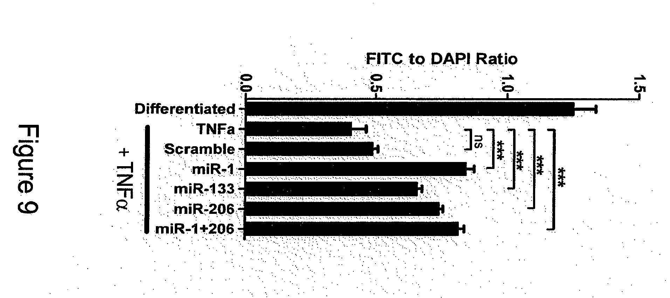

**Figure 9****. miR-1, miR-133, or miR-206 restored MYH2 expression during myoblast-to-myocyte differentiation blocked by TNF-alpha.** C2C12 cells in maintenance media were transfected with miR scrambled control, miR-1, miR-133, or miR-206 at 200 nM, or with miR-1 and miR-206 at 100 nM each. After transfection, cells were cultured in differentiation media alone (Cells group) or differentiation media with TNF-alpha at 20 ng/ml (all other groups). Media +/-TNF-alpha was changed every two days. At day 7 post transfection, cells were stained with DAPI and anti-MYH2-FITC (right immunofluorescence panels in Figure 8). Fluorescent images were captured in quadruplicate from four wells per treatment group using a 4x CFI Plan Fluor ELWD objective. The amount of MYH2 per sample was quantified by dividing the total FITC fluorescence by the total DAPI fluorescence for each image, then calculating an average ratio. NS signifies "not significant", * a p-value < 0.05, ** a p-value <0.01, *** a p-value <0.001.

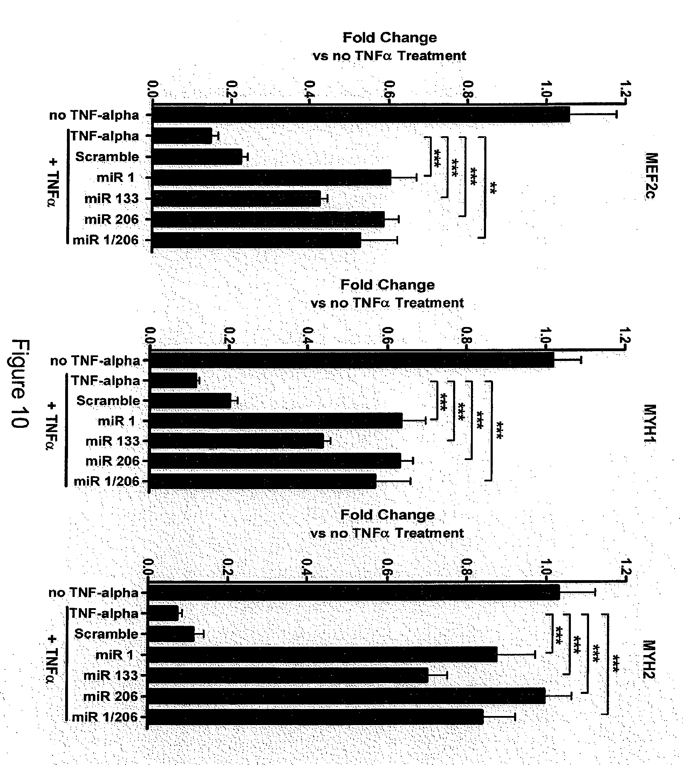

**Figure 10****. Ectopic expression of muscle differentiation miRs-1, miR-133, or miR-206 increased expression of myocyte/myofiber differentiation markers blocked by TNF-alpha.** C2C12 cells were transfected with scrambled control-miR, miR-1, -133, -206 (all at 200 nM), or with a combination of miR-1 and miR-206 (at 100 nm each). 24 hrs post transfection (day 0) cells were washed and exposed to differentiation media with or without TNF-alpha (10 ng/ml). At day 4, cells were lysed, total RNA isolated, and myocyte specific gene expression was measured by RT-PCR. Shown is the fold change ratio in gene expression compared to C2C12 cells differentiated to myocytes in differentiation media alone.

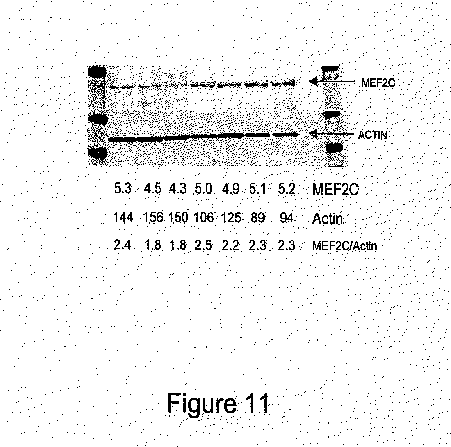

**Figure 11****. Expression of miR-1, -133, or -206 recovered MEF2C protein expression suppressed by TNF-alpha.** C2C12 cells were transfected as described. 24 hrs post transfection (day 0) cells were washed and exposed to differentiation media with or without TNF-alpha (20 ng/ml). At day 7, cells were lysed, protein was isolated, and MEF2C protein levels were measured by Western blot. Beta-actin was used as control. Relative amounts of MEF2C present in each sample were quantified using densitometry and are shown as the MEF2C/beta-actin ratio.

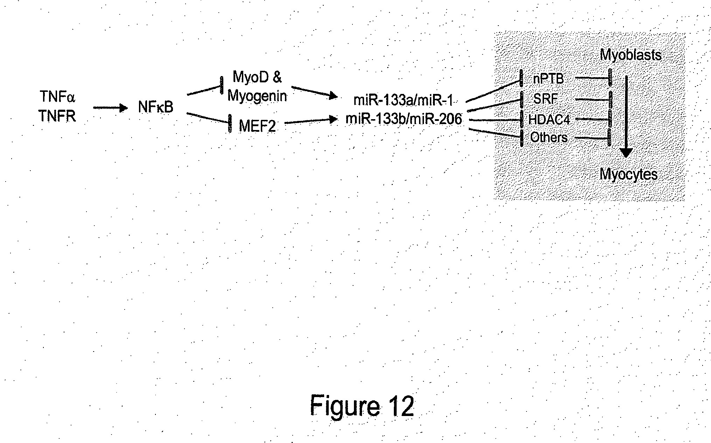

**Figure 12****. Models for the linked auto-immunologic and differentiation effects of TNF-alpha in muscle cells driving the pathology of myositis.** In normal muscle development and skeletal muscle maintenance MYOD, MYOG, and MEF2 drive myoblast-to-myocyte differentiation by driving expression of miR-1, -133, and -206. These three microRNAs then drive the differentiation process by inhibiting translation of HDA4 which is an inhibitor of myoblast proliferation, as well as nPTB and SRF which are both potent inhibitors of myoblast differentiation to myocytes. TNF-alpha signaling inhibits expression of these three microRNAs by activating NF-kappa-B which potently inhibits the three transcription factors (MYOD, MYOG, MEF2) that drive miR-133, -1, and-206 expression in muscle cells. The diagram illustrates how TNF-alpha produced by the autoimmune reaction seems to be responsible for the muscle wasting pathology observed in myositis.

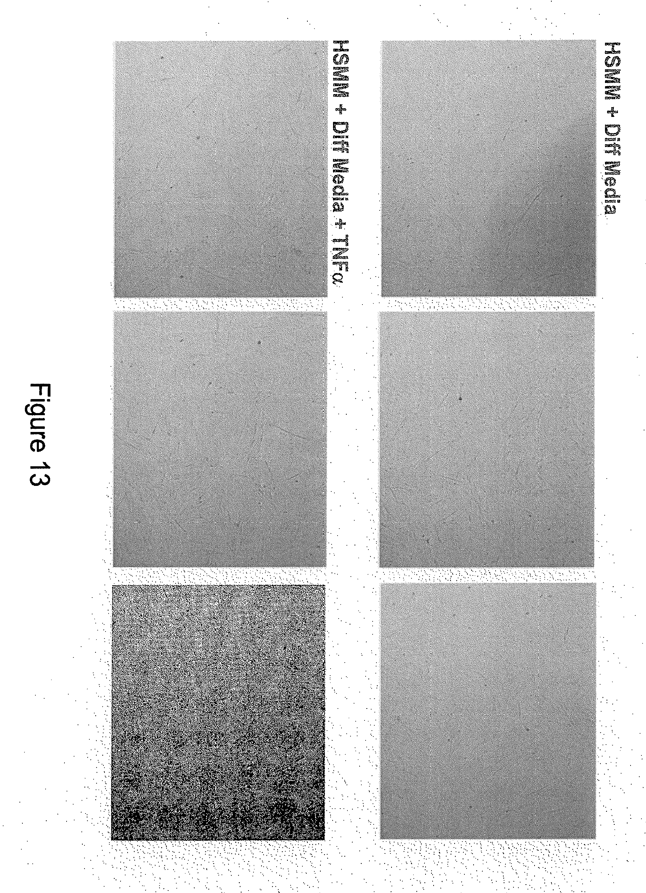

**Figure 13****. TNF-alpha blocked the differentiation of HSMM myoblasts to myocytes.** Shown are photos of HSMM at day 0, i.e., prior to exposure to differentiation media with or without inclusion of TNF-alpha (10 ng/ml). All of the cells display short uni-cell morphology indicative of myoblasts.

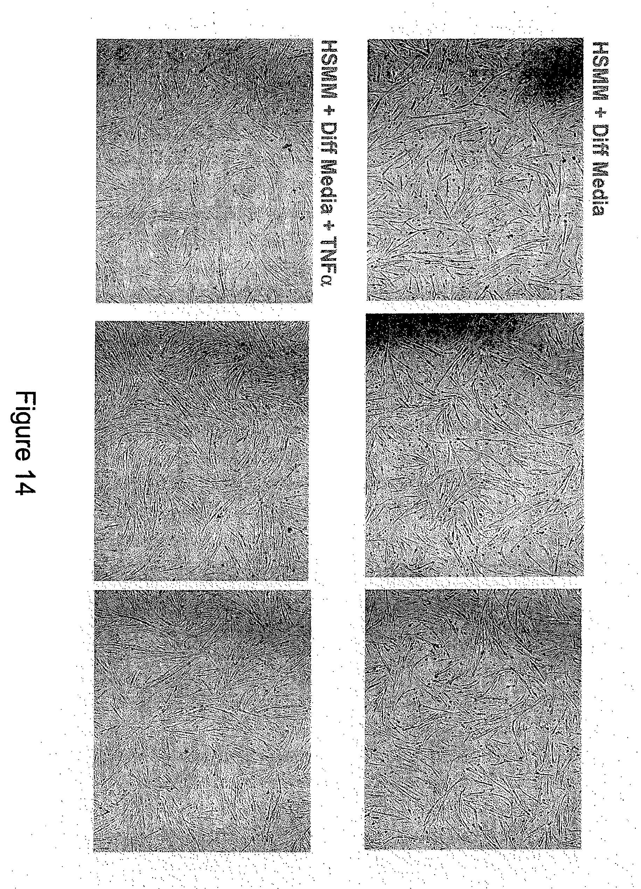

**Figure 14****. TNF-alpha blocked the differentiation of HSMM myoblasts to myocytes.** Shown are photos of HSMM at day 3 post exposure to differentiation media with or without inclusion of TNF-alpha (10 ng/ml). Cells treated with differentiation media alone show decreased cell number indicating reduced cell growth, and are forming in long thin multinucleated cells indicative of myocytes. Cells additionally treated with TNF-alpha remain short single-nucleated cells, and show a much higher cell density. These cells display the morphology and growth characteristics of HSMM myoblasts, indicating that TNF-alpha has blocked their differentiation to myocytes.

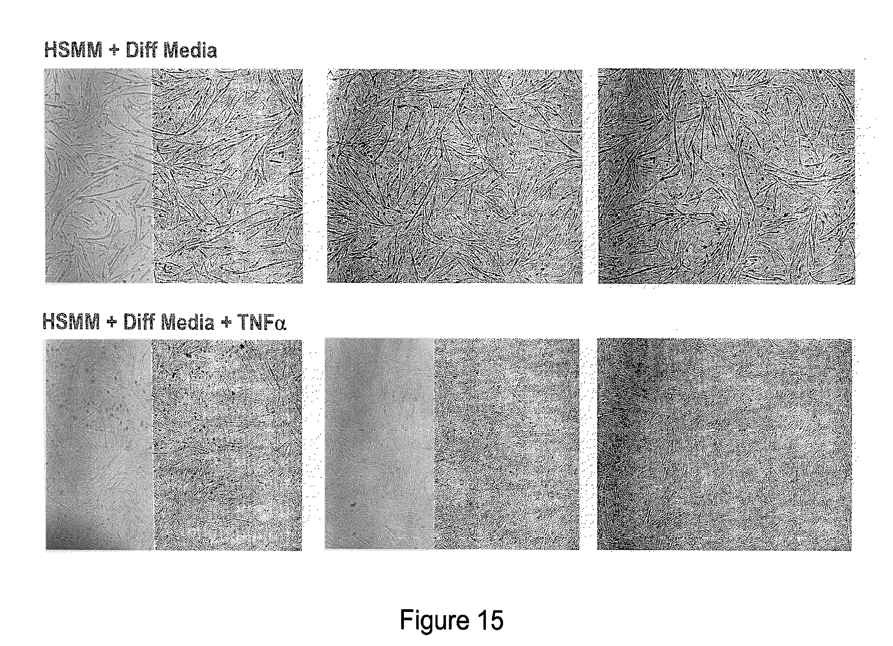

**Figure 15****. TNF-alpha blocked the differentiation of HSMM myoblasts to myocytes.** Shown are photos of HSMM at day 4 post exposure to differentiation media with or without inclusion of TNF-alpha (10 ng/ml). Cells treated with differentiation media alone continue to show decreased cell number indicating reduced cell growth, and have formed pronounced long thin multinucleated cells indicative of myocytes. Cells additionally treated with TNF-alpha remain short single-nucleated cells, and show a higher cell density than at day 3. These cells display the morphology and growth characteristics of HSMM myoblasts, indicating that TNF-alpha has blocked their differentiation to myocytes.

### Detailed Description

The technology described herein provides therapeutic treatments of muscle diseases. The disclosed technology also provides personalized medicine treatments for muscle degenerative diseases by calibrating timing and drugs for treatments using discrete microRNAs as biomarkers. Different subjects can metabolize a therapeutic drug at different rates and in different manners. This variability can result in varying effects of a drug in different subjects when treating a muscle degenerative disease. Technology described herein optimizes therapeutic methods for treating muscle degenerative diseases by allowing a clinician to track a biomarker linked to a muscle degenerative disease, and determine whether a subsequent dose of a drug for administration to a subject should be maintained, reduced or increased.

For example, it has been determined that increased levels of certain biomarkers, referred to herein as "over-represented biomarkers," are linked to muscle degenerative diseases (e.g., GM-CSF, TNF-alpha or other pro-inflammatory cytokine acting through NF kappa B). It also has been determined that decreased levels of certain biomarkers, referred to herein as "under-represented biomarkers," are linked to the muscle degenerative diseases (e.g., particular microRNA (e.g., hsa-miRNA-1, miRNA-133, miRNA-206)). Predetermined target levels of such biomarkers, or biomarker thresholds, are provided, and permits the determination of whether a subsequent dose of the drug should be increased, decreased or maintained or the dose scheduling should be altered or maintained. Typically, a clinician can make such a determination based on whether the presence, absence or amount of a biomarker is below, above or about the same as a biomarker threshold, respectively, in certain embodiments.

For example, determining that an over-represented biomarker level is significantly reduced and/or that an under-represented biomarker level is significantly increased after drug treatment provides an indication to a clinician that an administered drug is exerting a therapeutic effect. Based on such a biomarker determination, a clinician could make a decision to maintain a subsequent dose of the drug or lower the subsequent dose. In another example, determining that an over-represented biomarker level is not significantly reduced and/or that an under-represented biomarker level is not significantly increased provides an indication to a clinician that an administered drug is not significantly exerting a therapeutic effect. Based on such a biomarker determination, a clinician could make a decision to increase a subsequent dose of the drug. Given that drugs can be toxic to a subject and exert side effects, methods provided herein optimize therapeutic approaches as they provide the clinician with the ability to "dial in" an efficacious dosage of a drug and minimize side effects. In specific examples, methods provided herein allow a clinician to "dial up" the dose of a drug to a therapeutically efficacious level, where the dialed up dosage is below a toxic threshold level. Accordingly, treatment methods described herein enhance efficacy and reduce the likelihood of toxic side effects.

### Degenerative Muscle Disorders

Methods described herein can be used to treat a degenerative muscle disorder. Muscle disorders are a diverse group of diseases caused by various defective structural proteins, abnormal signaling molecules, enzymes and proteins involved in posttranslational modifications, and other mechanisms. Primary muscle disorders involve different groups of diseases, including muscular dystrophies, inflammatory myopathies, and congenital myopathies. These diseases are defined and classified in accordance with their clinical and pathological manifestations and the distribution of predominant muscle weakness.

Muscular dystrophies are a large heterogeneous group of more than thirty different inherited disorders characterized by muscle wasting and weakness of variable distribution and severity, manifesting at any age from birth to middle years, and resulting in significant morbidity and disability. Whereas some forms involve mutations within genes encoding structural members of the dystrophin-associated glycoprotein complex of the muscle membrane cytoskeleton, other mutations interfere with mRNA processing, alter protein posttranslational modifications, or modify enzymatic activities.

Abnormalities of dystrophin are known as a common cause of muscular dystrophy accounting for both Duchenne muscular dystrophy (DMD), one of the most severe types with rapidly progressive skeletal muscle weakness, and the milder Becker muscular dystrophy (BMD) phenotype. The highly heterogeneous limb girdle muscular dystrophies (LGMDs) are another major group of muscular dystrophies. Notably, mutated calpain-3 in patients with LGMD type 2A (LGMD2A) was the first enzyme, rather than structural protein, to be associated with muscular dystrophy. Mutations in dysferlin, a muscle membrane protein that plays a role in membrane repair, cause the LGMD type 2B (LGMD2B) and Miyoshi myopathy (MM). Facioscapulo-humeral muscular dystrophy (FSHD), a progressive muscle disease affecting mainly the muscles of the face and upper arms caused by deletions of a 3.3-kb repeat region located on 4q35.2 (6), is an additional common type of muscular dystrophy.

Other types of muscular dystrophy include: distal with an onset age of 40 to 60 years and characterized by weakness and wasting of muscles of the hands, forearms, and lower legs; Emery-Dreifuss, with on onset age of childhood to early teens, causing weakness and wasting of shoulder, upper arm, and shin muscles and often joint deformities; myotonic, with an onset age of 20 to 40 years and symptoms that include weakness of all muscle groups accompanied by delayed relaxation of muscles after contraction, first affecting the face, feet, hands, and neck; and oculopharyngeal, with an onset age of 40 to 70 years and affect muscles of the eyelids and throat causing weakening of throat muscles, ultimately causing inability to swallow.

Congenital myopathies are distinguished primarily by their histologic features, symptoms, and prognosis. Diagnosis is indicated by characteristic clinical findings and confirmed by muscle biopsy. Treatment consists of physical therapy, which may help preserve function. Among the group of congenital myopathies, nemaline myopathy (NM) is the most common nondystrophic congenital myopathy and is characterized by relatively nonprogressive proximal weakness of often, but not always, congenital onset and the presence of nema-line rod structures in the affected myofibers. Mutations in six different genes encoding the thin filament proteins and other skeletal muscle proteins account for the majority of disease cases.

Other types of congenital myopathy include: myotubular myopathy, which can be either autosomal or X-linked and produces mild weakness and hypotonia in both sexes or, in the X-linked variation, severe skeletal muscle weakness and hypotonia, facial weakness, impaired swallowing, and respiratory muscle weakness and respiratory failure; central core myopathy, which is autosomal dominant and causes hypotonia and mild, non-progressive, proximal muscle weakness to develop in neonates, often accompanied by facial weakness; congenital fiber type disproportion, which is inherited by a poorly understood pattern and causes hypotonia and weakness of the face, neck, trunk, and limbs, often accompanied by skeletal abnormalities and dysmorphic features and, rarely, respiratory failure; and multicore myopathy, which is usually autosomal recessive but may be autosomal dominant and typically presents as proximal weakness in infants, but in some children presents later with generalized weakness.

Methods described herein can be used to treat an inflammatory myopathy. Distinct idiopathic inflammatory myopathies exhibit notable clinical and histopathologic overlap with the inherited muscular disorders. General symptoms of chronic inflammatory myopathy include slow but progressive muscle weakness that starts in the proximal muscles--those muscles closest to the trunk of the body. Inflammation damages the muscle fibers, causing weakness, and may affect the arteries and blood vessels that run through the muscle. Other symptoms include fatigue after walking or standing, tripping or falling, and difficulty swallowing or breathing. Some patients may have slight muscle pain or muscles that are tender to touch.

Polymyositis (PM), the most common of the inflammatory myopathies, is a T cell- mediated pathology in which a cellular immune response is a key feature in promoting muscle damage. PM affects skeletal muscles (involved with making movement) on both sides of the body. It is rarely seen in persons under age 18; most cases are in patients between the ages of 31 and 60. Progressive muscle weakness leads to difficulty swallowing, speaking, rising from a sitting position, climbing stairs, lifting objects, or reaching overhead. Patients with PM may also experience arthritis, shortness of breath, and heart arrhythmias.

Dermatomyositis (DM) is characterized by a skin rash that precedes or accompanies progressive muscle weakness. The rash looks patchy, with bluish-purple or red discolorations, and characteristically develops on the eyelids and on muscles used to extend or straighten joints, including knuckles, elbows, heels, and toes. Red rashes may also occur on the face, neck, shoulders, upper chest, back, and other locations, and there may be swelling in the affected areas. The rash sometimes occurs without obvious muscle involvement. Adults with DM may experience weight loss or a low-grade fever, have inflamed lungs, and be sensitive to light. Adult DM, unlike PM, may accompany tumors of the breast, lung, female genitalia, or bowel. Children and adults with DM may develop calcium deposits, which appear as hard bumps under the skin or in the muscle (called calcinosis). Calcinosis most often occurs 1-3 years after disease onset but may occur many years later. These deposits are seen more often in childhood DM than in DM that begins in adults. DM may be associated with collagen-vascular or autoimmune diseases.

In some cases of PM and DM, distal muscles (away from the trunk of the body, such as those in the forearms and around the ankles and wrists) may be affected as the disease progresses. PM and DM may be associated with collagen-vascular or autoimmune diseases. PM may also be associated with infectious disorders.

Juvenile myositis (JM) has some similarities to adult DM and PM. It typically affects children ages 2 to 15 years, with symptoms that include proximal muscle weakness and inflammation, edema (an abnormal collection of fluids within body tissues that causes swelling), muscle pain, fatigue, skin rashes, abdominal pain, fever, and contractures (chronic shortening of muscles or tendons around joints, caused by inflammation in the muscle tendons, which prevents the joints from moving freely). Children with JM may also have difficulty swallowing and breathing, and the heart may be affected. Approximately 20 to 30 percent of children with JM develop calcinosis. Juvenile patients may not show higher than normal levels of the muscle enzyme creatine kinase in their blood but have higher than normal levels of other muscle enzymes.

Inclusion Body Myositis (IBM) is a common myopathology acquired by patients over 50 years of age, with prevalence in the USA of 7.06/100,000. IBM is characterized by progressive muscle weakness and wasting. The onset of muscle weakness is generally gradual (over months or years) and affects both proximal and distal muscles. Muscle weakness may affect only one side of the body. Falling and tripping are usually the first noticeable symptoms of IBM. For some patients the disorder begins with weakness in the wrists and fingers that causes difficulty with pinching, buttoning, and gripping objects. There may be weakness of the wrist and finger muscles and atrophy (thinning or loss of muscle bulk) of the forearm muscles and quadricep muscles in the legs. Difficulty swallowing occurs in approximately half of IBM cases. Symptoms of the disease usually begin after the age of 50, although the disease can occur earlier. Unlike PM and DM, IBM occurs more frequently in men than in women.

Inclusion body myositis patients present with two distinct pathologies. The first is chronic muscle inflammation. CD8+ T lymphocytes, macrophages, and dendritic cells invade the muscle and secrete cytokines causing localized low level chronic inflammation. B cells and plasma cells are also resident in muscle tissue and may contribute to pathology. However, these immunocytes do not appear to induce the cellular cytotoxicity or necrosis normally mediated by CD8+ cytotoxic T cells or macrophages. They instead seem to invade the interface of the myofibers and basil lamina. The myofibers associated with immune cells appear abnormal, but are not necrotic.

The invading immunocytes express a number of pro-inflammatory cytokines, including TNF-alpha and IL-6, which have been suggested to play a role in myositis inflammation. Current hypotheses propose that these cytokines both engender direct autoimmune effects on target muscle cells, and act to recruit autoimmune lymphocytes to the diseased muscle. These findings have led to the speculation that IBM is primarily an autoimmune disease.

The second pathology observed in IBM is systemic chronic muscle wasting, leading to the hypothesis that IBM is primarily a degenerative disease with inflammation as a secondary affect. Diseased muscle displays cells with myonuclear abnormalities, rimmed vacuoles, marked reduction of the caliber of a variable number of muscle fibers, focal increase of endomysial connective tissue, hypertrophied fibers, and abnormalities of mitochondria in muscle fibers with multiple mitochondrial DNA deletions. A number of studies have suggested that aberrantly expressed proteins, such as beta-amyloid, not normally found in muscle fibers lends to muscle cell wasting. Important degenerative observations include the lack of necrotic cells, a lack of regenerating muscle fibers, and loss of muscle mass (i.e. wasting). Thus, IBM has been suspected to be a primary degenerative myopathy, like DM and PM, or a primary degenerative myopathic disorder, such as a dystrophy with secondary inflammation.

### Cytokine Biomarkers

Provided herein are cytokine biomarkers associated with a degenerative muscle disease (e.g., inflammatory myopathy, myositis, inclusion body myositis). Such cytokine biomarkers generally are over-represented in a degenerative muscle disease.

Cytokines are a large and diverse family of polypeptide regulators produced widely throughout the body by cells of diverse origin. Cytokines are small secreted proteins, including peptides and glycoproteins, which mediate and regulate immunity, inflammation, and hematopoiesis. They are produced de novo in response to an immune stimulus. Cytokines generally (although not always) act over short distances and short time spans and at low concentration. They generally act by binding to specific membrane receptors, which then signal the cell via second messengers, often tyrosine kinases, to alter cell behavior (e.g., gene expression). Responses to cytokines include, for example, increasing or decreasing expression of membrane proteins (including cytokine receptors), proliferation, and secretion of effector molecules.

The term "cytokine" is a general description of a large family of proteins and glycoproteins. Other names include lymphokine (cytokines made by lymphocytes), monokine (cytokines made by monocytes), chemokine (cytokines with chemotactic activities), and interleukin (cytokines made by one leukocyte and acting on other leukocytes). Cytokines may act on cells that secrete them (autocrine action), on nearby cells (paracrine action), or in some instances on distant cells (endocrine action).

Examples of cytokines include, without limitation, interleukins (e.g., IL-1, IL-2, IL-3, IL-4, IL-5, IL-6, IL-7, IL-8, IL-9, IL-10, IL-11, IL-12, IL-13, IL-14, IL-15, IL-16, IL-17, IL-18 and the like), interferons (e.g., IFN-alpha, IFN-beta, IFN-gamma and the like), tumor necrosis factors (e.g., TNF-alpha, TNF-beta and the like), lymphokines, monokines and chemokines; growth factors (e.g., transforming growth factors (e.g., TGF-alpha, TGF-beta and the like)); colony-stimulating factors (e.g. GM-CSF, granulocyte colony-simulating factor (G-CSF) etc.); and the like.

A cytokine often acts via a cell-surface receptor counterpart. Subsequent cascades of intracellular signaling then alter cell functions. This signaling may include upregulation and/or downregulation of several genes and their transcription factors, resulting in the production of other cytokines, an increase in the number of surface receptors for other molecules, or the suppression of their own effect by feedback inhibition.

Nuclear factor kappa-B (NF-kappa-B) is a transcription factor involved in signaling of certain pro-inflammatory cytokines. NF-kappa-B is a pleiotropic transcription factor influencing multiple protein end products and expressed in numerous different cell types after stimulation and/or cell activation by a wide variety of stimuli (cytokines, growth factors and mitogens, hormones, receptor ligation, crosslinking of surface molecules, viruses and viral proteins, oxidative stress, and chemical agents such as phorbol esters). Many different genes contain functional NF-kappa-B binding sites (GGGACTTTCC) in associated promoters. NF-kappa-B-responsive genes include those encoding a number of cytokines and growth factors, cytokine receptors, receptor signaling proteins, cell adhesion molecules, and many other proteins involved in various processes, including immune responses, acute phase reaction and inflammation, cell growth and differentiation, growth control of certain tumors, and cell death by apoptosis.

Non-limiting examples of pro-inflammatory cytokines that may act through NF-kappa-B include IL-1beta, IL-6, and TNF-alpha, INF-alpha and chemokines monocyte chemoattractant protein-1 and IL-8, as well as the AP-1-dependent basic fibroblast growth factor. It has been determined that in muscle cells, TNF-alpha induces NF-kappa-B expression and activation.

It has been determined that tumor necrosis factor alpha (TNF-alpha) is an over-represented biomarker associated with a degenerative muscle disease. TNF-alpha is a cytokine involved in systemic inflammation and is a member of a group of cytokines that stimulate the acute phase reaction. A role of TNF-alpha is in regulation of immune cells. TNF-alpha is also able to induce apoptotic cell death, induce inflammation, and to inhibit tumorigenesis and viral replication. TNF-alpha is primarily produced as a 212-amino acid-long type II transmembrane protein arranged in stable homotrimers. From this membrane-integrated form the soluble homotrimeric cytokine (sTNF) is released via proteolytic cleavage by the metalloprotease TNF alpha converting enzyme. The soluble 51 kDa trimeric sTNF tends to dissociate at concentrations below the nanomolar range, thereby losing its bioactivity.

Non-limiting examples of DNA, mRNA, and polypeptide sequences for human TNF-alpha (UniGene Hs.241570 and Pubmed Gene 7124) are provided hereafter.

### TNF-alpha DNA sequence

Accession NG_007462
Version NG_007462.1

1 gctccctcag caaggacagc agaggaccag ctaagaggga gagaagcaac tacagacccc
61 ccctgaaaac aaccctcaga cgccacatcc cctgacaagc tgccaggcag gttctcttcc
121 tctcacatac tgacccacgg ctccaccctc tctcccctgg aaaggacacc atgagcactg
181 aaagcatgat ccgggacgtg gagctggccg aggaggcgct ccccaagaag acaggggggc
241 cccagggctc caggcggtgc ttgttcctca gcctcttctc cttcctgatc gtggcaggcg
301 ccaccacgct cttctgcctg ctgcactttg gagtgatcgg cccccagagg gaagaggtga
361 gtgcctggcc agccttcatc cactctccca cccaagggga aatggagacg caagagaggg
421 agagagatgg gatgggtgaa agatgtgcgc tgatagggag ggatggagag aaaaaaacgt
481 ggagaaagac ggggatgcag aaagagatgt ggcaagagat ggggaagaga gagagagaaa
541 gatggagaga caggatgtct ggcacatgga aggtgctcac taagtgtgta tggagtgaat
601 gaatgaatga atgaatgaac aagcagatat ataaataaga tatggagaca gatgtggggt
661 gtgagaagag agatggggga agaaacaagt gatatgaata aagatggtga gacagaaaga
721 gcgggaaata tgacagctaa ggagagagat gggggagata aggagagaag aagatagggt
781 gtctggcaca cagaagacac tcagggaaag agctgttgaa tgcctggaag gtgaatacac
841 agatgaatgg agagagaaaa ccagacacct cagggctaag agcgcaggcc agacaggcag
901 ccagctgttc ctcctttaag ggtgactccc tcgatgttaa ccattctcct tctccccaac
961 agttccccag ggacctctct ctaatcagcc ctctggccca ggcagtcagt aagtgtctcc
1021 aaacctcttt cctaattctg ggtttgggtt tgggggtagg gttagtaccg gtatggaagc
1081 agtgggggaa atttaaagtt ttggtcttgg gggaggatgg atggaggtga aagtaggggg
1141 gtattttcta ggaagtttaa gggtctcagc tttttctttt ctctctcctc ttcaggatca
1201 tcttctcgaa ccccgagtga caagcctgta gcccatgttg taggtaagag ctctgaggat
1261 gtgtcttgga acttggaggg ctaggatttg gggattgaag cccggctgat ggtaggcaga
1321 acttggagac aatgtgagaa ggactcgctg agctcaaggg aagggtggag gaacagcaca
1381 ggccttagtg ggatactcag aacgtcatgg ccaggtggga tgtgggatga cagacagaga
1441 ggacaggaac cggatgtggg gtgggcagag ctcgagggcc aggatgtgga gagtgaaccg
1501 acatggccac actgactctc ctctccctct ctccctccct ccagcaaacc ctcaagctga
1561 ggggcagctc cagtggctga accgccgggc caatgccctc ctggccaatg gcgtggagct
1621 gagagataac cagctggtgg tgccatcaga gggcctgtac ctcatctact cccaggtcct
1681 cttcaagggc caaggctgcc cctccaccca tgtgctcctc acccacacca tcagccgcat
1741 cgccgtctcc taccagacca aggtcaacct cctctctgcc atcaagagcc cctgccagag
1801 ggagacccca gagggggctg aggccaagcc ctggtatgag cccatctatc tgggaggggt
1861 cttccagctg gagaagggtg accgactcag cgctgagatc aatcggcccg actatctcga
1921 ctttgccgag tctgggcagg tctactttgg gatcattgcc ctgtgaggag gacgaacatc
1981 caaccttccc aaacgcctcc cctgccccaa tccctttatt accccctcct tcagacaccc
2041 tcaacctctt ctggctcaaa aagagaattg ggggcttagg gtcggaaccc aagcttagaa
2101 ctttaagcaa caagaccacc acttcgaaac ctgggattca ggaatgtgtg gcctgcacag
2161 tgaagtgctg gcaaccacta agaattcaaa ctggggcctc cagaactcac tggggcctac
2221 agctttgatc cctgacatct ggaatctgga gaccagggag cctttggttc tggccagaat
2281 gctgcaggac ttgagaagac ctcacctaga aattgacaca agtggacctt aggccttcct
2341 ctctccagat gtttccagac ttccttgaga cacggagccc agccctcccc atggagccag
2401 ctccctctat ttatgtttgc acttgtgatt atttattatt tatttattat ttatttattt

2461 acagatgaat gtatttattt gggagaccgg ggtatcctgg gggacccaat gtaggagctg
2521 ccttggctca gacatgtttt ccgtgaaaac ggagctgaac aataggctgt tcccatgtag
2581 ccccctggcc tctgtgcctt cttttgatta tgttttttaa aatatttatc tgattaagtt
2641 gtctaaacaa tgctgatttg gtgaccaact gtcactcatt gctgagcctc tgctccccag
2701 gggagttgtg tctgtaatcg ccctactatt cagtggcgag aaataaagtt tgcttagaaa
2761 aga

### TNF-alpha cDNA sequence

Accession NM_000594
Version NM_000594.2

1 ctccctcagc aaggacagca gaggaccagc taagagggag agaagcaact acagaccccc
61 cctgaaaaca accctcagac gccacatccc ctgacaagct gccaggcagg ttctcttcct
121 ctcacatact gacccacggc tccaccctct ctcccctgga aaggacacca tgagcactga
181 aagcatgatc cgggacgtgg agctggccga ggaggcgctc cccaagaaga caggggggcc
241 ccagggctcc aggcggtgct tgttcctcag cctcttctcc ttcctgatcg tggcaggcgc
301 caccacgctc ttctgcctgc tgcactttgg agtgatcggc ccccagaggg aagagttccc
361 cagggacctc tctctaatca gccctctggc ccaggcagtc agatcatctt ctcgaacccc
421 gagtgacaag cctgtagccc atgttgtagc aaaccctcaa gctgaggggc agctccagtg
481 gctgaaccgc cgggccaatg ccctcctggc caatggcgtg gagctgagag ataaccagct
541 ggtggtgcca tcagagggcc tgtacctcat ctactcccag gtcctcttca agggccaagg
601 ctgcccctcc acccatgtgc tcctcaccca caccatcagc cgcatcgccg tctcctacca
661 gaccaaggtc aacctcctct ctgccatcaa gagcccctgc cagagggaga ccccagaggg
721 ggctgaggcc aagccctggt atgagcccat ctatctggga ggggtcttcc agctggagaa
781 gggtgaccga ctcagcgctg agatcaatcg gcccgactat ctcgactttg ccgagtctgg
841 gcaggtctac tttgggatca ttgccctgtg aggaggacga acatccaacc ttcccaaacg
901 cctcccctgc cccaatccct ttattacccc ctccttcaga caccctcaac ctcttctggc
961 tcaaaaagag aattgggggc ttagggtcgg aacccaagct tagaacttta agcaacaaga
1021 ccaccacttc gaaacctggg attcaggaat gtgtggcctg cacagtgaag tgctggcaac
1081 cactaagaat tcaaactggg gcctccagaa ctcactgggg cctacagctt tgatccctga
1141 catctggaat ctggagacca gggagccttt ggttctggcc agaatgctgc aggacttgag
1201 aagacctcac ctagaaattg acacaagtgg accttaggcc ttcctctctc cagatgtttc
1261 cagacttcct tgagacacgg agcccagccc tccccatgga gccagctccc tctatttatg
1321 tttgcacttg tgattattta ttatttattt attatttatt tatttacaga tgaatgtatt
1381 tatttgggag accggggtat cctgggggac ccaatgtagg agctgccttg gctcagacat
1441 gttttccgtg aaaacggagc tgaacaatag gctgttccca tgtagccccc tggcctctgt
1501 gccttctttt gattatgttt tttaaaatat ttatctgatt aagttgtcta aacaatgctg
1561 atttggtgac caactgtcac tcattgctga gcctctgctc cccaggggag ttgtgtctgt
1621 aatcgcccta ctattcagtg gcgagaaata aagtttgctt agaaaagaa

### TNF-alpha amino acid sequence

Accession AAA61200
Version AAA61200.1

1 MSTESMIRDV ELAEEALPKK TGGPQGSRRC LFLSLFSFLI VAGATTLFCL LHFGVIGPQR
61 EEFPRDLSLI SPLAQAVRSS SRTPSDKPVA HVVANPQAEG QLQWLNRRAN ALLANGVELR
121 DNQLVVPSEG LYLIYSQVLF KGQGCPSTHV LLTHTISRIA VSYQTKVNLL SAIKSPCQRE
181 TPEGAEAKPW YEPIYLGGVF QLEKGDRLSA EINRPDYLDF AESGQVYFGI IAL

It also has been determined that granulocyte macrophage colony-stimulating factor (GM-CSF) is an over-represented biomarker associated with a degenerative muscle disease. GM-CSF is a cytokine secreted by macrophages, T cells, mast cells, endothelial cells and fibroblasts. GM-CSF functions as a white blood cell growth factor and stimulates stem cells to produce granulocytes (neutrophils, eosinophils, and basophils) and monocytes. Monocytes exit the circulation and migrate into tissue, whereupon they mature into macrophages. GM-CSF is therefore part of the immune/inflammatory cascade, by which activation of a small number of macrophages can rapidly lead to an increase in their numbers.

Non-limiting examples of DNA, mRNA, and polypeptide sequences for human GM-CSF (UniGene Hs.1349 and Pubmed Gene 1437) are provided hereafter.

### GM-CSF DNA sequence

Accession NC_000005.9
NT_034772.6

1 tacacagaga gaaaggctaa agttctctgg aggatgtggc tgcagagcct gctgctcttg
61 ggcactgtgg cctgcagcat ctctgcaccc gcccgctcgc ccagccccag cacgcagccc
121 tgggagcatg tgaatgccat ccaggaggcc cggcgtctcc tgaacctgag tagagacact
181 gctgctgaga tggtaagtga gagaatgtgg gcctgtgcct aggccaccca gctggcccct
241 gactggccac gcctgtcagc ttgataacat gacattttcc ttttctacag aatgaaacag
301 tagaagtcat ctcagaaatg tttgacctcc aggtaagatg cttctctctg acatagcttt
361 ccagaagccc ctgccctggg gtggaggtgg ggactccatt ttagatggca ccacacaggg
421 ttgtccactt tctctccagt cagctggctg caggaggagg gggtagcaac tgggtgctca
481 agaggctgct ggccgtgccc ctatggcagt cacatgagct cctttatcag ctgagcggcc
541 atgggcagac ctagcattca atggccagga gtcaccaggg gacaggtggt aaagtggggg
601 tcacttcatg agacaggagc tgtgggtttg gggcgctcac tgtgccccga gaccaagtcc
661 tgttgagaca gtgctgacta cagagaggca cagaggggtt tcaggaacaa cccttgccca
721 cccagcaggt ccaggtgagg ccccaccccc ctctccctga atgatggggt gagagtcacc
781 tccttcccta aggctgggct cctctccagg tgccgctgag ggtggcctgg gcggggcagt
841 gagaagggca ggttcgtgcc tgccatggac agggcagggt ctatgactgg acccagcctg
901 tgcccctccc aagccctact cctgggggct gggggcagca gcaaaaagga gtggtggaga
961 gttcttgtac cactgtgggc acttggccac tgctcaccga cgaacgacat tttccacagg
1021 agccgacctg cctacagacc cgcctggagc tgtacaagca gggcctgcgg ggcagcctca
1081 ccaagctcaa gggccccttg accatgatgg ccagccacta caagcagcac tgccctccaa
1141 ccccggtgag tgcctacggc agggcctcca gcaggaatgt cttaatctag ggggtggggt
1201 cgacatgggg agagatctat ggctgtggct gttcaggacc ccagggggtt tctgtgccaa
1261 cagttatgta atgattagcc ctccagagag gaggcagaca gcccatttca tcccaaggag
1321 tcagagccac agagcgctga agcccacagt gctccccagc aggagctgct cctatcctgg
1381 tcattattgt cattatggtt aatgaggtca gaggtgaggg caaacccaag gaaacttggg
1441 gcctgcccaa ggcccagagg aagtgcccag gcccaagtgc caccttctgg caggactttc
1501 ctctggcccc acatggggtg cttgaattgc agaggatcaa ggaagggagg ctacttggaa
1561 tggacaagga cctcaggcac tccttcctgc gggaagggag caaagtttgt ggccttgact
1621 ccactccttc tgggtgccca gagacgacct cagcccagct gccctgctct gccctgggac
1681 caaaaaggca ggcgtttgac tgcccagaag gccaacctca ggctggcact taagtcaggc
1741 ccttgactct ggctgccact ggcagagcta tgcactcctt ggggaacacg tgggtggcag
1801 cagcgtcacc tgacccaggt cagtgggtgt gtcctggagt gggcctcctg gcctctgagt
1861 tctaagaggc agtagagaaa catgctggtg cttccttccc ccacgttacc cacttgcctg
1921 gactcaagtg ttttttattt ttcttttttt aaaggaaact tcctgtgcaa cccagattat
1981 cacctttgaa agtttcaaag agaacctgaa ggactttctg cttgtcatcc cctttgactg
2041 ctgggagcca gtccaggagt gagaccggcc agatgaggct ggccaagccg gggagctgct

2101 ctctcatgaa acaagagcta gaaactcagg atggtcatct tggagggacc aaggggtggg
2161 ccacagccat ggtgggagtg gcctggacct gccctgggcc acactgaccc tgatacaggc
2221 atggcagaag aatgggaata ttttatactg acagaaatca gtaatattta tatatttata
2281 tttttaaaat atttatttat ttatttattt aagttcatat tccatattta ttcaagatgt
2341 tttaccgtaa taattattat taaaaatatg cttct

### GM-CSF cDNA sequence

### Accession NM_0000758.2

1 acacagagag aaaggctaaa gttctctgga ggatgtggct gcagagcctg ctgctcttgg
61 gcactgtggc ctgcagcatc tctgcacccg cccgctcgcc cagccccagc acgcagccct
121 gggagcatgt gaatgccatc caggaggccc ggcgtctcct gaacctgagt agagacactg
181 ctgctgagat gaatgaaaca gtagaagtca tctcagaaat gtttgacctc caggagccga
241 cctgcctaca gacccgcctg gagctgtaca agcagggcct gcggggcagc ctcaccaagc
301 tcaagggccc cttgaccatg atggccagcc actacaagca gcactgccct ccaaccccgg
361 aaacttcctg tgcaacccag attatcacct ttgaaagttt caaagagaac ctgaaggact
421 ttctgcttgt catccccttt gactgctggg agccagtcca ggagtgagac cggccagatg
481 aggctggcca agccggggag ctgctctctc atgaaacaag agctagaaac tcaggatggt
541 catcttggag ggaccaaggg gtgggccaca gccatggtgg gagtggcctg gacctgccct
601 gggccacact gaccctgata caggcatggc agaagaatgg gaatatttta tactgacaga
661 aatcagtaat atttatatat ttatattttt aaaatattta tttatttatt tatttaagtt
721 catattccat atttattcaa gatgttttac cgtaataatt attattaaaa atatgcttct
781 a

### GM-CSF amino acid

### Accession NP_000749.2

1 mwlqsllllg tvacsisapa rspspstqpw ehvnaiqear rllnlsrdta aemnetvevi
61 semfdlqept clqtrlelyk qglrgsltkl kgpltmmash ykqhcpptpe tscatqiitf
121 esfkenlkdf llvipfdcwe pvqe

Cytokines may be detected as full-length (e.g., whole) proteins, polypeptides, metabolites, messenger RNA (mRNA), complementary DNA (cDNA), and various intermediate products and fragments of the foregoing (e.g., cleavage products (e.g., peptides, mRNA fragments)). For example, TNF-alpha protein may be detected as the complete, full-length molecule or as any fragment large enough to provide varying levels of positive identification. Such a fragment may comprise amino acids numbering less than 10, from 10 to 20, from 20 to 50, from 50 to 100, from 100 to 150, from 150 to 200 and above. Likewise, GM-CSF protein can be detected as the complete, full-length amino acid molecule or as any fragment large enough to provide varying levels of positive identification. Such a fragment may comprise amino acids numbering less than 10, from 10 to 20, from 20 to 50, from 50 to 100, from 100 to 150 and above.

In certain embodiments, cytokine mRNA may be detected by targeting a complete sequence or any sufficient fragment for specific detection. An mRNA fragment may include fewer than 10 nucleotides or any larger number. A fragment may comprise the 3' end of the mRNA strand with any portion of the strand, the 5' end with any portion of the strand, and any center portion of the strand.

Detection may be performed using any suitable method known in the art, including, without limitation, mass spectrometry (e.g., matrix-assisted laser desorption ionization mass spectrometry (MALDI-MS), electrospray mass spectrometry (ES-MS)), electrophoresis (e.g., capillary electrophoresis), high performance liquid chromatography (HPLC), nucleic acid affinity (e.g., hybridization), amplification and detection (e.g., real-time or reverse-transcriptase polymerase chain reaction (RT-PCR)), and antibody assays (e.g., antibody array, enzyme-linked immunosorbant assay (ELISA)). Non-limiting examples of commercially available products include ELISA (Invitrogen KHC2012) for GM-CSF protein determination and TaqMan Custom Assay, ABI for GM-CSF mRNA determination. A transignal mouse antibody array specific for GM-CSF and TNF-alpha and other cytokines is commercially available for detection of these cytokines (Panomics/Affymetrix). Prepared ELISA kits for human and mouse GM-CSF and TNF-alpha also are commercially available (eBioscience).

### Nucleic Acids

A "nucleic acid" as used herein generally refers to a molecule (one, two or more strands) of DNA, RNA or a derivative or analog thereof, comprising a nucleobase. A nucleobase includes, for example, a naturally occurring purine or pyrimidine base found in DNA (e.g., an adenine "A," a guanine "G," a thymine "T" or a cytosine "C") or RNA (e.g., an A, a G, an uracil "U" or a C). The term "nucleic acid" encompasses the terms "oligonucleotide" and "polynucleotide," each as a subgenus of the term "nucleic acid." Nucleic acids as provided herein include without limitation microRNA, siNA, and antisense RNA. Nucleic acids may be, be at least, be at most, or be about 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 441, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 nucleotides, or any range derivable therein, in length. Such lengths cover the lengths of processed miRNA or siNA, miRNA or siNAmolecules, precursor miRNA or siNA, miRNA or siNA containing vectors, control nucleic acids, and other molecules, probes and primers. In many embodiments, miRNA are 19-24 nucleotides in length, while miRNA precursors are generally between 62 and 110 nucleotides in humans.

Nucleic acids herein provided may have regions of identity or complementarity to another nucleic acid. It is contemplated that the region of complementarity or identity can be at least 5 contiguous residues, though it is specifically contemplated that the region is, is at least, is at most, or is about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 441, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 contiguous nucleotides. It is further understood that such lengths of complementarity are within a precursor miRNA or between a miRNA drug and that a target molecule or a miRNA gene are such lengths.

A nucleic acid may also comprise a vector, including without limitation a plasmid or virus. The vector may code for a pre-processed nucleic acid molecule, or for the mature post-processed molecule (e.g., pre-processed or post-processed miRNA or siRNA).

As provided herein a "synthetic nucleic acid" means that the nucleic acid does not have a chemical structure or sequence of a naturally occurring nucleic acid. Consequently, it is understood that the term "synthetic miRNA" refers to a "synthetic nucleic acid" that is not isolated from a cell and is artificially manufactured, but which may sometimes function in a cell or under physiological conditions.

While some embodiments may involve synthetic miRNAs or other synthetic nucleic acids, in certain embodiments, the nucleic acid molecule(s) need not be "synthetic." In such embodiments, a non-synthetic miRNA employed in methods and compositions may have the entire sequence and structure of a naturally occurring miRNA precursor or the mature miRNA. For example, non-synthetic miRNAs used in methods and compositions as herein provided may not have one or more modified nucleotides or nucleotide analogs. In these embodiments, the non-synthetic miRNA may or may not be recombinantly produced. In particular embodiments, the nucleic acid in methods and/or compositions provided herein is specifically a synthetic miRNA and not a non-synthetic miRNA (that is, not a miRNA that qualifies as "synthetic"). In some embodiments a non-synthetic miRNA and not a synthetic miRNA may be utilized. Any embodiments discussed with respect to the use of synthetic miRNAs can be applied with respect to non-synthetic miRNAs, and vice versa.

As used herein the term "naturally occurring" refers to something found in an organism without any intervention by a person; it could refer to a naturally-occurring wild-type or mutant molecule. In some embodiments a synthetic miRNA molecule does not have the sequence of a naturally occurring miRNA molecule. In other embodiments, a synthetic miRNA molecule may have the sequence of a naturally occurring miRNA molecule, but the chemical structure of the molecule, particularly in the part unrelated specifically to the precise sequence (non-sequence chemical structure) differs from chemical structure of the naturally occurring miRNA molecule with that sequence. In some cases, the synthetic miRNA has a sequence and non-sequence chemical structure that are not found in a naturally-occurring miRNA. Moreover, the sequence of the synthetic molecules can identify which miRNA is effectively being provided or inhibited. The endogenous miRNA is referred to herein as the "corresponding miRNA." Corresponding miRNA sequences that can be used in as herein provided include, but are not limited to, all or a portion of those sequences previously listed herein, as well as any other miRNA sequence, miRNA precursor sequence, or any sequence complementary thereof.

As used herein, "hybridization", "hybridizes" or "capable of hybridizing" is understood to mean forming a double or triple stranded molecule or a molecule with partial double or triple stranded nature. The term "anneal" as used herein is synonymous with "hybridize." The term "hybridization", "hybridize(s)" or "capable of hybridizing" encompasses the terms "stringent condition(s)" or "high stringency" and the terms "low stringency" or "low stringency condition(s)."

As used herein "stringent condition(s)" or "high stringency" are those conditions that allow hybridization between or within one or more nucleic acid strand(s) containing complementary sequence(s), but preclude hybridization of random sequences. Stringent conditions tolerate little, if any, mismatch between a nucleic acid and a target strand. Such conditions are known, and are preferred for applications requiring high selectivity. Non-limiting applications include isolating a nucleic acid, such as a gene or a nucleic acid segment thereof, or detecting at least one specific mRNA transcript or a nucleic acid segment thereof, and the like.

Stringent conditions may comprise low salt and/or high temperature conditions, such as provided by about 0.02 M to about 0.5 M NaCl at temperatures of about 42 degrees C to about 70 degrees C. It is understood that the temperature and ionic strength of a desired stringency are determined in part by the length of the particular nucleic acid(s), the length and nucleobase content of the target sequence(s), the charge composition of the nucleic acid(s), and the presence or concentration of formamide, tetramethylammonium chloride or other solvent(s) in a hybridization mixture.

It is understood that these ranges, compositions and conditions for hybridization are mentioned by way of non-limiting examples only, and that the desired stringency for a particular hybridization reaction is often determined empirically by comparison to one or more positive or negative controls. Depending on the application envisioned varying conditions of hybridization may be employed to achieve varying degrees of selectivity of a nucleic acid towards a target sequence. In a non-limiting example, identification or isolation of a related target nucleic acid that does not hybridize to a nucleic acid under stringent conditions may be achieved by hybridization at low temperature and/or high ionic strength. Such conditions are termed "low stringency" or "low stringency conditions," and non-limiting examples of low stringency include hybridization performed at about 0.15 M to about 0.9 M NaCl at a temperature range of about 20°C to about 50°C. The low or high stringency conditions may be further modified to suit a particular application.

### siNA

siNA refers to a class of nucleic acid molecules capable of mediating sequence specific RNAi, for example short interfering RNA (siRNA), double-stranded RNA (dsRNA), micro-RNA (miRNA), short hairpin RNA (shRNA), short interfering oligonucleotide, short interfering nucleic acid, short interfering modified oligonucleotide, chemically-modified siRNA, post-transcriptional gene silencing RNA (ptgsRNA), and others. In addition, as used herein, the term RNAi is meant to be equivalent to other terms used to describe sequence specific RNA interference, such as post transcriptional gene silencing, or epigenetics. For example, siNA molecules can be used to epigenetically silence genes at either or both of the post-transcriptional level and the pre-transcriptional level. In a non-limiting example, epigenetic regulation of gene expression by siNA molecules of the technology can result from siNA mediated modification of chromatin structure to alter gene expression. Thus, a siNA may be used therapeutically to mediate the level of a polypeptide or protein, for example GM-CSF, TNF-alpha or an NF-kappa-B modulating agent.

A siNA may be a double-stranded polynucleotide molecule comprising self-complementary sense and antisense regions, where the antisense region comprises nucleotide sequence that is complementary to nucleotide sequence in a target nucleic acid molecule or a portion thereof and the sense region having nucleotide sequence corresponding to the target nucleic acid sequence or a portion thereof. A siNA can be assembled from two separate oligonucleotides, where one strand is the sense strand and the other is the antisense strand, where the antisense and sense strands are self-complementary. In some embodiments, each strand comprises nucleotide sequence that is complementary to nucleotide sequence in the other strand; such as where the antisense strand and sense strand form a duplex or double stranded structure, for example where the double stranded region is about 1, 2, 3, 4, 5, 6, 7, 8, 9, 10, 11, 12, 13, 14, 15, 16, 17, 18, 19, 20, 21, 22, 23, 24, or 25 or more base pairs.

The antisense strand can comprise a nucleotide sequence that is complementary to nucleotide sequence in a target nucleic acid molecule or a portion thereof and the sense strand comprises nucleotide sequence corresponding to the target nucleic acid sequence or a portion thereof. In some embodiments, a siNA can be assembled from a single oligonucleotide, where the self-complementary sense and antisense regions of the siNA are linked by means of a nucleic acid based or non-nucleic acid-based linker(s). A siNA can be a polynucleotide with a hairpin secondary structure, having self-complementary sense and antisense regions, where the antisense region comprises nucleotide sequence that is complementary to nucleotide sequence in a separate target nucleic acid molecule or a portion thereof and the sense region having nucleotide sequence corresponding to the target nucleic acid sequence or a portion thereof. A siNA can be a circular single-stranded polynucleotide having two or more loop structures and a stem comprising self-complementary sense and antisense regions, where the antisense region comprises nucleotide sequence that is complementary to nucleotide sequence in a target nucleic acid molecule or a portion thereof and the sense region having nucleotide sequence corresponding to the target nucleic acid sequence or a portion thereof, and where the circular polynucleotide can be processed either in vivo or in vitro to generate an active siNA molecule capable of mediating RNAi.

In some embodiments a siNA comprises two strands of RNA. In certain embodiments a siNA comprises two strands of DNA. A siNA may sometimes be a hybrid, comprising one strand of RNA and one strand of DNA. One or both strands may also comprise mixed RNA and DNA. In some embodiments a strand of a siNA (e.g., a strand of a siRNA) may be about 5 to about 60 nucleotides in length (e.g., about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 41, 42, 43, 44 45 46 47 48, 49, 50, 51, 52, 53, 54, 55, 56, 57, 58 or 59 nucleotides). A siNA strand sometimes may exceed 60 nucleotides.

A siNA may also comprise a single-stranded polynucleotide having a nucleotide sequence complementary to nucleotide sequence in a target nucleic acid molecule or a portion thereof (for example, where such siNA molecule does not require the presence within the siNA molecule of nucleotide sequence corresponding to the target nucleic acid sequence or a portion thereof), where the single stranded polynucleotide can further comprise a terminal phosphate group, such as a 5'-phosphate or 5', 3'-diphosphate.

In certain embodiments, a siNA molecule may comprise separate sense and antisense sequences or regions, where the sense and antisense regions are covalently linked by nucleotide or non-nucleotide linkers molecules as is known in the art, or are alternately non-covalently linked by ionic interactions, hydrogen bonding, van der waals interactions, hydrophobic interactions, and/or stacking interactions. In certain embodiments, a siNA molecule comprises a nucleotide sequence that is complementary to nucleotide sequence of a target gene. In some embodiments, the siNA molecule interacts with nucleotide sequence of a target gene in a manner that causes inhibition of expression of the target gene.

In some embodiments, one or more nucleotides in an siRNA are substituted with another nucleotide, are a modified base, are deleted and/or are inserted (e.g., 1, 2, 3, 4, 5, 6, 7, 8, 9, 10, 11, 12, 13, 14, 15, 16, 17, 18, 19, 20 nucleotides in the siRNA are substituted, are a modified base, are deleted and/or are inserted) relative to a unmodified reference siRNA (e.g., a native siRNA). The function of such a modified siRNA in vivo or in vitro sometimes is the same as for a reference siRNA, and sometimes is modified (e.g., greater or reduced). A modified function typically is detectable and sometimes is within 100-fold greater (e.g., 2, 4, 6, 8, 10, 15, 20, 25, 30, 35, 40, 45, 50, 55, 60, 65, 70, 75, 80, 85, 90, 95-fold greater) or 100-fold reduced (e.g., 2, 4, 6, 8, 10, 15, 20, 25, 30, 35, 40, 45, 50, 55, 60, 65, 70, 75, 80, 85, 90, 95-fold reduced) of the function elicited by a reference siRNA.

### MicroRNA

MicroRNAs (also referred to herein as "miRNAs") are a class of non-coding regulatory RNAs of approximately from 15 to 30 nucleotides up to 80-120 nucleotides in length as precursor microRNA. The term "miRNA" generally refers to a single-stranded molecule, but in specific embodiments, may also encompass a region or an additional strand that is partially (between 10 and 50% complementary across length of strand), substantially (greater than 50% but less than 100% complementary across length of strand) or fully complementary to another region of the same single-stranded molecule or to another nucleic acid. Thus, miRNA may encompass a single-stranded, double-stranded or partially single-stranded molecule. For example, precursor miRNA may have a self-complementary region, which is up to 100% complementary.

Many microRNAs are highly conserved across a number of species while some are species specific. They regulate gene expression post-transcriptionally, primarily by associating with the 3'untranslated region (UTR) of their regulatory target mRNAs. MicroRNAs are implicated in cell proliferation, differentiation, and apoptosis, as well as other cellular, molecular, and developmental pathways. It is understood that some miRNA is derived from genomic sequences or a gene. In this respect, the term "gene" is used for simplicity to refer to the genomic sequence encoding the precursor miRNA for a given miRNA. However, some embodiments may involve genomic sequences of a miRNA that are involved in its expression, such as a promoter or other regulatory sequences. The term "recombinant" may be used and this generally refers to a molecule that has been manipulated in vitro or that is a replicated or expressed product of such a molecule.

Native miRNAs are regulatory RNAs that act as the recognition component of the complex RNA - induced Silencing Complex (RISC) riboprotein complex. The genes encoding miRNAs are longer than the processed mature miRNA molecule. Genomic microRNAs exist in many different forms, including individual genes, genetic clusters of multiple microRNAs, or encoded within the introns of protein coding genes. miRNAs are first transcribed as primary transcripts or pri-miRNA consisting of RNA transcripts averaging about 1.2 Kb, or within the introns of long protein coding transcripts.

Pri-miRs are processed by Drosha enzymes to short, roughly 70 to 120-nucleotide stem-loop structures, known as pre- or precursor miRNA in the cell nucleus. These pre-miRNAs then are processed to mature functional miRNAs in the cytoplasm by interaction with the endonucleases Argonaut, Dicer, and others to produce the RISC complex.

MicroRNAs generally inhibit translation or promote mRNA degradation by base-pairing to partially complementary sequences within the 3' untranslated regions (UTRs) of regulatory target mRNAs. Individual messenger RNAs (mRNAs) can be targeted by several miRNAs, and a single miRNA can regulate multiple target mRNAs. MicroRNAs can coordinately regulate a set of genes encoding proteins with related functions, providing enormous complexity and the potential of gene regulation.

MicroRNAs can be labeled, used in array analysis, or employed in diagnostic, therapeutic, or prognostic applications, particularly those related to pathological conditions such as inflammatory muscle disease. The RNA may have been endogenously produced by a cell, or been synthesized or produced chemically or by recombinant technology. They may be isolated and/or purified. Human miRNA molecules often are referenced herein with the prefix "hsa-miR-". Unless otherwise indicated, miRNAs referred to in the application are human sequences, and non-human miRNA sequences can be determined and prepared from these (e.g., for applications in non-human subjects).

In some embodiments, a miRNA may used that does not correspond to a known human miRNA. These non-human miRNAs may be used in certain embodiments or there may exist a human miRNA that is homologous to a non-human miRNA. In various embodiments, a mammalian cell, biological sample, or preparation thereof may be employed.

In some embodiments, one or more nucleotides in an miRNA are substituted with another nucleotide, are a modified base, are deleted and/or are inserted (e.g., 1, 2, 3, 4, 5, 6, 7, 8, 9, 10, 11, 12, 13, 14, 15, 16, 17, 18, 19 or 20 nucleotides in the miRNA are substituted, are a modified base, are deleted and/or are inserted) relative to an unmodified reference miRNA (e.g., a native miRNA). The function of such a modified miRNA in vivo or in vitro sometimes is the same as for the reference miRNA, and sometimes is modified (e.g., greater or reduced). A modified function typically is detectable and sometimes is within 100-fold greater (e.g., 2, 4, 6, 8, 10, 15, 20, 25, 30, 35, 40, 45, 50, 55, 60, 65, 70, 75, 80, 85, 90, 95-fold greater) or 100-fold reduced (e.g., 2, 4, 6, 8, 10, 15, 20, 25, 30, 35, 40, 45, 50, 55, 60, 65, 70, 75, 80, 85, 90, 95-fold reduced) of the function elicited by a reference miRNA.

### Nucleic Acid Modification

Any of the modifications described below may be applied to a nucleic acid, such as miRNA and siRNA, as appropriate. Examples of modifications include alterations to the RNA backbone, sugar or base, and various combinations thereof. Any suitable number of backbone linkages, sugars and/or bases in a miRNA or other nucleic acid can be modified (e.g., independently about 5%, 10%, 15%, 20%, 25%, 30%, 35%, 40%, 45%, 50%, 55%, 60%, 65%, 70%, 75%, 80%, 85%, 90%, 95%, up to 100% backbone linkages, sugars and/or bases are modified; or about 1, 2, 3, 4, 5, 6, 7, 8, 9, 10, 11, 12, 13, 14, 15, 16, 17, 18, 19 or 20 backbone linkages, sugars and/or bases are modified). An unmodified miRNA nucleoside is any one of the bases adenine, cytosine, guanine, thymine, or uracil joined to the 1' carbon of beta-D-ribo-furanose.

A modified base is a nucleotide base other than adenine, guanine, cytosine and uracil at a 1' position. Non-limiting examples of modified bases include inosine, purine, pyridin-4-one, pyridin-2-one, phenyl, pseudouracil, 2, 4, 6-trimethoxy benzene, 3-methyl uracil, dihydrouridine, naphthyl, aminophenyl, 5-alkylcytidines (e. g., 5-methylcytidine), 5-alkyluridines (e. g., ribothymidine), 5-halouridine (e. g., 5-bromouridine) or 6-azapyrimidines or 6-alkylpyrimidines (e. g. 6-methyluridine), propyne, and the like. Other non-limiting examples of modified bases include nitropyrrolyl (e.g., 3-nitropyrrolyl), nitroindolyl (e.g., 4-, 5-, 6-nitroindolyl), hypoxanthinyl, isoinosinyl, 2-aza-inosinyl, 7-deaza-inosinyl, nitroimidazolyl, nitropyrazolyl, nitrobenzimidazolyl, nitroindazolyl, aminoindolyl, pyrrolopyrimidinyl, difluorotolyl, 4-fluoro-6-methylbenzimidazole, 4-methylbenzimidazole, 3-methyl isocarbostyrilyl, 5-methyl isocarbostyrilyl, 3-methyl-7-propynyl isocarbostyrilyl, 7-azaindolyl, 6-methyl-7-azaindolyl, imidizopyridinyl, 9-methyl-imidizopyridinyl, pyrrolopyrizinyl, isocarbostyrilyl, 7-propynyl isocarbostyrilyl, propynyl-7-azaindolyl, 2,4,5-trimethylphenyl, 4-methylindolyl, 4,6-dimethylindolyl, phenyl, napthalenyl, anthracenyl, phenanthracenyl, pyrenyl, stilbenyl, tetracenyl, pentacenyl and the like.

In some embodiments, for example, a nucleic acid may comprise modified nucleic acid molecules, with phosphate backbone modifications. Non-limiting examples of backbone modifications include phosphorothioate, phosphorodithioate, methylphosphonate, phosphotriester, morpholino, amidate carbamate, carboxymethyl, acetamidate, polyamide, sulfonate, sulfonamide, sulfamate, formacetal, thioformacetal, and/or alkylsilyl modifications. In certain instances, a ribose sugar moiety that naturally occurs in a nucleoside is replaced with a hexose sugar, polycyclic heteroalkyl ring, or cyclohexenyl group. In certain instances, the hexose sugar is an allose, altrose, glucose, mannose, gulose, idose, galactose, talose, or a derivative thereof. The hexose may be a D-hexose, glucose, or mannose. In certain instances, the polycyclic heteroalkyl group may be a bicyclic ring containing one oxygen atom in the ring. In certain instances, the polycyclic heteroalkyl group is a bicyclo[2.2.1]heptane, a bicyclo[3.2.1]octane, or a bicyclo[3.3.1]nonane.

Nitropyrrolyl and nitroindolyl nucleobases are members of a class of compounds known as universal bases. Universal bases are those compounds that can replace any of the four naturally occurring bases without substantially affecting the melting behavior or activity of the oligonucleotide duplex. In contrast to the stabilizing, hydrogen-bonding interactions associated with naturally occurring nucleobases, oligonucleotide duplexes containing 3-nitropyrrolyl nucleobases may be stabilized solely by stacking interactions. The absence of significant hydrogen-bonding interactions with nitropyrrolyl nucleobases obviates the specificity for a specific complementary base. In addition, 4-, 5- and 6-nitroindolyl display very little specificity for the four natural bases. Procedures for the preparation of 1-(2'-O-methyl-.beta.-D-ribofuranosyl)-5-nitroindole are described in Gaubert, G.; Wengel, J. Tetrahedron Letters 2004, 45, 5629. Other universal bases include hypoxanthinyl, isoinosinyl, 2-aza-inosinyl, 7-deaza-inosinyl, nitroimidazolyl, nitropyrazolyl, nitrobenzimidazolyl, nitroindazolyl, aminoindolyl, pyrrolopyrimidinyl, and structural derivatives thereof.

Difluorotolyl is a non-natural nucleobase that functions as a universal base. Difluorotolyl is an isostere of the natural nucleobase thymine. But unlike thymine, difluorotolyl shows no appreciable selectivity for any of the natural bases. Other aromatic compounds that function as universal bases are 4-fluoro-6-methylbenzimidazole and 4-methylbenzimidazole. In addition, the relatively hydrophobic isocarbostyrilyl derivatives 3-methyl isocarbostyrilyl, 5-methyl isocarbostyrilyl, and 3-methyl-7-propynyl isocarbostyrilyl are universal bases which cause only slight destabilization of oligonucleotide duplexes compared to the oligonucleotide sequence containing only natural bases. Other non-natural nucleobases include 7-azaindolyl, 6-methyl-7-azaindolyl, imidizopyridinyl, 9-methyl-imidizopyridinyl, pyrrolopyrizinyl, isocarbostyrilyl, 7-propynyl isocarbostyrilyl, propynyl-7-azaindolyl, 2,4,5-trimethylphenyl, 4-methylindolyl, 4,6-dimethylindolyl, phenyl, napthalenyl, anthracenyl, phenanthracenyl, pyrenyl, stilbenyl, tetracenyl, pentacenyl, and structural derivates thereof. For a more detailed discussion, including synthetic procedures, of difluorotolyl, 4-fluoro-6-methylbenzimidazole, 4-methylbenzimidazole, and other non-natural bases mentioned above, see: Schweitzer et al., J. Org. Chem., 59:7238-7242 (1994);

In addition, chemical substituents, for example cross-linking agents, may be used to add further stability or irreversibility to the reaction. Non-limiting examples of cross-linking agents include, for example, 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl) dithio]propioimidate.

A nucleotide analog may also include a "locked" nucleic acid. Certain compositions can be used to essentially "anchor" or "lock" an endogenous nucleic acid into a particular structure. Anchoring sequences serve to prevent disassociation of a nucleic acid siNA complex, and thus not only can prevent copying but may also enable labeling, modification, and/or cloning of the endogenous sequence. The locked structure may regulate gene expression (i.e. inhibit or enhance transcription or replication), or can be used as a stable structure that can be used to label or otherwise modify the endogenous nucleic acid sequence, or can be used to isolate the endogenous sequence, i.e. for cloning.

Nucleic acid molecules need not be limited to those molecules containing only RNA or DNA, but further encompass chemically-modified nucleotides and non-nucleotides. The percent of non-nucleotides or modified nucleotides may be from 1% to 100% (e.g., about 5, 10, 15, 20, 25, 30, 35, 40, 45, 50, 55, 60, 65, 70, 75, 80, 85, 90 or 95% are non-nucleotides or modified nucleotides; or about 1, 2, 3, 4, 5, 6, 7, 8, 9, 10, 11, 12, 13, 14, 15, 16, 17, 18, 19 or 20 non-nucleotides or modified nucleotides in the nucleic acid). In certain embodiments, a nucleic acid lacks 2'- hydroxy (2'-OH) containing nucleotides. In certain embodiments a nucleic acid does not require the presence of nucleotides having a 2'- hydroxy group for mediating a function and as such, a nucleic acid may include no ribonucleotides (e. g., nucleotides having a 2'-OH group). Such nucleic acid molecules that do not require the presence of ribonucleotides to support a function can however have an attached linker or linkers or other attached or associated groups, moieties, or chains containing one or more nucleotides with 2'-OH groups. Sometimes nucleic acid molecules can comprise ribonucleotides at about 5, 10, 20, 30, 40, or 50% of the nucleotide positions.

### MicroRNA Biomarkers

Provided herein are microRNA (also referred to herein as "miRNA") biomarkers associated with a degenerative muscle disease (e.g., inflammatory myopathy, myositis, inclusion body myositis). Such miRNA biomarkers generally are under-represented in a degenerative muscle disease. miRNAs can have a nucleotide sequence corresponding to, or derived from, any suitable source, including without limitation, cells from a mammal (e.g., human). MicroRNA translational regulators of mRNA-to-protein translation have been shown to be critical regulators for inflammatory cytokine signaling. Furthermore, MicroRNAs miR-1, -133a, -133b, and -206 have been reported as regulators of the myoblast-to-myocyte differentiation under the control of myoD, Myogenin (MYOG), Myocyte Enhancing Factor 2 (MEF2), and MYF5. These microRNAs play a role in the conserved developmental program of normal muscle of many species including xenopus, zebrafish, chicken, mouse, and human. MicroRNA-1 drives myogenesis by decreasing translation of factors such as HDAC4, which is a repressor of myoblast differentiation.

MicroRNA-1, microRNA-133 and microRNA-206 are provided herein as under-represented biomarkers useful for optimizing treatments of a degenerative muscle disorder. Coding sequences for hsa-miR-206 (miRBase MI0000490; Ensembl miRNA Gene: ENSG00000207604) and hsa-miR-133b (miRBase MI0000822; Ensembl miRNA Gene: ENSG00000199080) are located on chromosome 6, and are spaced about 4.6 kilobases apart. Coding sequences for hsa-miR-1-2 (miRBase MI0000437; Ensembl miRNA Gene: ENSG00000207694) and hsa-miR-133a-1 (miRBase MI0000450; Ensembl miRNA Gene: ENSG00000207786) are located on chromosome 18, and are spaced about 3.3 kilobases apart. Coding sequences for miRNA-1-1 (miRBase MI0000651; Ensembl miRNA Gene: ENSG00000199017) and miRNA-133a-2 (miRBase MI0000451; Ensembl miRNA Gene: ENSG00000207764) are located on chromosome 20, and are spaced about 10.6 kilobases apart. Non-limiting examples of particular human mature miRNA biomarker sequences are as follows:

| | |
|---|---|
| hsa-miR-1-1 | uggaauguaaagaaguauguau |
| hsa-miR-1-2 | uggaauguaaagaaguauguau |
| hsa-miR-206 | uggaauguaaggaagugugugg |
| hsa-miR-133a-1 | uuugguccccuucaaccagcug |
| hsa-miR-133a-2 | uuugguccccuucaaccagcug |
| hsa-miR-133b | uuugguccccuucaaccagcua |

The miRNAs listed above may be accessed on the Ensembl or miRBase database under the accession numbers herein provided. miRNAs may be detected as whole, full-length molecules or fragments of the mature or precursor forms. A miRNA fragment may include less than about 10 nucleic acids or any larger number (e.g., about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 45, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120 nucleotides in length). A fragment may comprise the 3' end of the miRNA strand with any portion of the strand, the 5' end with any portion of the strand, and any center portion of the strand. Total miRNA may be isolated from a sample as known in the art, and commercially available kits are available (e.g., mirVana miRNA Isolation Kit (Ambion AM1560) and the like).

In some embodiments, one or more nucleotides in a miRNA listed above are substituted with another nucleotide, are a modified base, are deleted and/or are inserted into the miRNA listed above (e.g., 1, 2, 3, 4, 5, 6, 7, 8, 9, 10, 11, 12, 13, 14, 15, 16, 17, 18, 19 or 20 nucleotides in the miRNA are substituted, are a modified base, are deleted and/or are inserted into the miRNA listed above). The function of such a modified miRNA in vivo or in vitro sometimes is the same as for the miRNA listed above, and sometimes is modified (e.g., greater or reduced). A modified function typically is detectable and sometimes is within 100-fold greater (e.g., 2, 4, 6, 8, 10, 15, 20, 25, 30, 35, 40, 45, 50, 55, 60, 65, 70, 75, 80, 85, 90, 95-fold greater) or 100-fold reduced (e.g., 2, 4, 6, 8, 10, 15, 20, 25, 30, 35, 40, 45, 50, 55, 60, 65, 70, 75, 80, 85, 90, 95-fold reduced) of the function elicited by a miRNA listed above.

MicroRNA can be isolated and/or synthesized for use in determination methods described herein (e.g., used as an internal or external control). MicroRNA may be isolated using known molecular biology techniques including nucleic acid amplification (e.g., PCR), transfection, hybridization and transduction. In some embodiments miRNAs may be synthesized using synthetic methods known in the art.

### Additional Markers

The presence, absence or progression of muscle cell differentiation may be indicated by various additional markers. Such additional markers may be assessed in methods described herein to provide an indication for adjusting or maintaining a subsequent drug dose. Non-limiting examples of markers linked to undifferentiated myoblasts include, without limitation, HDAC4, PTB (PTBP1), SRF, MYH10. Non-limiting examples of markers linked to myocyte differentiation include miRNA-1, miRNA-133, miRNA-206, TNF-alpha, MyoD, Myogenin, myocyte enhancing factor (MEF2), and Myosin Heavy Chain 1, Myosin Heavy Chain 2, MYF4, MYOD1 at normal levels.

### Sources of Biomarkers

The presence, absence or amount of a biomarker can be determined within a subject (e.g., in situ) or outside a subject (e.g., ex vivo). In some embodiments, presence, absence or amount of a biomarker can be determined in cells (e.g., differentiated cells, stem cells), and in certain embodiments, presence, absence or amount of a biomarker can be determined in a substantially cell-free medium (e.g., in vitro). The term "identifying the presence, absence or amount of a biomarker in a subject" as used herein refers to any method known in the art for assessing the biomarker and inferring the presence, absence or amount in the subject (e.g., in situ, ex vivo or in vitro methods).

A fluid or tissue sample often is obtained from a subject for determining presence, absence or amount ex vivo. Non-limiting parts of the body from which a tissue sample may be obtained include leg, arm, abdomen, upper back, lower back, chest, hand, finger, fingernail, foot, toe, toenail, neck, rectum, nose, throat, mouth, scalp, face, spine, throat, heart, lung, breast, kidney, liver, intestine, colon, pancreas, bladder, cervix, testes, muscle, skin, hair, region of inflammation, region of muscle wasting and the like, in some embodiments. In certain embodiments, muscle may be obtained from any suitable part of the body, including, without limitation, bicep, tricep, deltoid or quadricep. A tissue sample can be obtained by any suitable method known in the art, including, without limitation, biopsy (e.g., shave, punch, incisional, excisional, curettage, fine needle aspirate, scoop, scallop, core needle, vacuum assisted, open surgical biopsies) and the like, in certain embodiments. Examples of a fluid that can be obtained from a subject includes, without limitation, blood, cerbrospinal fluid, spinal fluid, lavage fluid (e.g., bronchoalveolar, gastric, peritoneal, ductal, ear, athroscopic), urine, interstitial fluid, feces, sputum, saliva, nasal mucous, prostate fluid, lavage, semen, lymphatic fluid, bile, tears, sweat, breast milk, breast fluid, fluid from region of inflammation, fluid from region of muscle wasting and the like, in some embodiments.

A sample from a subject may be processed prior to determining presence, absence or amount of a biomarker. For example, a blood sample from a subject may be processed to yield a certain fraction, including without limitation, plasma, serum, buffy coat, red blood cell layer and the like, and biomarker presence, absence or amount can be determined in the fraction. In certain embodiments, a tissue sample (e.g., muscle biopsy sample) can be processed by slicing the tissue sample and observing the sample under a microscope before and/or after the sliced sample is contacted with an agent that visualizes a biomarker (e.g., antibody). In some embodiments, a tissue sample can be exposed to one or more of the following non-limiting conditions: washing, exposure to high salt or low salt solution (e.g., hypertonic, hypotonic, isotonic solution), exposure to shearing conditions (e.g., sonication, press (e.g., French press)), mincing, centrifugation, separation of cells, separation of tissue and the like. In certain embodiments, a biomarker can be separated from tissue and the presence, absence or amount determined in vitro. A sample also may be stored for a period of time prior to determining the presence, absence or amount of a biomarker (e.g., a sample may be frozen, cryopreserved, maintained in a preservation medium (e.g., formaldehyde)).

A sample can be obtained from a subject at any suitable time of collection after a drug is delivered to the subject. For example, a sample may be collected within about one hour after a drug is delivered to a subject (e.g., within about 5, 10, 15, 20, 25, 30, 35, 40, 45, 55 or 60 minutes of delivering a drug), within about one day after a drug is delivered to a subject (e.g., within about 2, 3, 4, 5, 6, 7, 8, 9, 10, 11, 12, 13, 14, 15, 16, 17, 18, 19, 20, 21, 22, 23 or 24 hours of delivering a drug) or within about two weeks after a drug is delivered to a subject (e.g., within about 2, 3, 4, 5, 6, 7, 8, 9, 10, 11, 12, 13 or 14 days of delivering the drug). A collection may be made on a specified schedule including hourly, daily, semi-weekly, weekly, bi-weekly, monthly, bi-monthly, quarterly, and yearly, and the like, for example. If a drug is administered continuously over a time period (e.g., infusion), the delay may be determined from the first moment of drug is introduced to the subject, from the time the drug administration ceases, or a point in-between (e.g., administration time frame midpoint or other point).

### Biomarker Detection

The presence, absence or amount of one or more biomarkers may be determined by any suitable method known in the art, and non-limiting determination methods are described herein. Determining the presence, absence or amount of a biomarker sometimes comprises use of a biological assay. In a biological assay, one or more signals detected in the assay can be converted to the presence, absence or amount of a biomarker. Converting a signal detected in the assay can comprise, for example, use of a standard curve, one or more standards (e.g., internal, external), a chart, a computer program that converts a signal to a presence, absence or amount of biomarker, and the like, and combinations of the foregoing.

Biomarker detected in an assay can be full-length biomarker, a biomarker fragment, an altered or modified biomarker (e.g., biomarker derivative, biomarker metabolite), or sum of two or more of the foregoing, for example. Modified biomarkers often have substantial sequence identity to a biomarker described herein. For example, percent identity between a modified biomarker and a biomarker described herein may be in the range of 15-20%, 20-30%, 31-40%, 41-50%, 51-60%, 61-70%, 71-80%, 81-90% and 91-100%, (e.g. 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, and 100 percent identity). A modified biomarker often has a sequence (e.g., amino acid sequence or nucleotide sequence) that is 90% or more identical to a sequence of a biomarker described herein. Percent sequence identity can be determined using alignment methods known in the art.

Detection of biomarkers may be performed using any suitable method known in the art, including, without limitation, mass spectrometry, antibody assay (e.g., ELISA), nucleic acid affinity, microarray hybridization, Northern blot, reverse PCR and RT-PCR. For example, RNA purity and concentration may be determined spectrophotometrically (260/280>1.9) on a Nanodrop 1000. RNA quality may be assessed using methods known in the art (e.g., Agilent 2100 Bioanalyzer; RNA 6000 Nano LabChip® and the like).

MicroRNA can be isolated and/or synthesized for use in determination and therapeutic methods described herein. MicroRNAs may be isolated using known molecular biology techniques including nucleic acid amplification (e.g., PCR), transfection, and transduction. In some embodiments miRNAs may be synthesized using synthetic methods known in the art.

MicroRNA may be detected using an array. After an array or a set of probes is prepared and/or the nucleic acid in the sample or probe is labeled, the population of target nucleic acids is contacted with the array or probes under hybridization conditions, where such conditions can be adjusted, as desired, to provide for an optimum level of specificity in view of the particular assay being performed. Suitable hybridization conditions are known in the art and reviewed in Sambrook et al. (2001) and WO 95/21944.

A single array or set of probes may be contacted with multiple samples. The samples may be labeled with different labels to distinguish the samples. For example, a single array can be contacted with a muscle tissue sample, and a normal tissue sample. Differences between the samples for particular miRNAs corresponding to probes on the array can be readily ascertained and quantified.

The small surface area of the array permits uniform hybridization conditions, such as temperature regulation and salt content. Moreover, because of the small area occupied by the high density arrays, hybridization may be carried out in extremely small fluid volumes (e.g., about 250 ul or less, including volumes of about or less than about 5, 10, 25, 50, 60, 70, 80, 90, 100 ul, or any range derivable therein). In small volumes, hybridization may proceed very rapidly.

### Indication for Adjusting or Maintaining Subsequent Drug Dose

An indication for adjusting or maintaining a subsequent drug dose can be based on the presence or absence of a biomarker. For example, when (i) low sensitivity determinations of biomarker levels are available, (ii) biomarker levels shift sharply in response to a drug, (iii) low levels or high levels of biomarker are present, and/or (iv) a drug is not appreciably toxic at levels of administration, presence or absence of a biomarker can be sufficient for generating an indication of adjusting or maintaining a subsequent drug dose.

An indication for adjusting or maintaining a subsequent drug dose often is based on the amount or level of a biomarker. An amount of a biomarker can be a mean, median, nominal, range, interval, maximum, minimum, or relative amount, in some embodiments. An amount of a biomarker can be expressed with or without a measurement error window in certain embodiments. An amount of a biomarker in some embodiments can be expressed as a biomarker concentration, biomarker weight per unit weight, biomarker weight per unit volume, biomarker moles, biomarker moles per unit volume, biomarker moles per unit weight, biomarker weight per unit cells, biomarker volume per unit cells, biomarker moles per unit cells and the like. Weight can be expressed as femtograms, picograms, nanograms, micrograms, milligrams and grams, for example. Volume can be expressed as femtoliters, picoliters, nanoliters, microliters, milliliters and liters, for example. Moles can be expressed in picomoles, nanomoles, micromoles, millimoles and moles, for example. In some embodiments, unit weight can be weight of subject or weight of sample from subject, unit volume can be volume of sample from the subject (e.g., blood sample volume) and unit cells can be per one cell or per a certain number of cells (e.g., micrograms of biomarker per 1000 cells). In some embodiments, an amount of biomarker determined from one tissue or fluid can be correlated to an amount of biomarker in another fluid or tissue, as known in the art. For example, if the amount of a biomarker is determined in circulating blood, the amount of the biomarker can be extrapolated to the amount in muscle, in certain embodiments.

An indication for adjusting or maintaining a subsequent drug dose often is generated by comparing a determined level of biomarker in a subject to a predetermined level of biomarker. A predetermined level of biomarker sometimes is linked to a therapeutic or efficacious amount of drug in a subject (e.g., in muscle of a subject), sometimes is linked to a toxic level of a drug, sometimes is linked to presence of a condition, sometimes is linked to a treatment midpoint and sometimes is linked to a treatment endpoint, in certain embodiments. A predetermined level of a biomarker sometimes includes time as an element, and in some embodiments, a threshold is a time-dependent signature.

For example, a TNF-alpha level of about 18-fold more than a normal level, or greater (e.g., 14-fold to 22-fold more than a normal level or greater; about 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55-fold more than a normal level), may indicate that the dosage of the drug should be increased in a subsequent administration. A TNF-alpha level less than about 18-fold more than a normal level (e.g., less than 14-fold to 22-fold more than a normal level; about 22, 21, 20, 19, 18, 17, 16, 15, 14, 13, 12, 11, 10, 9, 8, 7, 6, 5, 4, 3, 2 or 1-fold more than a normal level, or less than or equal to a normal level) may indicate that the dosage may be maintained or decreased in a subsequent administration. A GM-CSF level of about 63-fold more than a normal level, or greater (e.g., 55-fold to 70-fold more than a normal level or greater; about 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89 or 90-fold more than a normal level or more) may indicate that the dosage of the drug should be increased in a subsequent administration. A GM-CSF level less than about 63-fold more than a normal level (e.g., less than 55-fold to 70-fold more than a normal level; about 70, 69, 68, 67, 66, 65, 64, 63, 62, 61, 60, 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2 or 1-fold more than a normal level or less, or less than or equal to a normal level) may indicate that the dosage may be maintained or decreased in a subsequent administration. A normal level of TNF-alpha or GM-CSF may be assessed in a subject not diagnosed with a muscle disorder under treatment in a patient.

A miRNA-1 level of about 9-fold lower than a normal level, or less (e.g., less than 5-fold to 13-fold lower than a normal level; about 5, 6, 7, 8, 9, 10, 11, 12, 13, 14, 15, 16, 17, 18, 19 or 20-fold lower than a normal level may indicate that the dosage of the drug should be increased in a subsequent administration. A miRNA-1 level greater than about 9-fold lower than a normal level (e.g., greater than 5-fold to 13-fold lower than a normal level; about 13, 12, 11, 10, 9, 8, 7, 6, 5, 4, 3, 2 or 1-fold lower than a normal level, or greater than or equal to a normal level) may indicate that the drug dosage should be maintained or decreased in a subsequent administration.

A miRNA-133a level of about 5-fold lower than a normal level, or less (e.g., less than 2-fold to 8-fold lower than a normal level; about 2, 3, 4, 5, 6, 7, 8, 9, 10, 11, 12, 13, 14, 15-fold lower than a normal level) may indicate that a drug dosage should be increased in a subsequent administration. A miRNA-133a level greater than about 5-fold lower than a normal level (e.g., greater than 2-fold to 8-fold lower than a normal level; about 8, 7, 6, 5, 4, 3, 2, or 1-fold lower than a normal level, or greater than or equal to a normal level) may indicate that the drug dosage should be maintained or decreased in a subsequent administration.

A miRNA-133b level of about 8-fold lower than a normal level, or less (e.g., less than 4-fold to 12-fold lower than a normal level; about 4, 5, 6, 7, 8, 9, 10, 11, 12, 13, 14, 15, 16, 17, 18, 19, 20, 21, 22, 23 or 24-fold, lower than a normal level) may indicate that the drug dosage should be increased in a subsequent administration. A miRNA-133b level greater than about 8-fold lower than a normal level (e.g., greater than 4-fold to 12-fold lower than a normal level; 12, 11, 10, 9, 8, 7, 6, 5, 4, 3, 2 or 1-fold lower than a normal level, or greater than or equal to a normal level) may indicate that the drug dosage should be maintained or decreased in a subsequent administration.

A miRNA-206 level of about 2-fold lower than a normal level, or less (e.g., less than 1.2-fold to 5-fold lower than a normal level; about 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3, 4, 5, 6, 7, 8, 9, 10-fold lower than a normal level) may indicate that the drug dosage should be increased in a subsequent administration. A miRNA-206 level greater than 2-fold lower than a normal level (e.g., greater than 1.2-fold to 5-fold lower than a normal level; 5, 4, 3, 2 or 1-fold lower than a normal level, or greater than or equal to a normal level) may indicate that the drug dosage should be maintained or decreased in a subsequent administration.

A normal level of miRNA-1, miRNA-133a, miRNA-133b or miRNA-206 may be assessed in a subject not diagnosed with a muscle disorder under treatment in a patient.

Some treatment methods comprise (i) administering a drug to a subject in one or more administrations (e.g., 1, 2, 3, 4, 5, 6, 7, 8, 9 or 10 doses), (ii) determining the presence, absence or amount of a biomarker in or from the subject after (i), (iii) providing an indication of increasing, decreasing or maintaining a subsequent dose of the drug for administration to the subject, and (iv) optionally administering the subsequent dose to the subject, where the subsequent dose is increased, decreased or maintained relative to the earlier dose(s) in (i). In some embodiments, presence, absence or amount of a biomarker is determined after each dose of drug has been administered to the subject, and sometimes presence, absence or amount of a biomarker is not determined after each dose of the drug has been administered (e.g., a biomarker is assessed after one or more of the first, second, third, fourth, fifth, sixth, seventh, eighth, ninth or tenth dose, but not assessed every time after each dose is administered).

An indication for adjusting a subsequent drug dose can be considered a need to increase or a need to decrease a subsequent drug dose. An indication for adjusting or maintaining a subsequent drug dose can be considered by a clinician, and the clinician may act on the indication in certain embodiments. In some embodiments, a clinician may opt not to act on an indication. Thus, a clinician can opt to adjust or not adjust a subsequent drug dose based on the indication provided.

An indication for adjusting a drug dose or subsequent drug dose can be carried out by adjusting the dose of the drug or the schedule of the dosing. Thus, an indication for adjusting or maintaining a subsequent drug dose schedule can be considered by a clinician, and the clinician may act on the indication in certain embodiments. In some embodiments, a clinician may opt not to act on an indication. Thus, a clinician can opt to adjust or not adjust a drug dose schedule or subsequent drug dose schedule based on the indication provided.

An indication of adjusting or maintaining a subsequent drug dose, and/or the subsequent drug dosage, can be provided in any convenient manner. An indication may be provided in tabular form (e.g., in a physical or electronic medium) in some embodiments. For example, a biomarker threshold may be provided in a table, and a clinician may compare the presence, absence or amount of the biomarker determined for a subject to the threshold. The clinician then can identify from the table an indication for subsequent drug dose. In certain embodiments, an indication can be presented (e.g., displayed) by a computer after the presence, absence or amount of a biomarker is provided to computer (e.g., entered into memory on the computer). For example, presence, absence or amount of a biomarker determined for a subject can be provided to a computer (e.g., entered into computer memory by a user or transmitted to a computer via a remote device in a computer network), and software in the computer can generate an indication for adjusting or maintaining a subsequent drug dose, and/or provide the subsequent drug dose amount. A subsequent dose can be determined based on certain factors other than biomarker presence, absence or amount, such as weight of the subject, one or more metabolite levels for the subject (e.g., metabolite levels pertaining to liver function) and the like, for example.

Once a subsequent dose is determined based on the indication, a clinician may administer the subsequent dose or provide instructions to adjust the dose to another person or entity. The term "clinician" as used herein refers to a decision maker, and a clinician is a medical professional in certain embodiments. A decision maker can be a computer or a displayed computer program output in some embodiments, and a health service provider may act on the indication or subsequent drug dose displayed by the computer. A decision maker may administer the subsequent dose directly (e.g., infuse the subsequent dose into the subject) or remotely (e.g., pump parameters may be changed remotely by a decision maker).

A subject can be prescreened to determine whether or not the presence, absence or amount of a particular biomarker should be determined. Non-limiting examples of prescreens include identifying the presence or absence of a genetic marker (e.g., polymorphism, particular nucleotide sequence); identifying the presence, absence or amount of a particular metabolite (e.g., a metabolite indicative of muscle activity, muscle integrity, muscle wasting, liver activity, kidney activity). A prescreen result can be used by a clinician in combination with the presence, absence or amount of a biomarker to determine whether a subsequent drug dose should be adjusted or maintained.

### Nucleic Acid Preparation

In some embodiments, a nucleic acid is provided for use as a control or standard in an assay, or therapeutic, for example. A nucleic acid may be made by any technique known in the art, such as for example, chemical synthesis, enzymatic production or biological production. Nucleic acids may be recovered or isolated from a biological sample. The nucleic acid may be recombinant or it may be natural or endogenous to the cell (produced from the cell's genome). It is contemplated that a biological sample may be treated in a way so as to enhance the recovery of small nucleic acid molecules such as miRNA. Generally, methods may involve lysing cells with a solution having guanidinium and a detergent.

Nucleic acid synthesis may also be performed according to standard methods. Non-limiting examples of a synthetic nucleic acid (e.g., a synthetic oligonucleotide), include a nucleic acid made by in vitro chemical synthesis using phosphotriester, phosphite, or phosphoramidite chemistry and solid phase techniques or via deoxynucleoside H-phosphonate intermediates. Various different mechanisms of oligonucleotide synthesis have been disclosed elsewhere.

Nucleic acids may be isolated using known techniques. In particular embodiments, methods for isolating small nucleic acid molecules, and/or isolating RNA molecules can be employed. Chromatography is a process used to separate or isolate nucleic acids from protein or from other nucleic acids. Such methods can involve electrophoresis with a gel matrix, filter columns, alcohol precipitation, and/or other chromatography. If a nucleic acid, for example miRNA, from cells is to be used or evaluated, methods generally involve lysing the cells with a chaotropic (e.g., guanidinium isothiocyanate) and/or detergent (e.g., N-lauroyl sarcosine) prior to implementing processes for isolating particular populations of RNA.

In particular methods for separating, for example, a miRNA from other nucleic acids, a gel matrix may be prepared using polyacrylamide, though agarose can also be used. The gels may be graded by concentration or they may be uniform. Plates or tubing can be used to hold the gel matrix for electrophoresis. Usually one-dimensional electrophoresis is employed for the separation of nucleic acids. Plates are used to prepare a slab gel, while the tubing (glass or rubber, typically) can be used to prepare a tube gel. The phrase "tube electrophoresis" refers to the use of a tube or tubing, instead of plates, to form the gel. Materials for implementing tube electrophoresis can be readily prepared by a person of skill in the art or purchased, such as from C.B.S. Scientific Co., Inc. or Scie-Plas.

Methods may involve the use of organic solvents and/or alcohol to isolate nucleic acids, particularly miRNA used in methods and compositions herein provided. Generally, small RNA molecules may be isolated from cells by methods comprising: adding an alcohol solution to a cell lysate and applying the alcohol/lysate mixture to a solid support before eluting the RNA molecules from the solid support. In some embodiments, the amount of alcohol added to a cell lysate achieves an alcohol concentration of about 55% to 60%. While different alcohols can be employed, ethanol works well. A solid support may be any structure, and it includes beads, filters, and columns, which may include a mineral or polymer support with electronegative groups. A glass fiber filter or column is effective for such isolation procedures.

A nucleic acid isolation processes may sometimes include: a) lysing cells in the sample with a lysing solution comprising guanidinium, where a lysate with a concentration of at least about 1 M guanidinium is produced; b) extracting nucleic acid molecules from the lysate with an extraction solution comprising phenol; c) adding to the lysate an alcohol solution for form a lysate/alcohol mixture, wherein the concentration of alcohol in the mixture is between about 35% to about 70%; d) applying the lysate/alcohol mixture to a solid support; e) eluting the nucleic acid molecules from the solid support with an ionic solution; and, f) capturing the nucleic acid molecules. The sample may be dried down and resuspended in a liquid and volume appropriate for subsequent manipulation.

### Drugs

Any suitable drug can be employed in the disclosed methods. Such drugs include but are not limited to antibodies, biological active fragments and derviatives thereof as well as small molecules.

In some embodiments, an antibody or small molecule is provided for use as a control or standard in an assay, or a therapeutic, for example. In some embodiments, an antibody or other small molecule configured to bind to a cytokine or cytokine receptor, including without limitation GM-CSF, TNF-alpha or NF-kappa-B modulating agent, and alter the action of the cytokine. In certain embodiments an antibody or other small molecule may bind to an mRNA structure encoding for a cytokine or receptor.

The term small molecule as used herein means an organic molecule of approximately 800 or fewer Daltons. In certain embodiments small molecules may diffuse across cell membranes to reach intercellular sites of action. In some embodiments a small molecule binds with high affinity to a biopolymer such as protein, nucleic acid, or polysaccharide and may sometimes alter the activity or function of the biopolymer. In various embodiments small molecules may be natural (such as secondary metabolites) or artificial (such as antiviral drugs); they may have a beneficial effect against a disease (such as drugs) or may be detrimental (such as teratogens and carcinogens). By way of non-limiting example, small molecules may include ribo- or deoxyribonucleotides, amino acids, monosaccharides and small oligomers such as dinucleotides, peptides such as the antioxidant glutathione, and disaccharides such as sucrose.

The term antibody as used herein is to be understood as meaning a gamma globulin protein found in blood or other bodily fluids of vertebrates, and used by the immune system to identify and neutralize foreign objects, such as bacteria and viruses. Antibodies typically include basic structural units of two large heavy chains and two small light chains.

Specific binding to an antibody requires an antibody that is selected for its affinity for a particular protein. For example, polyclonal antibodies raised to a particular protein, polymorphic variants, alleles, orthologs, and conservatively modified variants, or splice variants, or portions thereof, can be selected to obtain only those polyclonal antibodies that are specifically immunoreactive with GM-CSF, TNF-alpha or NF-kappa-B modulating protein and not with other proteins. This selection may be achieved by subtracting out antibodies that cross-react with other molecules.

A drug may be an antibody or a fragment thereof. Antibodies sometimes are IgG, IgM, IgA, IgE, or an isotype thereof (e.g., IgG1, IgG2a, IgG2b or IgG3), sometimes are polyclonal or monoclonal, and sometimes are chimeric, humanized or bispecific versions of such antibodies. Polyclonal and monoclonal antibodies that bind specific antigens are commercially available, and methods for generating such antibodies are known. In general, polyclonal antibodies are produced by injecting an isolated antigen into a suitable animal (e.g., a goat or rabbit); collecting blood and/or other tissues from the animal containing antibodies specific for the antigen and purifying the antibody. As used herein, the term "monoclonal" is to be understood as designating an antibody (or its corresponding fragment) arising from a single clone of an antibody-producing cell such as a B cell, and recognizing a single epitope on the antigen bound. Methods for generating monoclonal antibodies, in general, include injecting an animal with an isolated antigen (e.g., often a mouse or a rat); isolating splenocytes from the animal; fusing the splenocytes with myeloma cells to form hybridomas; isolating the hybridomas and selecting hybridomas that produce monoclonal antibodies which specifically bind the antigen (e.g., Kohler & Milstein, Nature 256:495 497 (1975) and StGroth & Scheidegger, J Immunol Methods 5:1 21 (1980)). Examples of monoclonal antibodies are anti MDM 2 antibodies, anti-p53 antibodies (pAB421, DO 1, and an antibody that binds phosphoryl-ser15), anti-dsDNA antibodies and anti-BrdU antibodies, are described hereafter.

Methods for generating chimeric and humanized antibodies also are known (see, e.g., U.S. patent No. 5,530,101 (Queen, et al.), U.S. patent No. 5,707,622 (Fung, et al.) and U.S. patent Nos. 5,994,524 and 6,245,894 (Matsushima, et al.)), which generally involve transplanting an antibody variable region from one species (e.g., mouse) into an antibody constant domain of another species (e.g., human). Antigen-binding regions of antibodies (e.g., Fab regions) include a light chain and a heavy chain, and the variable region is composed of regions from the light chain and the heavy chain. Given that the variable region of an antibody is formed from six complementarity-determining regions (CDRs) in the heavy and light chain variable regions, one or more CDRs from one antibody can be substituted (i.e., grafted) with a CDR of another antibody to generate chimeric antibodies. Also, humanized antibodies are generated by introducing amino acid substitutions that render the resulting antibody less immunogenic when administered to humans.

The drug sometimes is an antibody fragment, such as a Fab, Fab', F(ab)'₂, Dab, Fv or single-chain Fv (ScFv) fragment, and methods for generating antibody fragments are known (see, e.g., U.S. Patent Nos. 6,099,842 and 5,990,296 and PCT/GB00/04317). In some embodiments, a binding partner in one or more hybrids is a single-chain antibody fragment, which sometimes are constructed by joining a heavy chain variable region with a light chain variable region by a polypeptide linker (e.g., the linker is attached at the C-terminus or N-terminus of each chain) by recombinant molecular biology processes. Such fragments often exhibit specificities and affinities for an antigen similar to the original monoclonal antibodies. Bifunctional antibodies sometimes are constructed by engineering two different binding specificities into a single antibody chain and sometimes are constructed by joining two Fab' regions together, where each Fab' region is from a different antibody (e.g., U.S. Patent No. 6,342,221). Antibody fragments often comprise engineered regions such as CDR-grafted or humanized fragments. In certain embodiments the drug is an intact immunoglobulin, and in some embodiments the drug may be a Fab monomer or a Fab dimer.

In various embodiments, the antibody or fragment thereof specifically binds to an epitope, including in some embodiments to a discontinuous epitope, of GM-CSF, TNF-alpha, NF-kappa-B modulating agent or other cytokine. In some embodiments antibodies may be configured to recognize GM-CSF, TNF-alpha, or NF-kappa-B modulating agent highly specifically, that is to say that from a mixture of the target molecule and other molecules. This means that, for example, a monoclonal antibody or fragment thereof according to these embodiments, when administered to a subject, may be expected to specifically bind to and neutralize only the desired target, whereas other undesired targets are neither bound nor neutralized. In certain embodiments an antibody drug may bind to a GM-CSF, TNF-alpha or NF-kappa-B modulating agent with extremely high affinity, meaning that that once the complex between a monoclonal antibody or fragment thereof on the one hand and the target molecule on the other hand is formed, it does not readily, or at least does not quickly separate.

A drug sometimes is an Immunosuppressive drug. Immunosuppressive drugs typically inhibit or prevent at least one activity of the immune system. Activities include but are not limited to cytokine production, cellular cytotoxicity, activation and/or inhibition of macrophages, activation and/or inhibition of dendritic cells and the like. In general, such drugs can be categorized as glucocorticoids, cytostatics, antibodies, drugs acting on immunophilins, among others. Immunosuppressive drugs include, without limitation: Azathioprine, Mycophenolic acid, Leflunomide, Teriflunomide, Methotrexate FKBP/Cyclophilin/Calcineurin, Tacrolimus, Cyclosporine, Pimecrolimus, Abetimus, Gusperimus, Thalidomide, Lenalidomide, Anakinra, Sirolimus, Deforolimus, Everolimus, Temsirolimus, Zotarolimus Complement component 5(Eculizumab), TNFs (Infliximab, Adalimumab, Certolizumab pegol, Afelimomab, Golimumab), Interleukin 5 (Mepolizumab), Immunoglobulin E (Omalizumab), BAYX (Nerelimomab), Interferon (Faralimomab), IL-6 (Elsilimomab), IL-12 and IL-13 (Ustekinumab), CD3 (Muromonab-CD3, Otelixizumab, Teplizumab, Visilizumab), CD4 (Clenoliximab, Keliximab, Zanolimumab), CD11a (Efalizumab), CD18 (Erlizumab), CD20 (Afutuzumab, Ocrelizumab, Pascolizumab), CD23 (Lumilixim), CD40 (Teneliximab, Toralizumab), CD62L/L-selectin (Aselizumab), CD80 (Galiximab), CD147/Basigin (Gavilimomab, Ziralimumab), CD154 (Ruplizumab), BLyS (Belimumab), CTLA-4 (Ipilimumab, Tremelimumab), CAT (Bertilimumab, Lerdelimumab, Metelimumab), Integrin (Natalizumab), Interleukin-6 receptor (Tocilizumab), LFA-1 (Odulimomab), IL-2 receptor/CD25 (Basiliximab, Daclizumab, Inolimomab), T-lymphocyte (Zolimomab aritox), Atlizumab, Atorolimumab, Cedelizumab, Dorlimomab aritox, Dorlixizumab, Fontolizumab, Gantenerumab, Gomiliximab, Lebrilizumab, Maslimomab, Morolimumab, Pexelizumab, Reslizumab, Rovelizumab, Siplizumab, Talizumab, Telimomab aritox, Vapaliximab, Vepalimomab, Anti-thymocyte globulin, Antilymphocyte globulin CTLA-4 (Abatacept, Belatacept), Aflibercept, Alefacept, Rilonacept, and TNF inhibitor (Etanercept).

### Pharmaceutical Formulation

The phrase "pharmaceutically acceptable" refers to molecular entities and compositions that do not produce an allergic or other untoward reaction when administered to a human.
Solutions of active pharmaceutical agents described herein can be prepared as free base or pharmacologically acceptable salts. Such agents also may be prepared in water suitably mixed with a surfactant, such as hydroxypropylcellulose, in some embodiments. Dispersions may also be prepared in glycerol, liquid polyethylene glycols, mixtures thereof, and in oils. Under ordinary conditions of storage and use, these preparations can contain a preservative to prevent the growth of microorganisms. The pharmaceutical forms suitable for injectable use include sterile aqueous solutions or dispersions and sterile powders for the extemporaneous preparation of sterile injectable solutions or dispersions. The form is often sterile and fluid to the extent that easy syringability exists. It may be stable under the conditions of manufacture and storage and may be preserved against the contaminating action of microorganisms, such as bacteria and fungi. The carrier can be a solvent or dispersion medium containing, for example, water, ethanol, polyol (e.g., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases isotonic agents, for example sugars or sodium chloride, may be included. Prolonged absorption of the injectable compositions can be brought about by the use in the compositions of agents delaying absorption, for example, aluminum monostearate and gelatin.

In certain formulations, a water-based formulation is employed while in others, it may be lipid-based. In particular embodiments, a composition comprising an active pharmaceutical agent or a nucleic acid encoding the same is in a water-based formulation. In other embodiments, the formulation is lipid based.

For parenteral administration in an aqueous solution, for example, the solution is often suitably buffered if necessary and the liquid diluent first rendered isotonic with sufficient saline or glucose. These particular aqueous solutions are suitable for intravenous, intramuscular, subcutaneous, intralesional, and intraperitoneal administration. In this connection, sterile aqueous media which can be employed are known to those of skill in the art in light of the present disclosure. For example, one dosage may be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage may necessarily occur depending on the condition of the subject being treated. The person responsible for administration may, in any event, determine the appropriate dose for the individual subject. Moreover, for human administration, preparations should meet sterility, pyrogenicity, general safety and purity standards as required by FDA Office of Biologics standards.

As used herein, a "carrier" includes any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

### Drug Administration

A drug can be administered to any appropriate subject having a biomarker or needing treatment for a condition described herein. Non-limiting examples of a subject include mammal, human, ape, monkey, ungulate (e.g., equine, bovine, caprine, ovine, porcine, buffalo, camel and the like), canine, feline, rodent (e.g., murine, mouse, rat) and the like. A subject may be male or female, and a drug can be administered to a subject in a particular age group, including, for example, juvenile, pediatric, adolescent, adult, and the like.

Any suitable drug can be administered for treating a muscle degenerative disease. In some embodiments, a drug may exert immunosuppressive effects. A drug, in certain embodiments, comprises as an active ingredient an antibody, antibody fragment, single-chain antibody, small molecule, a nucleic acid, nucleic acid derivative, miRNA, (including, without limitation, miR-1, miR-133, miR-206 an associated subtype, or combination of the foregoing) siNA, peptide, polypeptide, protein antibody, antibody fragment, single-chain antibody, small molecule, and the like. Various forms of miRNA or siRNA may be delivered, including post-processed miRNA or siRNA, pre-processed miRNA or siRNA or vector that encodes pre-processed or post-processed miRNA or siRNA. In certain embodiments a drug active ingredient sometimes interacts with a biomarker described herein, sometimes is capable of specifically binding to the biomarker, sometimes modulates the expression, persistence or level of the biomarker, and sometimes is capable of modifying the structure of the biomarker, in certain embodiments.

Methods as presented herein include without limitation the delivery of an effective amount of a nucleic or an expression construct encoding the same. An "effective amount" of the pharmaceutical composition, generally, is defined as that amount sufficient to detectably and repeatedly to achieve the stated desired result, for example, to ameliorate, reduce, minimize or limit the extent of the disease or its symptoms. Other more rigorous definitions may apply, including elimination, eradication or cure of disease. In some embodiments there may be a step of monitoring the biomarkers to evaluate the effectiveness of treatment and to control toxicity.

Drugs are administered in a manner compatible with the dosage formulation, and in such amount as may be therapeutically effective. Injection of nucleic acids may be delivered by syringe or any other method used for injection of a solution, as long as the nucleic acid and any associated components can pass through the particular gauge of needle required for injection. A syringe system has also been described for use in gene therapy that permits multiple injections of predetermined quantities of a solution precisely at any depth (U.S. Pat. No. 5,846,225).

The quantity to be administered depends on the subject to be treated, including, e.g., the aggressiveness of the disease, the size of the affected area, and the previous or other courses of treatment. Precise amounts of active ingredient required to be administered depend on the judgment of the practitioner. Suitable regimes for initial administration and subsequent administration are also variable, but are typified by an initial administration followed by other administrations. Such administration may be systemic, as a single dose, continuous over a period of time spanning 10, 20, 30, 40, 50, 60 minutes, and/or 1, 2, 3, 4, 5, 6, 7, 8, 9, 10, 11, 12, 13, 14, 15, 16, 17, 18, 19, 20, 21, 22, 23, 24 or more hours, and/or 1, 2, 3, 4, 5, 6, 7, days or more. Moreover, administration may be through a time release or sustained release mechanism, implemented by formulation and/or mode of administration.

The routes of administration may vary with the location and nature of the site to be targeted, and include, e.g., intramuscular, intradermal, subcutaneous, regional, parenteral, intravenous, intranasal, systemic, and oral administration and formulation. Injection may be direct injection, intramuscular injection, or injection into vasculature of the affected muscle. Local, regional, or systemic administration also may be appropriate. Multiple injections delivered as a single dose may comprise about 0.1 to about 0.5 ml volumes. Drugs may be administered in multiple injections to a targeted site. In certain aspects, injections may be spaced at approximately 1 cm intervals.

For example, an affected muscle may be injected or perfused with a formulation comprising a nucleic acid, other drug, or combinations thereof. Administration may be continued, for example, by leaving a catheter implanted at the site. Periodic treatment also is envisioned. Continuous perfusion of an expression construct or a viral construct also is contemplated.

Continuous administration also may be applied where appropriate, where targeted site is treated to eliminate residual, microscopic disease. Delivery via syringe or catherization is contemplated. Such continuous perfusion may take place for a period from about 1-2 hours, to about 2-6 hours, to about 6-12 hours, to about 12-24 hours, to about 1-2 days, to about 1-2 wk or longer following the initiation of treatment. Generally, the dose of the therapeutic composition via continuous perfusion may be equivalent to that given by a single or multiple injections, adjusted over a period of time during which the perfusion occurs.

Treatment regimens may vary as well and often depend on the muscle disease type, location, immune condition, target site, disease progression, and health and age of the patient. Certain muscle disease types may require more aggressive treatment. The clinician may be best suited to make such decisions based on the known efficacy and toxicity (if any) of the therapeutic formulations.

Treatments may include various "unit doses." A unit dose is defined as containing a predetermined quantity of a drug. The quantity to be administered, and the particular route and formulation, are within the skill of those in the clinical arts. A unit dose need not be administered as a single injection but may comprise continuous infusion over a set period of time. With respect to a viral component as presented herein, a unit dose may conveniently be described in terms of mg of nucleic acid or nucleic acid mimetic. Alternatively, the amount specified may be the amount administered as the average daily, average weekly, or average monthly dose.

Nucleic acid or other drug can be administered to the patient in a dose or doses of about or of at least about 0.5, 1, 5, 10, 15, 20, 25, 30, 35, 40, 45, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000 nanogram, microgram or milligram, or more, or any range derivable therein. Alternatively, the amount specified may be the amount administered as the average daily, average weekly, or average monthly dose, or it may be expressed in terms of mg/kg, where kg refers to the weight of the patient and the mg is specified above. In other embodiments, the amount specified is any number discussed above but expressed as mg/m.sup.2.

Some embodiments involve drug dose escalation, where (i) a relatively low dose of a drug is administered to a subject, (ii) presence, absence or amount of a biomarker is assessed, and if the assessment indicates there is no significant efficacious effect of the drug, then (iii) administering a subsequent higher dose of the drug to a subject, where (ii) and (iii) are repeated until a therapeutic effect is observed (e.g., the therapeutic effect may be observed based on the biomarker assessment). Such an approach can allow a clinician to "dial-in" an efficacious amount of a drug for a subject and minimize toxic side effects associated with higher amounts of the drug. A clinician, in some embodiments, may have information pertaining to the amount of drug administered that is likely to result in a significant toxic side effect for subjects and cease administration of drug if a subsequent dose is increased and is expected to have significant toxic side effects (e.g., a clinician may cease administration at or near a specified toxic threshold dose of the drug).

Administration can be in vivo, ex vivo or in vitro. A drug may be prepared as a pharmaceutically acceptable salt in some embodiments. A drug may be prepared as a pharmaceutically acceptable formulation, in certain embodiments, that comprises, for example, one or more of a liposome or other polymatrix, a penetration enhancer, surfactant, fatty acid, bile salt, carrier, excipient, adjuvant and the like.

For in vivo administration, a drug may be formulated for any convenient route of administration, including, without limitation, nasal, topical, oral, pulmonary, parenteral, intrathecal, and intranutrical administration. A drug can be prepared in a unit dosage form for systemic administration, in some embodiments, and may be incorporated into a hard or soft shell gelatin capsule, may be compressed into a tablet, or may be incorporated directly in food of subject's diet, for example. For oral therapeutic administration, a drug may be combined with one or more excipients and used in the form of ingestible tablets, buccal tablets, troches, capsules, elixirs, suspensions, syrups, wafers and the like, in certain embodiments. Such preparations sometimes contain at least 0.001% to 0.1% of active drug by weight and sometimes between about 0.1% to about 60% of the weight of the given unit dosage form.

For in vitro administration a drug may be delivered to cells by any convenient method. For example, a drug may be formulated as described herein, and in some embodiments, a pharmaceutical formulation (e.g., nucleic acid drug) may be exposed to calcium phosphate or calcium chloride co-precipitation, transduction/infection, DEAE-dextran-mediated transfection, lipofection, electroporation, and iontophoresis.

In some embodiments, in vitro administration may be applicable to methods pertaining to drug screening or selection. In certain embodiments, such methods comprise (i) contacting muscle cells with a drug in vitro, (ii) determining the presence, absence or amount of a biomarker associated with a muscle degenerative disorder, and (iii) selecting a drug for further screening or administration to a subject having a muscle degenerative disorder based on the presence, absence or amount of the biomarker. In some embodiments, a drug is selected for further screening or administration if the amount of an over-represented biomarker is reduced or is absent. In certain embodiments, a drug is selected for further screening if an under-represented biomarker is increased or present.

Muscle cells may be in part undifferentiated (e.g., myoblasts) or may be differentiated (e.g., myocytes). Muscle cells may be obtained from primary culture of muscle tissue, where the muscle tissue is from any suitable source of the body (e.g., skeletal muscle, smooth muscle, bicep, triceps, quadriceps, deltoid and the like) in certain embodiments. Muscle cells may be obtained from other cell cultures, including, without limitation, human skeletal muscle myoblasts (HSMM; Lonza CC-2580), skeletal muscle myocytes (SkMC; Lonza CC-2561), and the like.

### Combination Therapy

A drug suitable for treating a muscle degenerative disorder sometimes is administered in combination with one or more other drugs or inactive ingredients. One or more of the other drugs may also treat a muscle degenerative disorder in some embodiments, and sometimes, one or more of the other drugs may not specifically treat a muscle degenerative disorder. One or more inactive ingredients may treat a side effect of an active agent that treats the muscle degenerative disorder (e.g., anti-diuretic, anti-nausea, anti-diarrhea, depressant, stimulant and the like), for example. An additional drug may also enhance the curative effect of the primary drug. A drug can be administered to any appropriate subject having a muscular disease as described herein. Non-limiting examples of a subject include mammal, human, ape, monkey, ungulate (e.g., equine, bovine, caprine, ovine, porcine, buffalo, camel and the like), canine, feline, rodent (e.g., murine, mouse, rat) and the like. A subject may be male or female, and a drug can be administered to a subject in a particular age group, including, for example, juvenile, pediatric, adolescent, adult and the like.

In certain embodiments, the compositions and methods as herein provided involve a miRNA or a functional fragment thereof, or expression construct encoding such. These miRNA compositions can be used in combination with additional therapies to enhance the effect of the miRNA therapy, or increase the therapeutic effect of another therapy being employed. These compositions would be provided in a combined amount effective to achieve the desired effect, such as the elimination or amelioration of muscle disease. This process may involve administering the miRNA or additional therapy at the same or different time. This may be achieved by administering one or more compositions or pharmacological formulation that includes or more of the agents, or by administering two or more distinct compositions or formulations, wherein one composition provides (1) miRNA; and/or (2) a second therapy.

In some embodiments a patient may receive the miRNA therapy and the additional therapy within about 12-24 h of each other and, more preferably, within about 6-12 h of each other. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations.

In certain embodiments, a course of treatment may last 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 days or more. For example, one agent may be given on day 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, and/or 90, any combination thereof, and another agent is given on day 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, and/or 90, or any combination thereof. Within a single day (24-hour period), the patient may be given one or multiple administrations of the agent(s). Moreover, after a course of treatment, there may be a period of time at which no treatment is administered. This time period may last 1, 2, 3, 4, 5, 6, 7 days, and/or 1, 2, 3, 4, 5 weeks, and/or 1, 2, 3, 4, 5, 6, 7, 8, 9, 10, 11, 12 months or more, depending on the condition of the patient, such as their prognosis, strength, health, etc.

Various combinations may be employed. For example miRNA therapy is "A" and a second therapy is "B" in the following combination embodiments.
A/B/A B/A/B B/B/A A/A/B A/B/B B/A/A A/B/B/B B/A/B/B
B/B/B/A B/B/A/B A/A/B/B A/B/A/B A/B/B/A B/B/A/A
B/A/B/A B/A/A/B A/A/A/B B/A/A/A A/B/A/A A/A/B/A

In some embodiments a combination may include miRNA therapy "A" and additional therapies "B," "C," and so forth in any order of administration. Administration of any drug or therapy herein provided to a patient may follow general protocols for the administration of such compounds, taking into account the toxicity, if any, of the vector or any protein or other agent. Therefore, in some embodiments there is a step of monitoring toxicity that is attributable to combination therapy. It is expected that the treatment cycles would be repeated as necessary. It also is contemplated that various standard therapies, as well as surgical intervention, may be applied in combination with the described therapy.

In certain embodiments an additional therapy, such as in non-limiting example, an immunosuppressive drug, is employed in combination with the drug therapy provided herein. Immunosuppressive drugs typically inhibit or prevent activity of the immune system. In general, such drugs can be categorized as glucocorticoids, cytostatics, antibodies, drugs acting on immunophilins, among others. In some embodiments an additional therapy is an antiinflammatory drug. In general, such drugs can be classified as steroids, non-steroid antiinflammatory (NSAID), cyclooxygenase (COX) inhibitor, COX-2 inhibitor, COX-1 inhibitor, non-selective COX inhibitor and others. An additional therapy may sometimes be an antibiotic drug. In general, such drugs may be classified as aminoclycosides, Ansamycins, carbacephem, carapenems, cephalosporins, glycopeptides, macrolides, monobactams, penicillins, polypeptides, quinolones, sulfonamides and tetracyclines, among others. In some embodiments an additional therapy is an anti-viral drug. In general, such drugs can be classified as non-nucleoside reverse transcriptase inhibitors, nucleoside reverse transcriptase inhibitors, protease inhibitors and nucleotide analog reverse transcriptase inhibitors, among others. In certain embodiments an additional therapy is a steroid drug. In general, such drugs may be classified as corticosteroids and anabolic steroids, among others. In some embodiments an additional therapy is a chemotherapy drug. In general, such drugs may be classified as alkylating agents, antimetabolites, anti-tumor antibiotics, topoisomerase inhibitors, mitotic inhibitors and corticosteroids, among others. An additional therapy may sometimes be a hormone therapy drug. In general, such drugs may be classified as anti-estrogens, aromatase inhibitors, progestins, estrogens, anti-androgens and LHRH agonists, among others.

### Examples

The examples set forth below illustrate certain embodiments and do not limit the technology.

### Example 1: Inclusion Body Myositis Autoimmunity and Muscle Weakness are Both Linked to TNF-alpha modulation of microRNAs-1, -133, -206 in Myoblasts and Myocytes.

### Materials and Methods

### Cell Lines

Human Skeletal Muscle Myoblasts (HSMM; Lonza CC-2580) and Skeletal Muscle Myocytes (SkMC; Lonza CC-2561) were maintained and passed in SkGM-2 media (Lonza #CC-3245) and SkGB media (Lonza #CC-3160), respectively, as specified by the supplied cell line information and protocols. C2C12 murine myoblast cells (ATCC CRL-1772) were cultured and passaged as specified by the ATCC provided protocol (World Wide Web URL atcc.org/ATCCAdvancedCatalogSearch/ProductDetails/tabid/452/Default.aspx?ATCCNum=CRL-1772&Template=cellBiology).

### mRNA and miR expression

Total mRNA (consisting of both large and small species RNA) was isolated from all samples with the mirVana miRNA Isolation Kit (Ambion AM1560). RNA purity and concentration were determined spectrophotometrically (260/280>1.9) on a Nanodrop 1000. RNA quality was assessed on an Agilent 2100 Bioanalyzer using the RNA 6000 Nano LabChip®.

The generation of biotin-labeled amplified cRNA from 2 µg of total RNA was accomplished with the SuperScript® Double Strand cDNA Synthesis kit (Invitrogen 11917-020) and the Affymetrix GeneChip® IVT Labeling kit (Affymetrix 900449). The concentration and purity of the cRNA product were determined spectrophotometrically on a Nanodrop 1000. Twenty micrograms of each biotin-labeled cRNA was fragmented for hybridization on Affymetrix Human Genome U133 Plus 2.0 GeneChip® arrays. All GeneChip® washing, staining, and scanning procedures were performed with Affymetrix standard equipment. Data capture and initial array quality assessments were performed with the GeneChip Operating Software (GCOS) tool.

MicroRNAs were prepared for expression profiling using the TaqMan MicroRNA Reverse Transcription kit (ABI 4366597) and Multiplex RT for TaqMan MicroRNA Assays, Human Pool Set (ABI 4384791). MicroRNA expression was quantified with the TaqMan Low-Density Human MicroRNA Array v1.0 (ABI 4384792) using standard protocols. Specific mRNAs were quantified with TaqMan quantitative RT-PCR (qRT-PCR) assays, and specific microRNAs with either TaqMan microRNA qRT-PCR assays, or Exiqon mercury LNA Primer sets, miRCURY LNA First-strand cDNA Kit (Exiqon #201100), and miRCURY LNA SYBR Green Master Mix (Exiqon #201000).

TNF-alpha treatment. HSMM, or SkMC cells were seeded at 3-5x10⁵ cells per well in 6 well culture plates, or at 1x10⁵ cells/well in 24 well culture plates, overnight. TNF-alpha (R&D Systems 210-TA-050) was added at final concentration of 10 ng/ml. Also, cells were washed with once with PBS and total RNA was isolated from all with the mirVana miRNA Isolation Kit (Ambion AM1560). MicroRNA expression was quantified with individual TaqMan RT-PCR assays or on TLDA arrays.

### Myoblast differentiation.

HSMM or C2C12 cells were seeded at 3x10⁵ cells per well in 6 well, or at 6x10⁴ cells per well in 24 well, culture plates overnight to yield 60% confluency. Twenty-four (24) hrs later normal growth media was replaced with differentiation media (1:1 DMEM [Invitrogen #12430-104]:F12 nutrient mixture [Invitrogen #11765]) containing PBS vehicle control or 10 ng/ml TNF-alpha R&D Systems 210-TA-050). C2C12 cells were alternatively allowed to spontaneously differentiate in normal media. Cells were subjectively checked for myocyte differentiation by visualization on a Nikon Eclipse TE2000E inverted microscope, and digital images captured with Nikon NIS-Elements AR 3.00, SP7, Hotfix 9 (build 550) software. Elongated cells with greater than 3 nuclei were counted as myocytes/myotubes, while single nucleus cells were counted as myoblasts.

Myocyte differentiation was also quantified by expression of myocyte specific gene expression. Total RNA was isolated from cells with the miRVana RNA Isolation Kit (Ambion #AM1560). cDNA was synthesized with the SuperScript III Double Strand Synthesis Kit (Invitrogen #11752). mRNA expression was quantified with ABI TaqMan qRT-PCR assays for MYOD1 (4331182-HS00159528_M1), MYH1 (4331182-HS00428600-M1), MYH2 (4331182-HS00440042-M2), MYF5 (4331182-HS00929416-G1), MRF4/6 (4331182-HS01547104-M1), MYH10 (MM01325186_m1), NFKB1 (MM01316094_m1), NFKB2 (MM01201181_m1), NPTB (4331182-HS0021842-M1), PTB (4331182-HS00738537_M1), SRF (4331182-HS00182371_M1), HDAC4 (4331182-HS01041648_M1), Desmin (4331182-HS01090875_M1), Myogenin (4331182-HS01072232_M1), MEF2A 4331182-HS (4331182-HS00271535_M1), MEF2B (4331182-HS), MEF2C (MM00600427_m1), and MEF2D (MM00504936_m1). GAPDH (4331182-HS99999905-M1), b-actin (4331182-HS99999903-M1), and 18s RNA (4331182-HS99999901-S1) were run as normalization controls.

MicroRNA expression was quantified with Taqman qRT-PCR microRNA detection kits as described above. Data was analyzed with the -ΔA Ct method, referencing β-actin for normalization, to determine fold change in gene expression in HSMM cultured in differentiation media as compared to HSMM cells cultured in differentiation media and stimulated with TNF-alpha. The same methods were used when HSMM cell in these conditions were also transfected with microRNA mimics.

### MicroRNA transfection and transduction.

Artificial miR-1-1 (Dharmacon #C300586), miR-133a-1 (#C300600), miR-133b (#C300709), miR-206 (#C300644) microRNA mimics or scramble control (#CN00100) were transfected into HSMM and SkMC cells were transfected using PrimeFect siRNA (Lonza #PA-3269) or Lipofectamine 2000 (Invitrogen #11668) using the supplied protocols.

### Results

### IBM, PM and DM expression profiles

Expression profiles of mRNA and microRNA in IBM, polymyositis (PM), and dermatomyositis (DM) patient muscle biopsies compared to normal donor muscle biopsies were determined to examine the role of pro-inflammatory cytokines and microRNAs in the mechanism and pathology. Both TNF-alpha and GM-CSF transcripts were highly over-expressed, and miRs-1, 133, and 206 highly under-expressed, in myositic muscle and particularly in IBM muscle. Functional studies showed that TNF-alpha drives the expression of GM-CSF in muscle cells. It was also found that TNF-alpha suppressed expression of miR-1, miR-133a, miR-133b and miR-206 in muscle cells, leading to a block in myoblast-to-myocyte differentiation, as these three miRs are essential for driving the myoblast-to-myocyte transition.

The expression profiles indicated that the low level, non-necrotic/cytotoxic, inflammatory response leads infiltrating lymphocytes to chronically express cytokines, such as TNF-alpha, which suppresses microRNAs-1, -133a, -133b, and -206 expression in muscle myoblasts and myocytes. Suppression of these miRs appears to cause to muscle wasting due to suppression of miR-1, -133, and -206 leading to a defect in muscle repair by myoblast to myocyte development, which has been demonstrated to result in muscle wasting. Thus, the inflammation and muscle degeneration observed in myositis do not appear to be separate phenomena. Rather, IBM is primarily an autoimmune disease that drives muscle wasting by blocking myoblast-to-myocyte differentiation mediated muscle regeneration through blockade of expression of muscle critical microRNAs. These findings represent a novel mechanism for autoimmunity, and for inflammation related degenerative diseases; namely that pro-inflammatory signaling modulates miR expression leading to profound pathologies.

*TNF- alpha and GM-CSF transcripts were over-expressed and miRs hsa-1, -133, and -206 were under-expressed in IBM muscle.*
Inclusion Body Myositis (IBM) has been equally alternatively described primarily as either an autoimmune disease, or as a degenerative disease. Cytokines play a role in autoimmunity in both their effects on immunocytes and direct effects on target tissues. In particular, IFNγ, TNF-alpha, IL-1β, CCL-3, and/or CCL-4 have been suggested to possibly play roles in myositis. MicroRNAs have been shown to regulate immune responses and cytokine signaling, as well as tissue development, maintenance, and differentiation.

In order to determine the probable role of specific cytokines and miRs in myositis pathology mRNA and miR expression were compared by microarrays and/or quantitative PCR in 28 myositis patient muscle biopsies (10 with IBM, 10 with DM, and 8 with PM) to 17 normal donor muscle biopsies (Figure 1). TNF-alpha and GM-CSF were over-expressed, as assayed by quantitative PCR, by an average of 18.2-fold (P<0.001) and 63.0-fold (P<0.001) respectively in IBM muscle (Figure 1A). MicroRNAs hsa-miR-1, -133a, -133b, and -206, as assayed by TLDA (TaqMan Low Density Array), were all under-expressed in IBM muscle; 9-fold (P=7.9 E-8), 5-fold (P=0.000082), and 8-fold (P=1.8 E-8), and 2-fold (P=0..09) fold respectively (Figure 1 B). Similarly, GM-CSF protein was overexpressed in IBM muscle biopsies compared to normal donor muscle biopsies (Figure 2).

Thus, the current data demonstrated that the autoimmune and muscle wasting/degenerative pathologies of IBM are linked components of the same mechanistic phenomena mediated by proinflammatory cytokine modulation of muscle specific microRNAs. While not being bound by a particular theory, the data indicated that TNF-alpha production by muscle infiltrating autoimmunocytes leads to increased muscle cell GM-CSF production. Increased GM-CSF production by muscle cells apparently acted to recruit additional autoimmunocytes to the muscle perivasculature and endomycium. TNF-alpha also strongly inhibits myoblast and myocyte expression of miRs-1, -133, and -206; the three microRNAs shown to be critical for the differentiation of myoblasts to myocytes. Suppression of these miRs may cause muscle wasting due to suppression of miR-1, -133, and -206 leading to a defect in muscle repair by myoblast to myocyte development, which has been demonstrated to result in muscle wasting.

TNF-alpha mediated reduced expression of these three muscle critical miRs appears to lead to a profound defect in myoblast-to-myocyte differentiation that may be responsible for muscle cell degeneration and muscle wasting. Therefore, it appears that autoimmune production of cytokines such as TNF-alpha may lead to both the inflammatory and degenerative muscle wasting pathologies observed in IBM. Comparison of myositic muscle to normal donor muscle revealed greatly increased mRNA expression of the pro-inflammatory cytokines TNF-alpha and GM-CSF in PM, DM and IBM (Figure 1A). DM & IBM, which commonly display muscle wasting, showed decreased expression of all three muscle differentiation miRs. An inverse correlation was seen in decreased expression of microRNAs hsa-miR-1, -133, and -206 in DM, and IBM (Figure 1B). On the other hand, PM which displays severe muscle weakness, but not muscle wasting, showed decreased expression of only the miR-1/133a clusters, but expressed relatively normal amounts of the miR-133b/206 cluster. This raises the possibility that suppression of all four (miRs-1, 133a, 133b, and 206) miRs may be required to interrupt muscle regeneration and repair, and that suppression of miR-1 and/or -133a may be critical for normal muscle function.

### TNF-alpha potently suppresses myoblast-to-myocyte differentiation

TNF-alpha mediated inhibition of miRs-1, -133, and -206 in muscle cells (Figure 3) raised the possibility that TNF-alpha has the ability to affect muscle repair and regeneration, perhaps thereby leading to muscle wasting and degeneration seen in IBM. This is especially true when put into the context of other studies examining muscle differentiation and muscle wasting: NF-kappa-B, the major downstream mediator of TNF-alpha, seems to be highly involved in muscle wasting diseases. TNF-alpha signaling negatively regulated the action and expression of myosin, MyoD and Myogenin as well as Myocyte enhancing factor 2 (MEF2) (Figs 4 & 5). MyoD, Myogenin, and MEF2 drive expression of miR-133, as well as their clustered partners miR-1 and -206, in muscle cells. These three microRNAs have a role in skeletal muscle development and maintenance. Increases in miRs-1 and -206 drive myocyte differentiation and miR-133 affected myoblast proliferation. MiR-133 negatively regulates protein expression of nPTB and SRF, both factors that potently inhibit the differentiation of myoblasts to myocytes.

Tests were conducted to determine whether TNF-alpha blocks myoblast-to-myocyte differentiation, and thereby plays a role not only in the autoimmune inflammatory reaction, but also in the muscle wasting pathology of myositis.

HSMM cells exposed to differentiation media were >90% elongated multi-nucleated myocytes at day 4, where <10% of cells exposed to differentiation media and stimulated with TNF-alpha displayed myocyte morphology (Figure 4; Figures 13-15), indicating that TNF-alpha blocked differentiation. Since counting the number of multinucleated cells is a relatively subjective assay, the expression of myocyte specific gene expression in these cell cultures was also evaluated. Expression of a panel of myocyte specific genes, including Myosin Heavy Chain 1, Myosin Heavy Chain 2, MYF2 and MYOD1 were statistically significantly suppressed 1.2- to 2.6-fold in TNF-alpha stimulated HSMM cells (Figure 6), further indicating that TNF-alpha exposed myoblasts remained myoblasts and did not differentiate to myocytes when treated with differentiation media. As expected, TNF-alpha stimulation also increased the expression of TNF signal mediators NF-kappa-B1 and NF-kappa-B2 (Figure 6).

Similarly, TNF-alpha blocked differentiation of C2C12 myoblasts to myotubes (Figure 5, 7). After 7 days of exposure to differentiation media, C2C12 cells show lower cell density and have fused to long multi-nucleated cells indicative of myocyte/myotubes. However, in cells treated with TNF-alpha (10 ng/ml), a short single-cell morphology was observed, indicative of undifferentiated myoblasts. This lack of differentiation in the presence of TNF-alpha was further confirmed at the gene expression level. Figure 7 illustrates the decreased expression of myocyte-specific genes in C2C12 cells treated with TNF-alpha (10 ng/ml) compared to C2C12 cells cultured in differentiation media alone.

To determine if miR-1, -133, or -206 were involved in mediating the effects of TNFa on MiR-1 or myoblast-to-myocyte differentiation, C2C12 cells in maintenance media were transfected with miR scrambled control, miR-1, miR-133, or miR-206 at 200 nM, or with miR-1 and miR-206 at 100 nM each. After transfection, cells were cultured in differentiation media alone (Cells group) or differentiation media with TNF-alpha at 20 ng/ml (all other groups). At day 7 post transfection, cells were stained with DAPI and anti-MYH2-FITC (right immunofluorescence panels in Figure 8). The amount of MYH2 per sample was quantified by dividing the total FITC fluorescence by the total DAPI fluorescence for each image, then calculating an average ratio. Figure 9 shows that miR-1 and miR-206 (or a combination of both) are able to partially rescue the block in C2C12 differentiation induced by TNF-alpha. miR-133 also plays a role in rescuing the phenotype, although to a lesser extent that the other miRs.

To confirm the MYH2 staining data and the role of miRs-1, -133, and -206 in controlling C2C12 differentiation, gene expression and protein levels of known differentiation markers were measured. C2C12 cells were transfected with scrambled control-miR, miR-1, -133, -206 (all at 200 nM), or with a combination of miR-1 and miR-206 (at 100 nm each). 24 hrs post transfection (day 0) cells were washed and exposed to differentiation media with or without TNF-alpha (10 ng/ml). At day 4, cells were lysed, total RNA isolated, and myocyte specific gene expression (MYH1, MYH2, and MEF2C) was measured by RT-PCR (Figure 10). Additionally, at day 7, cells were lysed, protein was isolated, and MEF2C protein levels were measured by Western blot. Beta-actin was used as control. Relative amounts of MEF2C present in each sample were quantified using densitometry and are shown as the MEF2C/beta-actin ratio (Figure 11). Taken together, both the gene expression and protein results confirm that the addition of miR-1 and miR-206 (or a combination of both) reversed the block in muscle differentiation induced by TNF-alpha. As observed by MYH2 staining, miR-133 also plays a role in rescuing the phenotype, but to a lesser extent that the other miRs. Thus, forced microRNA expression overcame the TNF-alpha blockade of myocyte differentiation (Figure 8) and restored the expression of myocyte differentiation markers (Figures 8-11).

### MicroRNAs 1, 133, and 206 are critical for myoblast-to-myocyte differentiation

MiR-1, miR-133, and miR-206 have been shown to be essential for skeletal muscle development and maintenance. Increases in miRs-1 and -206 drive myocyte differentiation, and miR-133 affects myoblast proliferation. MiR-133 negatively regulates protein expression of nPTB and SRF, both of which are normally expressed in myoblast cells and function to potently block myoblast growth and myoblast-to-myocyte/myofiber differentiation. MiR-1 suppresses HDAC4 which is a transcriptional repressor of myocyte/myofiber gene expression, as well as muscle development associated genes such as Delta, fibronectin, Hand 2, HSP60, HSP70 and RasGAP. MiR-206 suppresses Fstl1, Pola1, and Utrn, all also muscle development associated genes. TNF-alpha signaling suppressed these microRNAs (Figure 3) in myoblasts as well as blocking their transition to myocytes (Figure 4, 5).

### GM-CSF does not appear to induce TNF-alpha

One hypothesis was that TNF-alpha was produced by infiltrating lymphocytes, and in turn induced GM-CSF expression by muscle cells. A second hypothesis was that GM-CSF, the only other proinflammatory cytokine over-represented in IBM muscle, induced muscle cells to produce TNF-alpha. To test this second hypothesis, HSMM and SkMC cells were stimulated with GM-CSF. TNF-alpha was not produced by either cell line at either the protein (measured by ELISA), or at the mRNA (measured by TaqMan RT-PCR) level (data not shown).

These data support TNF-alpha's contribution to both the autoimmune response and muscle wasting pathologies observed in IBM by inhibiting expression of muscle specific microRNAs hsa-*miR-1, -133,* and -206 (Figure 12).

In the immunologic component TNF-alpha signaling leads to a suppression of miR-133, a microRNA that potently suppresses GM-CSF protein expression. TNF-alpha signaling leads to NFkB activation, which suppresses MyoD, Myogenin and MEF2. In muscle cells MyoD, Myogenin, and MEF2 drive the expression of miR-1, miR-133, and miR-206. MiR-133 plays a role in myoblast-to-myocyte differentiation in that it suppresses nPTB and SRF, both of which are normally expressed in myoblast cells and function to potently block the myoblast-to-myocyte/myofiber differentiation. MiR-1 suppresses HDAC4 which is a transcriptional repressor of myocyte/myofiber gene expression, as well as muscle development associated genes such as Delta, fibronectin, Hand 2, HSP60, HSP70 and RasGAP. MiR-206 suppresses Fstl1, Pola1, and Utrn, all also muscle development associated genes. TNF-alpha signaling suppresses these microRNAs in myoblasts and appears to have blocked their transition to myocytes.

### Example 2: Examples of Embodiments

Provided hereafter are non-limiting examples of certain embodiments of the technology.
A1. A method comprising:
   (a) administering an immunosuppressive drug to a subject having an inflammatory myopathy;
   (b) identifying the presence, absence or amount of a biomarker in the subject, wherein the biomarker is selected from the group consisting of granulocyte macrophage colony-stimulating factor (GM-CSF) polypeptide, NF-kappa-B-modulating pro-inflammatory cytokine transcript or polypeptide, microRNA-1, microRNA-133, microRNA-206 or a portion of the foregoing; and
   (c) maintaining a subsequent dosage of the drug or adjusting a subsequent dosage of the drug administered to the subject based on the presence, absence or amount of the biomarker identified in the subject.
A2. A method comprising:
   (a) administering an immunosuppressive drug to a subject having an inflammatory myopathy;
   (b) identifying the presence, absence or amount of a biomarker in the subject, wherein the biomarker is selected from the group consisting of granulocyte macrophage colony-stimulating factor (GM-CSF) polypeptide, NF-kappa-B-modulating pro-inflammatory cytokine transcript or polypeptide, microRNA-1, microRNA-133, microRNA-206 or a portion of the foregoing; and
   (c) determining whether the dosage of the drug subsequently administered to the subject is adjusted based on the presence, absence or amount of the biomarker identified in the subject.
A3. A method comprising:
   (a) identifying the presence, absence or amount of a biomarker in a subject having an inflammatory myopathy to whom an immunosuppressive drug has been administered, wherein the biomarker is selected from the group consisting of granulocyte macrophage colony-stimulating factor (GM-CSF) polypeptide, NF-kappa-B-modulating pro-inflammatory cytokine transcript or polypeptide, microRNA-1, microRNA-133, microRNA-206 or a portion of the foregoing; and
   (b) maintaining a subsequent dosage of the drug or adjusting a subsequent dosage of the drug administered to the subject based on the presence, absence or amount of the biomarker identified in the subject.
A4. A method comprising:
   (a) identifying the presence, absence or amount of a biomarker in a subject having an inflammatory myopathy to whom an immunosuppressive drug has been administered, wherein the biomarker is selected from the group consisting of granulocyte macrophage colony-stimulating factor (GM-CSF) polypeptide, NF-kappa-B-modulating pro-inflammatory cytokine transcript or polypeptide, microRNA-1, microRNA-133, microRNA-206 or a portion of the foregoing; and
   (b) determining whether the dosage of the drug subsequently administered to the subject is adjusted based on the presence, absence or amount of the biomarker identified in the subject.
A5. A method comprising:
   (a) receiving information comprising the presence, absence or amount of a biomarker in a subject having an inflammatory myopathy to whom an immunosuppressive drug has been administered, wherein the biomarker is selected from the group consisting of granulocyte macrophage colony-stimulating factor (GM-CSF) polypeptide, NF-kappa-B-modulating pro-inflammatory cytokine transcript or polypeptide, microRNA-1, microRNA-133, microRNA-206 or a portion of the foregoing; and
   (b) maintaining a subsequent dosage of the drug or adjusting a subsequent dosage of the drug administered to the subject based on the presence, absence or amount of the biomarker identified in the subject.
A6. A method comprising:
   (a) identifying the presence, absence or amount of a biomarker in a subject having an inflammatory myopathy to whom an immunosuppressive drug has been administered, wherein the biomarker is selected from the group consisting of granulocyte macrophage colony-stimulating factor (GM-CSF) polypeptide, NF-kappa-B-modulating pro-inflammatory cytokine transcript or polypeptide, microRNA-1, microRNA-133, microRNA-206 or a portion of the foregoing; and
   (b) transmitting the presence, absence or amount of the biomarker to a decision maker who maintains a subsequent dosage of the drug or adjusts a subsequent dosage of the drug administered to the subject based on the presence, absence or amount of the biomarker identified in the subject.
A7. A method comprising:
   (a) identifying the presence, absence or amount of a biomarker in a subject having an inflammatory myopathy to whom an immunosuppressive drug has been administered, wherein the biomarker is selected from the group consisting of granulocyte macrophage colony-stimulating factor (GM-CSF) polypeptide, NF-kappa-B-modulating pro-inflammatory cytokine transcript or polypeptide, microRNA-1, microRNA-133, microRNA-206 or a portion of the foregoing; and
   (b) transmitting an indication to maintain a subsequent dosage of the drug or adjust a subsequent dosage of the drug administered to the subject based on the presence, absence or amount of the biomarker identified in the subject.
B1. A method for optimizing therapeutic efficacy of a treatment of an inflammatory myopathy, comprising:
   (a) administering an immunosuppressive drug to a subject having an inflammatory myopathy; and
   (b) identifying the presence, absence or amount of a biomarker in the subject, wherein the biomarker is selected from the group consisting of granulocyte macrophage colony-stimulating factor (GM-CSF) polypeptide, NF-kappa-B-modulating pro-inflammatory cytokine transcript or polypeptide, microRNA-1, microRNA-133, microRNA-206 or a portion of the foregoing; and
   (c) maintaining a subsequent dosage of the drug or adjusting a subsequent dosage of the drug administered to the subject based on the presence, absence or amount of the biomarker identified in the subject.
B2. A method for reducing toxicity of a treatment of an inflammatory myopathy, comprising:
   (a) administering an immunosuppressive drug to a subject having an inflammatory myopathy; and
   (b) identifying the presence, absence or amount of a biomarker in the subject, wherein the biomarker is selected from the group consisting of granulocyte macrophage colony-stimulating factor (GM-CSF) polypeptide, NF-kappa-B-modulating pro-inflammatory cytokine transcript or polypeptide, microRNA-1, microRNA-133, microRNA-206 or a portion of the foregoing; and
   (c) maintaining a subsequent dosage of the drug or adjusting a subsequent dosage of the drug administered to the subject based on the presence, absence or amount of the biomarker identified in the subject.
C1. A method comprising
   (a) administering an immunosuppressive drug to a subject having an inflammatory myopathy; and
   (b) identifying the amount of a granulocyte macrophage colony-stimulating factor (GM-CSF) polypeptide or portion thereof in the subject; and
   (c) maintaining a subsequent dosage of the drug or adjusting a subsequent dosage of the drug administered to the subject based on the amount of the GM-CSF polypeptide or portion thereof identified in the subject.
C2. A method comprising
   (a) administering an immunosuppressive drug to a subject having an inflammatory myopathy; and
   (b) identifying the amount of a tumor necrosis factor-alpha (TNF-alpha) transcript or polypeptide or portion thereof in the subject; and
   (c) maintaining a subsequent dosage of the drug or adjusting a subsequent dosage of the drug administered to the subject based on the amount of the TNF-alpha identified in the subject.
C3. A method comprising
   (a) administering an immunosuppressive drug to a subject having an inflammatory myopathy; and
   (b) identifying the amount of a microRNA-133 or portion thereof in the subject; and
   (c) maintaining a subsequent dosage of the drug or adjusting a subsequent dosage of the drug administered to the subject based on the amount of the microRNA-133 or portion thereof identified in the subject.
C4. The method of embodiment C3, wherein the microRNA-133 is one or more of microRNA-133a-1, microRNA-133a-2 and mircroRNA-133b.
C5. The method of embodiment C3 or C4, further comprising identifying the presence, absence or amount of one or more of a microRNA-1 and microRNA-206 or portion thereof and determining whether the dosage of the drug is adjusted for the subject based on the presence, absence or amount of the one or more of the microRNA-1 and microRNA-206 or portion thereof.
C6. The method of embodiment C5, wherein the microRNA-1 is microRNA-1-1, microRNA-1-2 or microRNA-1-1 and microRNA-1-2.
D1. The method of any one of embodiments A1 to C6, wherein the inflammatory myopathy is a myositis.
D2. The method of any one of embodiments A1 to D1, wherein the subject having the inflammatory myopathy has levels of GM-CSF polypeptide elevated relative to healthy subjects prior to administration of the immunosuppressive agent.
D2.1. The method of any one of embodiments A1 to D2, which comprises determining whether the subject having the inflammatory myopathy has levels of GM-CSF polypeptide elevated relative to healthy subjects prior to administration of the immunosuppressive agent.
D3. The method of any one of embodiments A1 to D2.1, wherein the subject having the inflammatory myopathy has levels of TNF-alpha transcript or polypeptide elevated relative to healthy subjects prior to administration of the immunosuppressive agent.
D3.1. The method of any one of embodiments A1 to D3, which comprises determining whether the subject having the inflammatory myopathy has levels of TNF-alpha transcript or polypeptide elevated relative to healthy subjects prior to administration of the immunosuppressive agent.
D4. The method of any one of embodiments A1 to D3.1, wherein the subject having the inflammatory myopathy has levels of microRNA-1, microRNA-133 or microRNA-206 reduced relative to healthy subjects prior to administration of the immunosuppressive agent.
D4.1. The method of any one of embodiments A1 to D4, which comprises determining whether the subject having the inflammatory myopathy has levels of microRNA-1, microRNA-133 or microRNA-206 reduced relative to healthy subjects prior to administration of the immunosuppressive agent.
D5. The method of embodiment D1, wherein the myositis is inclusion body myositis (IBM).
D6. The method of embodiment D1, wherein the myositis is dermatomyositis (DM).
D7. The method of embodiment D1, wherein the myositis is polymyositis (PM).
D7.1. The method of embodiment D1, wherein the myositis is juvenile myositis (JM).
D8. The method of any one of embodiments A1 to B2 and D1 to D7.1, wherein the microRNA-133 is one or more of microRNA-133a-1, microRNA-133a-2 and mircroRNA-133b.
D9. The method of any one of embodiments A1 to B2 and D1 to D8, wherein the microRNA-1 is microRNA-1-1, microRNA-1-2 or microRNA-1-1 and microRNA-1-2.
D10. The method of any one of embodiments A1 to D9, wherein the presence, absence or amount of the biomarker is determined from a biological sample from the subject.
D11. The method of embodiment 10, wherein sample contains blood or a blood fraction.
D12. The method of embodiment 10, wherein the sample contains a muscle biopsy product.
D13. The method of any one of embodiments A1 to D12, wherein the biomarker is the GM-CSF polypeptide or portion thereof.
D14. The method of embodiment D13, wherein the presence, absence or amount of the GM-CSF polypeptide or portion thereof is determined by a method that comprises contacting the GM-CSF polypeptide or portion thereof with an antibody that specifically binds to the GM-CSF polypeptide or portion thereof.
D15. The method of embodiment D13, wherein the presence, absence or amount of the GM-CSF polypeptide or portion thereof is determined by a method that comprises analyzing the GM-CSF polypeptide or portion thereof by high performance liquid chromatography.
D16. The method of embodiment D13, wherein the presence, absence or amount of the GM-CSF polypeptide or portion thereof is determined by a method that comprises analyzing the GM-CSF polypeptide or portion thereof by mass spectrometry.
D17. The method of any one of embodiments A1 to D11, wherein the biomarker is the NF-kappa-B-modulating pro-inflammatory cytokine polypeptide or portion thereof.
D18. The method of embodiment D17, wherein the presence, absence or amount of the NF-kappa-B-modulating pro-inflammatory cytokine polypeptide or portion thereof is determined by a method that comprises contacting a sample of the subject with an antibody that specifically binds to the NF-kappa-B-modulating pro-inflammatory cytokine polypeptide or portion thereof.
D19. The method of embodiment D17, wherein the presence, absence or amount of the NF-kappa-B-modulating pro-inflammatory cytokine polypeptide or portion thereof is determined by a method that comprises analyzing the NF-kappa-B-modulating pro-inflammatory cytokine polypeptide or portion thereof by high performance liquid chromatography.
D20. The method of embodiment D17, wherein the presence, absence or amount of the NF-kappa-B-modulating pro-inflammatory cytokine polypeptide or portion thereof is determined by a method that comprises analyzing the NF-kappa-B-modulating pro-inflammatory cytokine polypeptide or portion thereof by mass spectrometry.
D21. The method of any one of embodiments A1 to D11, wherein the biomarker is the NF-kappa-B-modulating pro-inflammatory cytokine transcript or portion thereof.
D22. The method of embodiment D21, wherein the presence, absence or amount of the NF-kappa-B-modulating pro-inflammatory cytokine transcript or portion thereof is determined by a method that comprises contacting a sample of the subject with a nucleic acid substantially complementary to the NF-kappa-B-modulating pro-inflammatory cytokine transcript or portion thereof.
D23. The method of embodiment D22, wherein the nucleic acid substantially complementary to the TNF-alpha transcript or portion thereof is in a nucleic acid array.
D24. The method of any one of embodiments A1 to D11, wherein the biomarker is one or more of the microRNA-1, microRNA-133, microRNA-206 or portion thereof.
D25. The method of embodiment D24, wherein the presence, absence or amount of the one or more of the microRNA-1, microRNA-133, microRNA-206 or portion thereof is determined by a method that comprises contacting a sample of the subject with a nucleic acid substantially complementary to the microRNA-1, microRNA-133, microRNA-206 or portion thereof.
D26. The method of any one of embodiments A1 to D25, wherein the subject is human.
D27. The method of any one of embodiments A1 to D26, wherein the NF-kappa-B-modulating pro-inflammatory cytokine is selected from the group consisting of tumor necrosis factor-alpha (TNF-alpha), IL-1 beta, IL-6, chemokines monocyte chemoattractant protein-1, IL-8, and the AP-1-dependent basic fibroblast growth factor.
E1. A method for treating an inflammatory myopathy in a subject, comprising administering a pharmaceutical composition in an amount effective to reduce the amount of granulocyte macrophage colony-stimulating factor (GM-CSF) in the subject.
E2. The method of embodiment E1, comprising administering an immunosuppressive drug to the subject.
E3. The method of embodiment E1 or E2, comprising administering an antibody to the subject.
E4. The method of any one of embodiments E1 to E3, comprising administering an siNA to the subject.
E5. The method of any one of embodiments E1 to E4, comprising administering an antiinflammatory agent to the subject.
E6. The method of any one of embodiments E1 to E5, comprising administering an antibiotic agent to the subject.
E7. The method of any one of embodiments E1 to E6, comprising administering an anti-viral agent to the subject.
E8. The method of any one of embodiments E1 to E7, comprising administering a steroid agent to the subject.
E9. The method of any one of embodiments E1 to E8, comprising administering a chemotherapy agent to the subject.
E10. The method of any one of embodiments E1 to E9, wherein the pharmaceutical composition comprises a nucleic acid composition.
E11. The method of any one of embodiments E1 to E9, wherein the pharmaceutical composition comprises a microRNA.
E12. The method of any one of embodiments E1 to E9, wherein the pharmaceutical composition consists of a microRNA.
E12.1. The method of any one of embodiments E1 to E12, wherein the microRNA is one or more of microRNA-1, microRNA-133 and microRNA-206.
E12.2. The method of embodiment E12.1, wherein the microRNA-133 is microRNA133a-1, microRNA133a-2 or combination thereof.
E13. The method of any one of embodiment E12.1, wherein the microRNA-1 is microRNA-1-1, microRNA-1-2 or combination thereof.
E14. The method of any one of embodiments E1 to E13, wherein the nucleic acid composition comprises an siNA.
E15. The method of any one of embodiments E1 toE14 wherein the nucleic acid composition consists of an siNA.
E16. The method of any one of embodiments E1 to E15, comprising
   (a) identifying the presence, absence or amount of a biomarker in the subject, wherein the biomarker is selected from the group consisting of granulocyte macrophage colony-stimulating factor (GM-CSF) polypeptide, tumor necrosis factor alpha (TNF-alpha), NF-kappa-B-modulating pro-inflammatory cytokine transcript or polypeptide, microRNA-1, microRNA-133, microRNA-206 or a portion of the foregoing; and
   (b) maintaining a subsequent dosage of the microRNA drug or adjusting a subsequent dosage of the microRNA drug administered to the subject based on the presence, absence or amount of the biomarker identified in the subject.
E17. The method of any one of embodiments E1 to E16, wherein the subject having the inflammatory myopathy is identified as having levels of GM-CSF polypeptide elevated relative to healthy subjects prior to administration of the immunosuppressive agent.
E18. The method of any one of embodiments E16 and E17, wherein the subject having the inflammatory myopathy is identified as having levels of TNF-alpha transcript or polypeptide elevated relative to healthy subjects prior to administration of the immunosuppressive agent.
E19. The method of any one of embodiments E16 toE18, wherein the subject having the inflammatory myopathy is identified as having levels of microRNA-1, microRNA-133 or microRNA-206 reduced relative to healthy subjects prior to administration of the nucleic acid composition.
E20. The method of any one of embodiments E1 to E19, wherein the inflammatory myopathy is a myositis.
E21. The method of any one of embodiments E1 to E20, wherein the myositis is inclusion body myositis (IBM).
E22. The method of any one of embodiments E1 to E21, wherein the myositis is dermatomyositis (DM).
E23. The method of any one of embodiments E1 to E22, wherein the myositis is polymyositis (PM).
F1. A method for treating an inflammatory myopathy in a subject, comprising administering a pharmaceutical composition in an amount effective to reduce the amount of tumor necrosis factor-alpha (TNF-alpha) transcript or polypeptide in the subject.
F2. The method of embodiment F1, comprising administering an immunosuppressive drug to the subject.
F3. The method of embodiment F1 or F2, comprising administering an antibody to the subject.
F4. The method of any one of embodiments F1 to F3, comprising administering an siNA to the subject.
F5. The method of any one of embodiments F1 to F4, comprising administering an antiinflammatory agent to a subject.
F6. The method of any one of embodiments F1 to F5, comprising administering an antibiotic agent to a subject.
F7. The method of any one of embodiments F1 to F6, comprising administering an anti-viral agent to a subject.
F8. The method of any one of embodiments F1 to F7, comprising administering a steroid agent to a subject.
F9. The method of any one of embodiments F1 to F8, comprising administering a chemotherapy agent to a subject.
F10. The method of any one of embodiments F1 to F9, wherein the pharmaceutical composition comprises a nucleic acid.
F11. The method of any one of embodiments F1 to F9, wherein the pharmaceutical composition comprises a microRNA.
F12. The method of any one of embodiments F1 to F9, wherein the pharmaceutical composition consists of a microRNA.
F12.1. The method of any one of embodiments F1 to F12, wherein the microRNA is one or more of microRNA-1, microRNA-133 and microRNA-206.
F12.2. The method of embodiment F12.1, wherein the microRNA is one or more of microRNA133a-1 and microRNA133a-2.
F13. The method of any one of embodiment F12.1, wherein the microRNA is one or more of microRNA-1-1, microRNA-1-2.
F14. The method of any one of embodiments F1 to F13, wherein the nucleic acid composition comprises an siNA.
F15. The method of any one of embodiments F1 toF14, wherein the nucleic acid composition consists of an siNA.
F16. The method of any one of embodiments F1 to F15, comprising
   (a) identifying the presence, absence or amount of a biomarker in the subject, wherein the biomarker is selected from the group consisting of granulocyte macrophage colony-stimulating factor (GM-CSF) polypeptide, tumor necrosis factor alpha (TNF-alpha), NF-kappa-B-modulating pro-inflammatory cytokine transcript or polypeptide, microRNA-1, microRNA-133, microRNA-206 or a portion of the foregoing; and
   (b) maintaining a subsequent dosage of the microRNA drug or adjusting a subsequent dosage of the microRNA drug administered to the subject based on the presence, absence or amount of the biomarker identified in the subject.
F17. The method of any one of embodiments F1 to F16, wherein the subject having the inflammatory myopathy is identified as having levels of GM-CSF polypeptide elevated relative to healthy subjects prior to administration of the immunosuppressive agent.
F18. The method of any one of embodiments F16 to F17, wherein the subject having the inflammatory myopathy is identified as having levels of TNF-alpha transcript or polypeptide elevated relative to healthy subjects prior to administration of the immunosuppressive agent.
F19. The method of any one of embodiments F16 to F18, wherein the subject having the inflammatory myopathy is identified as having levels of microRNA-1, microRNA-133 or microRNA-206 reduced relative to healthy subjects prior to administration of the nucleic acid composition.
F20. The method of any one of embodiments F1 toF19, wherein the inflammatory myopathy is a myositis.
F21. The method of any one of embodiments F1 to F20, wherein the myositis is inclusion body myositis (IBM).
F22. The method of any one of embodiments F1 to F21, wherein the myositis is dermatomyositis (DM).
F23. The method of any one of embodiments F1 to F22, wherein the myositis is polymyositis (PM).
G1. A method for treating an inflammatory myopathy in a subject, comprising administering a pharmaceutical composition in an amount effective to modulate the amount of microRNA-1 in the subject.
G2. A method for treating an inflammatory myopathy in a subject, comprising administering a pharmaceutical composition in an amount effective to modulate the amount of microRNA-133 in the subject.
G3. A method for treating an inflammatory myopathy in a subject, comprising administering a pharmaceutical composition in an amount effective to modulate the amount of microRNA-206 in the subject.
G4. The method of one or more of embodiments G1 to G4, wherein the pharmaceutical composition comprises a microRNA.
G5. The method of embodiment G4, wherein the microRNA comprises microRNA-1.
G6. The method of embodiment G4, wherein the microRNA comprises microRNA-133.
G7. The method of embodiment G4, wherein the microRNA comprises microRNA-206.
H1. A method for differentiating myoblasts, comprising contacting myoblasts with a composition that reduces the amount of active TNF-alpha in the myoblasts, which composition is in an amount effective to differentiate the myoblasts to myocytes and/or myotubes.
H2. The method of embodiment H1, wherein the composition comprises an siRNA against a TNF-alpha transcript.
H3. The method of embodiment H1, wherein the composition comprises an antibody that neutralizes or inhibits TNF-alpha.
H4. A method for differentiating myoblasts, comprising contacting myoblasts with a composition that reduces the amount of active GM-CSF in the myoblasts, which composition is in an amount effective to differentiate the myoblasts to myocytes and/or myotubes.
H5. The method of embodiment H4, wherein the composition comprises an siRNA against a GM-CSF transcript.
H6. The method of embodiment H4, wherein the composition comprises an antibody that neutralizes or inhibits GM-CSF.
H7. A method for differentiating myoblasts, comprising contacting myoblasts with a composition that increases the amount of miRNA-1, miRNA-133 and/or miRNA-206 in the myoblasts, which composition is in an amount effective to differentiate the myoblasts to myocytes and/or myotubes.
H8. The method of embodiment H7, wherein the composition comprises the miRNA-1, miRNA-133 and/or miRNA-206.
H9. The method of embodiment H7, wherein the composition comprises one or more nucleic acids that encode the miRNA-1, miRNA-133 and/or miRNA-206.
H10. The method of any one of embodiments H1 to H9, wherein the myoblasts are in vitro.
H11. The method of embodiment H10, which comprises administering the myoblasts, and/or myocytes and/or myotubes differentiated therefrom, to a subject after the myoblasts are contacted with the composition.
H12. The method of any one of embodiments H1 to H9, wherein the myoblasts are in a subject and the composition is administered to the subject.
H13. The method of embodiment H12, comprising administering an immunosuppressive drug to the subject.
H14. The method of embodiment H12 or H13, comprising administering an antibody to the subject.
H15. The method of any one of embodiments H12 to H14, comprising administering an siNAto the subject.
H16. The method of any one of embodiments H12 to H15, comprising administering an antiinflammatory agent to a subject.
H17. The method of any one of embodiments H12 to H16, comprising administering an antibiotic agent to a subject.
H18. The method of any one of embodiments H12 to H17, comprising administering an anti-viral agent to a subject.
H19. The method of any one of embodiments H12 to H18, comprising administering a steroid agent to a subject.
H20. The method of any one of embodiments H12 to H19, comprising administering a chemotherapy agent to a subject.
H21. The method of any one of embodiments H7 to H20, wherein the microRNA-133 is one or more of microRNA133a-1 and microRNA133a-2.
H22. The method of any one of embodiments H7 to H21, wherein the microRNA-1 is one or more of microRNA-1-1 and microRNA-1-2.
H23. The method of any one of embodiments H1 to H22, comprising
   (a) identifying the presence, absence or amount of a biomarker in the myoblasts, myocytes and/or myotubes, wherein the biomarker is selected from the group consisting of granulocyte macrophage colony-stimulating factor (GM-CSH) polypeptide, tumor necrosis factor alpha (TNH-alpha), NH-kappa-B-modulating pro-inflammatory cytokine transcript or polypeptide, microRNA-1, microRNA-133, microRNA-206 or a portion of the foregoing; and
   (b) maintaining a subsequent dosage of the composition or adjusting a subsequent dosage of the composition contacting the myoblasts based on the presence, absence or amount of the biomarker identified in the subject.
H24. The method of any one of embodiments H1 to H23, wherein the myoblasts are from a subject diagnosed with an inflammatory myopathy.
H25. The method of embodiment H24, wherein the inflammatory myopathy is a myositis.
H26. The method of embodiment H25, wherein the myositis is inclusion body myositis (IBM).
H27. The method of embodiment H25, wherein the myositis is dermatomyositis (DM).
H28. The method of embodiment H25, wherein the myositis is polymyositis (PM).
H29. The method of embodiment H25, wherein the myositis is juvenile myositis (JM).
I1. The method of any one of embodiments E1 to H28, wherein the presence, absence or amount of a biomarker is determined from a biological sample from the subject.
I2. The method of embodiment I1, wherein sample contains blood or a blood fraction.
I3. The method of embodiment I1, wherein the sample contains a muscle biopsy product.
I4. The method of any one of embodiments I1 to I3, wherein the biomarker is GM-CSF polypeptide or portion thereof.
I6. The method of embodiment I4, wherein the presence, absence or amount of the GM-CSF polypeptide or portion thereof is determined by a method that comprises contacting the GM-CSF polypeptide or portion thereof with an antibody that specifically binds to the GM-CSF polypeptide or portion thereof.
I7. The method of embodiment I4, wherein the presence, absence or amount of the GM-CSF polypeptide or portion thereof is determined by a method that comprises analyzing the GM-CSF polypeptide or portion thereof by high performance liquid chromatography.
I8. The method of embodiment 14, wherein the presence, absence or amount of the GM-CSF polypeptide or portion thereof is determined by a method that comprises analyzing the GM-CSF polypeptide or portion thereof by mass spectrometry.
I9. The method of any one of embodiments I1 to I3, wherein the biomarker is TNF-alpha polypeptide or a portion thereof.
I10. The method of embodiment I9, wherein the presence, absence or amount of the TNF-alpha polypeptide or portion thereof is determined by a method that comprises contacting a sample of the subject with an antibody that specifically binds to the TNF-alpha polypeptide or portion thereof.
I11. The method of embodiment 19, wherein the presence, absence or amount of the TNF-alpha polypeptide or portion thereof is determined by a method that comprises analyzing the TNF-alpha polypeptide or portion thereof by high performance liquid chromatography.
I12. The method of embodiment I9, wherein the presence, absence or amount of the TNF-alpha polypeptide or portion thereof is determined by a method that comprises analyzing a nucleic acid that binds to the TNF-alpha polypeptide or portion thereof by PCR or real-time PCR.
I13. The method of embodiment I9, wherein the presence, absence or amount of the TNF-alpha polypeptide or portion thereof is determined by a method that comprises analyzing the TNF-alpha polypeptide or portion thereof by mass spectrometry.
I14. The method of any one of embodiments I1 to I3 wherein the biomarker is a TNF-alpha transcript or portion thereof.
I15. The method of embodiment I14, wherein the presence, absence or amount of the TNF-alpha transcript or portion thereof is determined by a method that comprises contacting a sample of the subject with a nucleic acid substantially complementary to the TNF-alpha transcript or portion thereof.
I16. The method of embodiment I15, wherein the nucleic acid substantially complementary to the TNF-alpha transcript or portion thereof is in a nucleic acid array.
I17. The method of any one of embodiments I1 to 13, wherein the biomarker is one or more of microRNA-1, microRNA-133, microRNA-206 or portion thereof.
I18. The method of embodiment I17, wherein the presence, absence or amount of the one or more of the microRNA-1, microRNA-133, microRNA-206 or portion thereof is determined by a method that comprises contacting a sample of the subject with a nucleic acid substantially complementary to the microRNA-1, microRNA-133, microRNA-206 or portion thereof.
I19. The method of embodiment I18, wherein the nucleic acid substantially complementary to microRNA-1, microRNA-133, microRNA-206 or portion thereof is in a nucleic acid array.
I20. The method of any one of embodiments E1 to I19, wherein the subject is human.

The entirety of each patent, patent application, publication and document referenced herein hereby is incorporated by reference. Citation of the above patents, patent applications, publications and documents is not an admission that any of the foregoing is pertinent prior art, nor does it constitute any admission as to the contents or date of these publications or documents.

Modifications may be made to the foregoing without departing from the basic aspects of the technology. Although the technology has been described in substantial detail with reference to one or more specific embodiments, those of ordinary skill in the art will recognize that changes may be made to the embodiments specifically disclosed in this application, yet these modifications and improvements are within the scope and spirit of the technology.

The technology illustratively described herein suitably may be practiced in the absence of any element(s) not specifically disclosed herein. Thus, for example, in each instance herein any of the terms "comprising," "consisting essentially of," and "consisting of" may be replaced with either of the other two terms. The terms and expressions which have been employed are used as terms of description and not of limitation, and use of such terms and expressions do not exclude any equivalents of the features shown and described or portions thereof, and various modifications are possible within the scope of the technology claimed. The term "a" or "an" can refer to one of or a plurality of the elements it modifies (e.g., "a reagent" can mean one or more reagents) unless it is contextually clear either one of the elements or more than one of the elements is described. The term "about" as used herein refers to a value within 10% of the underlying parameter (i.e., plus or minus 10%), and use of the term "about" at the beginning of a string of values modifies each of the values (i.e., "about 1, 2 and 3" refers to about 1, about 2 and about 3). For example, a weight of "about 100 grams" can include weights between 90 grams and 110 grams. Further, when a listing of values is described herein (e.g., about 50%, 60%, 70%, 80%, 85% or 86%) the listing includes all intermediate and fractional values thereof (e.g., 54%, 85.4%). Thus, it should be understood that although the present technology has been specifically disclosed by representative embodiments and optional features, modification and variation of the concepts herein disclosed may be resorted to by those skilled in the art, and such modifications and variations are considered within the scope of this technology.

Certain embodiments of the technology are set forth in the claim(s) that follow(s).

## Claims

1. A method comprising:
(a) identifying the presence, absence or amount of a biomarker in a subject having an inflammatory myopathy to whom an immunosuppressive drug has been administered, wherein the biomarker is a microRNA-133 selected from one or more of the group consisting of microRNA-133a-1, microRNA-133a-2 and mircroRNA-133b; and
(b) maintaining a subsequent dosage of the drug or adjusting a subsequent dosage of the drug administered to the subject based on the presence, absence or amount of the biomarker identified in the subject.

2. A method comprising:
(a) identifying the presence, absence or amount of a biomarker in a subject having an inflammatory myopathy to whom an immunosuppressive drug has been administered, wherein the biomarker is a microRNA-133, selected from one or more of the group consisting of microRNA-133a-1, microRNA-133a-2 and mircroRNA-133b; and
(b) determining whether the dosage of the drug subsequently administered to the subject is adjusted based on the presence, absence or amount of the biomarker identified in the subject.

3. A method for optimizing therapeutic efficacy of a treatment of an inflammatory myopathy, comprising:
(a) administering an immunosuppressive drug to a subject having an inflammatory myopathy; and
(b) identifying the presence, absence or amount of a biomarker in the subject, wherein the biomarker is a microRNA-133, selected from one or more of the group consisting of microRNA-133a-1, microRNA-133a-2 and mircroRNA-133b; and
(c) maintaining a subsequent dosage of the drug or adjusting a subsequent dosage of the drug administered to the subject based on the presence, absence or amount of the biomarker identified in the subject.

4. A method for reducing toxicity of a treatment of an inflammatory myopathy, comprising:
(a) administering an immunosuppressive drug to a subject having an inflammatory myopathy; and
(b) identifying the presence, absence or amount of a biomarker in the subject, wherein the biomarker is a microRNA-133 selected from one or more of the group consisting of microRNA-133a-1, microRNA-133a-2 and mircroRNA-133b; and
(c) maintaining a subsequent dosage of the drug or adjusting a subsequent dosage of the drug administered to the subject based on the presence, absence or amount of the biomarker identified in the subject.

5. The method of any preceding claim, further comprising identifying the presence, absence or amount of one or more of a microRNA-1 and microRNA-206 or portion thereof and determining whether the dosage of the drug is adjusted for the subject based on the presence, absence or amount of the one or more of the microRNA-1 and microRNA-206 or portion thereof.

6. The method of claim 5, wherein the microRNA-1 is microRNA-1-1, microRNA- 1-2 or microRNA-1-1 and microRNA-1-2.

7. The method of any preceding claim, wherein the inflammatory myopathy is a myositis.

8. The method of any preceding claim, wherein the subject having the inflammatory myopathy has levels of GM-CSF polypeptide, or levels of TNF-alpha transcript elevated relative to healthy subjects prior to administration of the immunosuppressive agent or has levels of microRNA-1, microRNA-133 or microRNA-206 reduced relative to healthy subjects prior to administration of the immunosuppressive agent.

9. The method of any preceding claim, wherein the myositis is selected from the group consisting of, inclusion body myositis (IBM), dermatomyositis (OM), polymyositis (PM),and juvenile myositis (JM).
